(19) Europäisches Patentamt

European Patent Office

Office européen des brevets

(11) EP 1 151 102 B1

(12) EUROPEAN PATENT SPECIFICATION

(45) Date of publication and mention
of the grant of the patent:
**19.04.2006 Bulletin 2006/16**

(51) Int Cl.:
*C12N 15/16* (2006.01)   *C07K 14/575* (2006.01)
*C12N 15/63* (2006.01)   *C12N 5/10* (2006.01)
*A61K 38/22* (2006.01)   *A61P 3/04* (2006.01)
*A61P 3/06* (2006.01)   *A61P 5/48* (2006.01)
*C07K 16/26* (2006.01)

(21) Application number: **00911784.7**

(22) Date of filing: **11.02.2000**

(86) International application number:
**PCT/US2000/003652**

(87) International publication number:
**WO 2000/047741 (17.08.2000 Gazette 2000/33)**

(54) **GLYCOSYLATED LEPTIN COMPOSITIONS AND RELATED METHODS**

GLYKOSYLIERTE LEPTINZUSAMMENSETZUNGEN UND ZUGEHÖRIGE VERFAHREN

COMPOSITIONS GLYCOSYLEES DE LEPTINE ET PROCEDES CORRESPONDANTS

(84) Designated Contracting States:
**AT BE CH CY DE DK ES FI FR GB GR IE IT LI LU MC NL PT SE**
Designated Extension States:
**AL LT LV MK RO SI**

(30) Priority: **12.02.1999 US 249675**

(43) Date of publication of application:
**07.11.2001 Bulletin 2001/45**

(73) Proprietor: **AMGEN INC.**
**Thousand Oaks, CA 91320-1799 (US)**

(72) Inventors:
• **MARTIN, Frances, H.**
**Newbury Park, CA 91320 (US)**
• **ELLIOTT, Steven, G.**
**Newbury Park, CA 91320 (US)**

(74) Representative: **Richardson, Kate et al**
**Forrester & Boehmert,**
**Pettenkoferstrasse 20-22**
**80336 München (DE)**

(56) References cited:
EP-A- 0 486 193       EP-A- 0 827 750
WO-A-00/09165       WO-A-00/21574
WO-A-95/05465       WO-A-97/18833
WO-A-97/20933       WO-A-97/26916
WO-A-99/23115       GB-A- 2 292 382

• **LE MAIRE M ET AL: "THE USE OF HIGH-PERFORMANCE LIQUID CHROMATOGRAPHY FOR THE DETERMINATION OF SIZE AND MOLECULAR WEIGHT OF PROTEINS A CAUTION AND A LIST OF MEMBRANE PROTEINS SUITABLE AS STANDARDS" ANALYTICAL BIOCHEMISTRY, vol. 154, no. 2, 1986, pages 525-535, XP000925150 ISSN: 0003-2697 cited in the application**
• **BAUDYS MIROSLAV ET AL: "Physical stabilization of insulin by glycosylation." JOURNAL OF PHARMACEUTICAL SCIENCES, vol. 84, no. 1, 1995, pages 28-33, XP000929132 ISSN: 0022-3549**
• **WANG CHANGQING ET AL: "Influence of the carbohydrate moiety on the stability of glycoproteins." BIOCHEMISTRY, vol. 35, no. 23, 1996, pages 7299-7307, XP000925259 ISSN: 0006-2960**
• **COUSIN PATRICE ET AL: "Human variant sex hormone-binding globulin (SHBG) with an additional carbohydrate chain has a reduced clearance rate in rabbit." JOURNAL OF CLINICAL ENDOCRINOLOGY & METABOLISM, vol. 83, no. 1, January 1998 (1998-01), pages 235-240, XP000925320 ISSN: 0021-972X**
• **ZHANG FAMING ET AL: "Crystal structure of the obese protein leptin-E100." NATURE (LONDON), vol. 387, no. 6629, 1997, pages 206-209, XP000885288 ISSN: 0028-0836 cited in the application**

- **TAKASHI MURAKAMI ET AL: "CLONING OF RAT OBESE CDNA AND ITS EXPRESSION IN OBESE RATS" BIOCHEMICAL AND BIOPHYSICAL RESEARCH COMMUNICATIONS,US,ACADEMIC PRESS INC. ORLANDO, FL, vol. 209, no. 3, 26 April 1995 (1995-04-26), pages 944-952, XP000602094 ISSN: 0006-291X cited in the application**
- **YIYING ZHANG ET AL: "POSITIONAL CLONING OF THE MOUSE OBESE GENE AND ITS HUMAN HOMOLOGUE" NATURE,GB,MACMILLAN JOURNALS LTD. LONDON, vol. 372, no. 6505, 1 December 1994 (1994-12-01), pages 425-432, XP000602062 ISSN: 0028-0836 cited in the application**
- **COUSIN PATRICE ET AL: "Influence of glycosylation on the clearance of recombinant human sex hormone-binding globulin from rabbit blood." JOURNAL OF STEROID BIOCHEMISTRY AND MOLECULAR BIOLOGY, vol. 70, no. 4-6, September 1999 (1999-09), pages 115-121, XP000925370 ISSN: 0960-0760**

SEQUENCE LISTING

<110> AMGEN INC.

<120> GLYCOSYLATED LEPTIN COMPOSITIONS AND RELATED METHODS

<130> A-549

<140> 09/249.675
<141> 1999-02-12

<160> 36

<170> Patent In Ver. 2.1

<210> 1
<211> 146
<212> PRT
<213> Artificial Sequence

<220>
<223> Description of Artificial Sequence: rHu leptin 1 to 146

<400> 1

```
Val Pro Ile Gln Lys Val Gln Asp Asp Thr Lys Thr Leu Ile Lys Thr
 1               5                  10                  15

Ile Val Thr Arg Ile Asn Asp Ile Ser His Thr Gln Ser Val Ser Ser
            20              25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
            85                  90                  95

His Leu Pro Trp Ala Ser Gly Leu Glu Thr Leu Asp Ser Leu Gly Gly
        100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145
```

<210> 2
<211> 145
<212> PRT
<213> Artificial Sequence

<220>
<223> Description of Artificial Sequence:rhu leptin 1 to 145

<400> 2

```
Val Pro Ile Gln Lys Val Gln Asp Asp Thr Lys Thr Leu Ile Lys Thr
 1               5                  10              15

Ile Val Thr Arg Ile Asn Asp Ile Ser His Thr Ser Val Ser Ser Lys
            20                  25              30

Gln Lys Val Thr Gly Leu Asp Phe Ile Pro Gly Leu His Pro Ile Leu
        35                  40              45

Thr Leu Ser Lys Met Asp Gln Thr Leu Ala Val Tyr Gln Gln Ile Leu
        50                  55                  60

Thr Ser Met Pro Ser Arg Asn Val Ile Gln Ile Ser Asn Asp Leu Glu
65                  70                  75                  80

Asn Leu Arg Asp Leu Leu His Val Leu Ala Phe Ser Lys Ser Cys His
                85                  90                  95

Leu Pro Trp Ala Ser Gly Leu Glu Thr Leu Asp Ser Leu Gly Gly Val
            100                 105                 110

Leu Glu Ala Ser Gly Tyr Ser Thr Glu Val Val Ala Leu Ser Arg Leu
        115                 120                 125

Gln Gly Ser Leu Gln Asp Met Leu Trp Gln Leu Asp Leu Ser Pro Gly
        130                 135                 140

Cys
145
```

<210> 3
<211> 21
<212> PRT
<213> Homo sapiens

<400> 3

```
Met His Trp Gly Thr Leu Cys Gly Phe Leu Trp Leu Trp Pro Tyr Leu
 1               5                  10              15

Phe Tyr Val Gln Ala
            20
```

<210> 4
<211> 22
<212> PRT
<213> Artificial Sequence

<220>
<223> Description of Artificial Sequence:modified human leptin signal peptide

<400> 4

```
Met His Trp Gly Thr Leu Cys Gly Phe Leu Trp Leu Trp Pro Tyr Leu
 1               5                  10              15

Phe Tyr Val Ser Pro Ser
            20
```

<210> 5
<211> 21
<212> PRT

<213> Artificial Sequence

<220>
<223> Description of Artificial Sequence: modified human leptin signal peptide

<400> 5

```
        Met His Trp Gly Thr Leu Cys Gly Phe Leu Trp Leu Trp Pro Tyr Leu
         1               5                  10                  15

        Phe Tyr Val Ser Pro
                        20
```

<210> 6
<211> 22
<212> PRT
<213> Artificial Sequence

<220>
<223> Description of Artificial Sequence: modified human leptin signal peptide

<400> 6

```
        Met His Trp Gly Thr Leu Cys Gly Phe Leu Trp Leu Trp Pro Tyr Leu
         1               5                  10                  15

        Phe Tyr Val Ser Pro Ala
                        20
```

<210> 7
<211> 22
<212> PRT
<213> Artificial Sequence

<220>
<223> Description of Artificial Sequence: modified human leptin signal peptide

<400> 7

```
        Met His Trp Gly Thr Leu Cys Gly Phe Leu Trp Leu Trp Pro Tyr Leu
         1               5                  10                  15

        Phe Tyr Val Ser Asn Ser
                        20
```

<210> 8
<211> 23
<212> PRT
<213> Homo sapiens

<400> 8

```
        Met Asp Ala Met Lys Arg Gly Leu Cys Cys Val Leu Leu Leu Cys Gly
         1               5                  10                  15

        Ala Val Phe Val Ser Pro Ser
                        20
```

<210> 9
<211> 22
<212> PRT
<213> Homo sapiens

<400> 9

```
Met Asp Ala Met Lys Arg Gly Leu Cys Cys Val Leu Leu Leu Cys Gly
 1               5                  10                      15

Ala Val Phe Val Ser Pro
                20
```

<210> 10
<211> 23
<212> PRT
<213> Artificial Sequence

<220>
<223> Description of Artificial Sequence: modified tPA signal peptide

<400> 10

```
Met Asp Ala Met Lys Arg Gly Leu Cys Cys Val Leu Leu Leu Cys Gly
 1               5                  10                      15

Ala Val Phe Val Ser Asn Ser
                20
```

<210> 11
<211> 23
<212> PRT
<213> Artificial Sequence

<220>
<223> Description of Artificial Sequence: modified tPA signal peptide

<400> 11

```
Met Asp Ala Met Lys Arg Gly Leu Cys Cys Val Leu Leu Leu Cys Gly
 1               5                  10                      15

Ala Val Phe Val Ser Pro Ala
                20
```

<210> 12
<211> 21
<212> PRT
<213> Artificial Sequence

<220>
<223> Description of Artificial Sequence: leptin/tPA signal peptide

<400> 12

```
        Met His Trp Gly Thr Leu Cys Cys Val Leu Leu Leu Cys Gly Ala Val
          1               5                   10                  15

        Phe Val Ser Pro Ser
                        20
```

<210> 13
<211> 20
<212> PRT
<213> Artificial Sequence

<220>
<223> Description of Artificial Sequence: leptin/tPA signal peptide

<400> 13

```
        Met His Trp Gly Thr Leu Cys Cys Val Leu Leu Leu Cys Gly Ala Val
          1               5                   10                  15

        Phe Val Ser Pro
                    20
```

<210> 14
<212> DNA
<213> Homo sapiens

<400> 14

```
        atgcattggg gaaccctgtg cggattcttg tggctttggc cctatctttt ctatgtccaa 60
        gct                                                                63
```

<210> 15
<212> DNA
<213> Artificial Sequence

<220>
<223> Description of Artificial Sequence: modified human leptin signal peptide DNA

<400> 15

```
        atgcattggg gaaccctgtg cggattcttg tggctttggc cctatctttt ctatgtttcg 60
        cccagc                                                              66
```

<210> 16
<211> 63
<212> DNA
<213> Artificial Sequence

<220>
<223> Description of Artificial Sequence: modified human leptin signal peptide DNA

<400> 16

```
atgcattggg gaaccctgtg cggattcttg tggctttggc cctatctttt ctatgtttcg 60
ccc                                                                63
```

<210> 17
<211> 66
<212> DNA
<213> Artificial Sequence

<220>
<223> Description of Artificial Sequence: modified human leptin signal peptide DNA

<400> 17

```
atgcattggg gaaccctgtg cggattcttg tggctttggc cctatctttt ctatgtttcg 60
cccgct                                                             66
```

<210> 18
<211> 66
<212> DNA
<213> Artificial Sequence

<220>
<223> Description of Artificial Sequence: modified human leptin signal peptide DNA

<400> 18

```
atgcattggg gaaccctgtg cggattcttg tggctttggc cctatctttt ctatgtttcg 60
aacagc                                                             66
```

<210> 19
<211> 68
<212> DNA
<213> Homo sapiens

<400> 19

```
atggatgcaa tgaagagagg gctctgctgt gtgctgctgc tgtgtggagc agtcttcgtt 60
tcgcccag                                                           68
```

<210> 20
<211> 66
<212> DNA
<213> Homo sapiens

<400> 20

```
atggatgcaa tgaagagagg gctctgctgt gtgctgctgc tgtgtggagc agtcttcgtt 60
tcgccc                                                             66
```

<210> 21
<211> 69
<212> DNA
<213> Artificial Sequence

<220>
<223> Description of Artificial Sequence: modified human tPA signal peptide DNA

<400> 21

```
atggatgcaa tgaagagagg gctctgctgt gtgctgctgc tgtgtggagc agtcttcgtt 60
tcgaacagc                                                          69
```

<210> 22
<211> 69
<212> DNA
<213> Artificial Sequence

<220>
<223> Description of Artificial Sequence: modified human tPA signal peptide DNA

<400> 22

```
atggatgcaa tgaagagagg gctctgctgt gtgctgctgc tgtgtggagc agtcttcgtt 60
```

```
tcgcccgct                                                          69
```

<210> 23
<211> 63
<212> DNA
<213> Artificial Sequence

<220>
<223> Description of Artificial Sequence: leptin/tPA signal peptide DNA

<400> 23

```
atgcattggg gaaccctgtg ctgtgtgctg ctgctgtgtg gagcagtctt cgtttcgccc 60
agc                                                                63
```

<210> 24
<211> 60
<212> DNA
<213> Artificial Sequence

<220>
<223> Description of Artificial Sequence: leptin/tPA signal peptide DNA

<400> 24

```
atgcattggg gaaccctgtg ctgtgtgctg ctgctgtgtg gagcagtctt cgtttcgccc 60
```

<210> 25
<211> 438

<212> DNA
<213> Artificial Sequence

<220>
<223> Description of Artificial Sequence: leptin 2, 47, 69 DNA

<400> 25

```
gtgaacatca caaaagtcca agatgacacc aaaaccctca tcaagacaat tgtcaccagg 60
atcaatgaca tttcacacac gcagtcagtc tcctccaaac agaaagtcac cggtttggac 120
ttcattcctg ggctccacaa catcacgacc ttatccaaga tggaccagac actggcagtc 180
taccaacaga tcctcaccag tatgaattcc acaaacgtga tccaaatatc caacgacctg 240
gagaacctcc gggatcttct tcacgtgctg gccttctcta agagctgcca cttgccctgg 300
gccagtggcc tggagacctt ggacagcctg gggggtgtcc tggaagcttc aggctactcc 360
acagaggtgg tggccctgag caggctgcag gggtctctgc aggacatgct gtggcagctg 420
gacctaagcc ctgggtgc                                               438
```

<210> 26
<211> 146
<212> PRT
<213> Artificial Sequence

<220>
<223> Description of Artificial Sequence: leptin 2, 47, 69 protein

<400> 26

```
Val Asn Ile Thr Lys Val Gln Asp Asp Thr Lys Thr Leu Ile Lys Thr
 1               5                  10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Asn Ile
        35                  40                  45

Thr Thr Leu Ser Lys Met Asp Gln Thr Leu Ala Val Tyr Gln Gln Ile
    50                  55                  60

Leu Thr Ser Met Asn Ser Thr Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
                100                 105                 110

Val Leu Glu Ala Ser Gly Tyr Ser Thr Glu Val Val Ala Leu Ser Arg
                115                 120                 125

Leu Gln Gly Ser Leu Gln Asp Met Leu Trp Gln Leu Asp Leu Ser Pro
            130                 135                 140

Gly Cys
145
```

<210> 27

<211> 438
<212> DNA
<213> Artificial Sequence

<220>
<223> Description of Artificial Sequence: leptin 2, 47, 69, 92 DNA

<400> 27

```
gtgaacatca caaaagtcca agatgacacc aaaaccctca tcaagacaat tgtcaccagg 60
atcaatgaca tttcacacac gcagtcagtc tcctccaaac agaaagtcac cggtttggac 120
ttcattcctg ggctccacaa catcacgacc ttatccaaga tggaccagac actggcagtc 180
taccaacaga tcctcaccag tatgaattcc acaaacgtga tccaaatatc caacgacctg 240
gagaacctcc gggatcttct tcacgtgctg gccaactcta ccagctgcca cttgccctgg 300
gccagtggcc tggagacctt ggacagcctg gggggtgtcc tggaagcttc aggctactcc 360
acagaggtgg tggccctgag caggctgcag gggtctctgc aggacatgct gtggcagctg 420
gacctcagcc ctgggtgc                                                438
```

<210> 28
<211> 146
<212> PRT
<213> Artificial Sequence

<220>
<223> Description of Artificial Sequence: leptin 2, 47, 69, 92 protein

<400> 28

```
Val Asn Ile Thr Lys Val Gln Asp Asp Thr Lys Thr Leu Ile Lys Thr
 1               5                  10                  15

Ile Val Thr Arg Ile Asn Asp Ile Ser His Thr Gln Ser Val Ser Ser
            20              25                  30

Lys Gln Lys Val Thr Gly Leu Asp Phe Ile Pro Gly Leu His Asn Ile
        35                  40                  45

Thr Thr Leu Ser Lys Met Asp Gln Thr Leu Ala Val Tyr Gln Gln Ile
    50                  55                  60

Leu Thr Ser Met Asn Ser Thr Asn Val Ile Gln Ile Ser Asn Asp Leu
65                  70                  75                  80

Glu Asn Leu Arg Asp Leu Leu His Val Leu Ala Asn Ser Thr Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
            115                 120                 125

Leu Gln Gly Ser Leu Gln Asp Met Leu Trp Gln Leu Asp Leu Ser Pro
    130                 135                 140

Gly Cys
145
```

<210> 29
<211> 438

<212> DNA
<213> Artificial Sequence

<220>
<223> Description of Artificial Sequence: leptin 2, 47, 69, 102 DNA

<400> 29

```
gtgaacatca caaaagtcca agatgacacc aaaaccctca tcaagacaat tgtcaccagg 60
atcaatgaca tttcacacac gcagtcagtc tcctccaaac agaaagtcac cggtttggac 120
ttcattcctg ggctccacaa catcacgacc ttatccaaga tggaccagac actggcagtc 180
taccaacaga tcctcaccag tatgaattcc acaaacgtga tccaaatatc caacgacctg 240
gagaacctcc gggatcttct tcacgtgctg gccttctcta agagctgcca cttgccctgg 300
gccaatggca cggagacctt ggacagcctg gggggtgtcc tggaagcttc aggctactcc 360
acagaggtgg tggccctgag caggctgcag gggtctctgc aggacatgct gtggcagctg 420
gacctcagcc ctgggtgc                                                 438
```

<210> 30
<211> 146
<212> PRT
<213> Artificial Sequence

<220>
<223> Description of Artificial Sequence: leptin 2, 47, 69, 102 protein

<400> 30

```
Val Asn Ile Thr Lys Val Gln Asp Asp Thr Lys Thr Leu Ile Lys Thr
 1               5                  10                  15

Ile Val Thr Arg Ile Asn Asp Ile Ser His Thr Gln Ser Val Ser Ser
             20                  25                  30

Lys Gln Lys Val Thr Gly Leu Asp Phe Ile Pro Gly Leu His Asn Ile
         35                  40                  45

Thr Thr Leu Ser Lys Met Asp Gln Thr Leu Ala Val Tyr Gln Gln Ile
```

```
        50                  55                  60

Leu Thr Ser Met Asn Ser Thr Asn Val Ile Gln Ile Ser Asn Asp Leu
 65                  70                  75                  80

Glu Asn Leu Arg Asp Leu Leu His Val Leu Ala Phe Ser Lys Ser Cys
                 85                  90                  95

His Leu Pro Trp Ala Asn Gly Thr Glu Thr Leu Asp Ser Leu Gly Gly
            100                 105                 110

Val Leu Glu Ala Ser Gly Tyr Ser Thr Glu Val Val Ala Leu Ser Arg
            115                 120                 125

Leu Gln Gly Ser Leu Gln Asp Met Leu Trp Gln Leu Asp Leu Ser Pro
        130                 135                 140

Gly Cys
145
```

EP 1 151 102 B1

<210> 31
<211> 438
<212> DNA
<213> Artificial Sequence

<220>
<223> Description of Artificial Sequence: leptin 47, 69, 102 DNA

<400> 31

```
gtgcccatcc aaaaagtcca agatgacacc aaaaccctca tcaagacaat tgtcaccagg 60
atcaatgaca tttcacacac gcagtcagtc tcctccaaac agaaagtcac cggtttggac 120
ttcattcctg ggctccacaa catcacgacc ttatccaaga tggaccagac actggcagtc 180
taccaacaga tcctcaccag tatgaattcc acaaacgtga tccaaatatc caacgacctg 240
gagaacctcc gggatcttct tcacgtgctg gccttctcta agagctgcca cttgccctgg 300
gccaatggca cggagacctt ggacagcctg gggggtgtcc tggaagcttc aggctactcc 360
acagaggtgg tggccctgag caggctgcag gggtctctgc aggacatgct gtggcagctg 420
gacctcagcc ctgggtgc                                                 438
```

<210> 32
<211> 146
<212> PRT
<213> Artificial Sequence

<220>
<223> Description of Artificial Sequence: leptin 47, 69, 102 protein

<400> 32

```
Val Pro Ile Gln Lys Val Gln Asp Asp Thr Lys Thr Leu Ile Lys Thr
 1               5                  10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Asn Ile
        35                  40                  45

Thr Thr Leu Ser Lys Met Asp Gln Thr Leu Ala Val Tyr Gln Gln Ile
    50                  55                  60

Leu Thr Ser Met Asn Ser Thr Asn Val Ile Gln Ile Ser Asn Asp Leu
65                  70                  75                  80

Glu Asn Leu Arg Asp Leu Leu His Val Leu Ala Phe Ser Lys Ser Cys
                    85                  90                  95

His Leu Pro Trp Ala Asn Gly Thr Glu Thr Leu Asp Ser Leu Gly Gly
                100                 105                 110

Val Leu Glu Ala Ser Gly Tyr Ser Thr Glu Val Val Ala Leu Ser Arg
            115                 120                 125

Leu Gln Gly Ser Leu Gln Asp Met Leu Trp Gln Leu Asp Leu Ser Pro
        130                 135                 140

Gly Cys
145
```

<210> 33
<211> 438
<212> DNA
<213> Artificial Sequence

<220>
<223> Description of Artificial Sequence: leptin 2, 47, 69, 92, 102 DNA

<400> 33

```
gtgaacatca caaaagtcca agatgacacc aaaaccctca tcaagacaat tgtcaccagg 60
atcaatgaca tttcacacac gcagtcagtc tcctccaaac agaaagtcac cggtttggac 120
ttcattcctg ggctccacaa catcacgacc ttatccaaga tggaccagac actggcagtc 180
taccaacaga tcctcaccag tatgaattcc acaaacgtga tccaaatatc caacgacctg 240
gagaacctcc gggatcttct tcacgtgctg gccaactcta ccagctgcca cttgcccctg 300
gccaatggca cggagacctt ggacagcctg gggggtgtcc tggaagcttc aggctactcc 360
acagaggtgg tggccctgag caggctgcag gggtctctgc aggacatgct gtggcagctg 420
gacctcagcc ctgggtgc                                                  438
```

<210> 34
<211> 146
<212> PRT
<213> Artificial Sequence

<220>
<223> Description of Artificial Sequence: leptin 2, 47, 69, 92, 102 protein

<400> 34

```
Val Asn Ile Thr Lys Val Gln Asp Asp Thr Lys Thr Leu Ile Lys Thr
  1           5               10               15

Ile Val Thr Arg Ile Asn Asp Ile Ser His Thr Gln Ser Val Ser Ser
         20               25               30

Lys Gln Lys Val Thr Gly Leu Asp Phe Ile Pro Gly Leu His Asn Ile
         35               40               45

Thr Thr Leu Ser Lys Met Asp Gln Thr Leu Ala Val Tyr Gln Gln Ile
      50               55               60

Leu Thr Ser Met Asn Ser Thr Asn Val Ile Gln Ile Ser Asn Asp Leu
   65               70               75               80

Glu Asn Leu Arg Asp Leu Leu His Val Leu Ala Asn Ser Thr Ser Cys
                 85               90               95

His Leu Pro Trp Ala Asn Gly Thr Glu Thr Leu Asp Ser Leu Gly Gly
            100              105              110

Val Leu Glu Ala Ser Gly Tyr Ser Thr Glu Val Val Ala Leu Ser Arg
         115              120              125

Leu Gln Gly Ser Leu Gln Asp Met Leu Trp Gln Leu Asp Leu Ser Pro
      130              135              140

Gly Cys
145
```

<210> 35
<211> 438
<212> DNA
<213> Artificial Sequence

<220>
<223> Description of Artificial Sequence: leptin 47, 69, 92, 102 DNA

<400> 35

```
gtgcccatcc aaaaagtcca agatgacacc aaaaccctca tcaagacaat tgtcaccagg   60
atcaatgaca tttcacacac gcagtcagtc tcctccaaac agaaagtcac cggtttggac  120
ttcattcctg ggctccacaa catcacgacc ttatccaaga tggaccagac actggcagtc  180
taccaacaga tcctcaccag tatgaattcc acaaacgtga tccaaatatc caacgacctg  240
gagaacctcc gggatcttct tcacgtgctg gccaactcta ccagctgcca cttgccctgg  300
gccaatggca cggagacctt ggacagcctg gggggtgtcc tggaagcttc aggctactcc  360
acagaggtgg tggccctgag caggctgcag gggtctctgc aggacatgct gtggcagctg  420
gacctcagcc ctgggtgc                                                438
```

<210> 36
<211> 146
<212> PRT
<213> Artificial Sequence

<220>
<223> Description of Artificial Sequence: leptin 47, 69, 92, 102 protein

<400> 36

```
Val Pro Ile Gln Lys Val Gln Asp Asp Thr Lys Thr Leu Ile Lys Thr
 1               5                  10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Asn Ile
            35                  40                  45

Thr Thr Leu Ser Lys Met Asp Gln Thr Leu Ala Val Tyr Gln Gln Ile
        50                  55                  60

Leu Thr Ser Met Asn Ser Thr Asn Val Ile Gln Ile Ser Asn Asp Leu
    65                  70                  75                  80

Glu Asn Leu Arg Asp Leu Leu His Val Leu Ala Asn Ser Thr Ser Cys
                85                  90                  95

His Leu Pro Trp Ala Asn Gly Thr Glu Thr Leu Asp Ser Leu Gly Gly
```

```
                    100                105                110
        Val Leu Glu Ala Ser Gly Tyr Ser Thr Glu Val Val Ala Leu Ser Arg
                115                120                125

        Leu Gln Gly Ser Leu Gln Asp Met Leu Trp Gln Leu Asp Leu Ser Pro
            130                135                140

        Gly Cys
        145
```

**Description**

Field of the Invention

The present invention relates to glycosylated leptin compositions and related methods. Included are glycosylated leptin proteins having a Stokes' radius allowing for improved properties, as well as glycosylated leptin proteins having selected sites for glycosylation, nucleic acids encoding such proteins, related host cells, vectors, processes for production, and methods of use of such compositions. Novel methods of producing glycosylated proteins are also provided.

Background

Although the molecular basis for obesity is largely unknown, the identification of the "OB gene" and protein encoded ("OB protein," also referred to herein as "leptin") has shed some light on mechanisms the body uses to regulate body fat deposition. Zhang et al., Nature 372: 425-432 (1994) incorporated herein by reference; see also, the Correction at Nature 374: 479 (1995) also incorporated herein by reference. The OB protein is active in vivo in both *ob/ob* mutant mice (mice obese due to a defect in the production of the OB gene product) as well as in normal, wild type mice. The biological activity manifests itself in, among other things, weight loss. See generally, Barinaga, "Obese" Protein Slims Mice, Science 269: 475-476 (1995). See PCT International Publication Number WO96/05309, "Modulators of Body Weight, Corresponding Nucleic Acids and Proteins, and Diagnostic and Therapeutic Uses Thereof," herein incorporated by reference in its entirety. See also, PCT International Publication Numbers WO96/40912, WO97/06816, WO97/18833, WO97/38014, WO98/08512, and WO98/28427, all of which describe OB methods and compositions in greater detail and are all herein incorporated by reference in their entirety.

The other biological effects of OB protein are not well characterized. See generally, Friedman et al., Nature 395: 763-770 (October 1998) for a review of leptin and the regulation of body weight in mammals, herein incorporated by reference. It is known, for instance, that in *ob/ob* mutant mice, administration of OB protein results in a decrease in serum insulin levels, and serum glucose levels. It is also known that administration of OB protein results in a decrease in body fat. This was observed in both *ob/ob* mutant mice, as well as non-obese normal mice. Pelleymounter et al., Science 269: 540-543 (1995); Halaas et al., Science 269: 543-546 (1995). See also, Campfield et al., Science 269: 546-549 (1995) (Peripheral and central administration of microgram doses of OB protein reduced food intake and body weight of *ob/ob* and diet-induced obese mice but not in *db/db* obese mice.) In none of these reports have toxicities been observed, even at the highest doses.

Recombinant leptin is effective in humans to result in weight loss. Greenberg AS, Heymsfield SB, Fujioka K, et al., Preliminary safety and efficacy of recombinant methionyl human leptin (rL) administered by SC injection in lean and obese subjects. Poster presented at: 58th Annual Meeting and Scientific Sessions of the American Diabetes Association; June 14, 1998; Chicago, IL, herein incorporated by reference. As has been demonstrated, administration of recombinant methionyl human leptin to obese humans has resulted in weight loss without toxicities. Further, the weight that is lost is predominantly fat. Heymsfield et al., Weight and body composition changes in lean and obese subjects treated with recombinant methionyl human leptin. Poster presented at: International Congress on Obesity; August 29 - September 3, 1998; Paris, France, herein incorporated by reference.

Native human leptin is known to have a relatively fast half life in humans. Lau et al., Pharmacokinetics of recombinant methionyl human leptin and the effect of antibody formation in lean and obese subjects following subcutaneous dosing. Poster presented at: International Congress on Obesity; August 29-September 3, 1998, Paris, France, herein incorporated by reference. In the systemic circulation, accumulation may be accomplished either by giving larger doses or more frequent doses of the subject protein. Reports indicate that exogenous, as well as endogenous leptin is removed from the circulation, at least in part, by the kidney. See, e.g., Cumin et al., Journal of Endocrinology, 155: 577-585 (1997) and Cumin et al., Internal Journal of Obesity 21: 495-504 (1997), both herein incorporated by reference.

In general, the kidney functions to clear the blood plasma of certain substances by concentrating them in the urine. See, e.g., Harth, The Function of the Kidneys, In: Human Physiology, Schmidt et al., eds., Springer-Verlag New York, Heidelberg, Berlin, 1983 at pp. 610-642, herein incorporated by reference. The rate or degree to which a serum protein may pass through the kidney is difficult to estimate, but, in general, the kidney anatomy allows for free passage of water and small solutes, but imposes a barrier to the passage of plasma proteins. Different substances have different "filterability", kidney clearance rates, see, Anderson et al., Renal and Systemic Manifestations of Glomerular Disease, In: The Kidney, Brener et al., eds., Harcort Brace Joanovich, Inc., Philadelphia, PA 1991 at pages 1831-1843, herein incorporated by reference.

Leptin may be accumulated in the systemic circulation by continuous administration, such as by osmotic pump or by chemically derivatizing the protein so that the circulation time is increased. See, e.g., PCT WO96/40912, published December 19, 1996 and herein incorporated by reference in its entirety. Chemical derivatization of a recombinantly-produced protein generally requires, however, a two (or more) step process: step one, make the protein; step two, add a chemical moiety (such as a polyethylene glycol or dextran moiety), see, e.g., PCT WO96/40912, supra, at page 8 et seg. for a description of N-terminally derivatizing leptin (therein referred to as OB Protein).

For a "one step" process, in a recombinant DNA system, one may encode a fusion protein (alternatively called a "chimeric" protein) where an additional polypeptide moiety is encoded along with the desired protein, so that both are expressed. Lengthening the protein may also increase circulation time. Polypeptides such as the "Fc" portion of an antibody, or albumin have been used in this regard. See e.g., PCT WO 98/28427, herein incorporated by reference, entitled, "OB Fusion Protein Compositions and Methods." The general disadvantage in manufacturing is that larger expression products are sometimes more difficult to fold into proper conformations, and yields may be lower than those for smaller products. Further, the overall protein load per dose is increased, and the proportion of therapeutic protein is decreased, with the use of increasingly large sized fusion proteins.

The presence of carbohydrate on a protein can affect its clearance rate and may improve its potency in vivo, while at the same time it can affect the protein's intrinsic activity, solubility, stability and immunogenicity. See, e.g. , European Patent Publication 0 640 619, published March 1, 1995, entitled, "Erythropoietin analogs with additional glycosylation sites," herein incorporated by reference, and PCT patent publication WO 96/25498, published August 22, 1996, entitled, "MPL Ligand Analogs" both herein incorporated by reference.

Furthermore, carbohydrate may be added by eukaryotic cell production, without the need for a two-step process. E.g., PCT/US96/06609, published November 14, 1996, herein incorporated by reference, proposes various mammalian signal sequences for secretion of an ob protein from a mammalian cell (at pages 11-12, for example). See also, PCT WO 97/20933, published June 12, 1997, entitled, "Mutational Variants of Mammalian OB Gene Proteins," particularly at page 11, which proposes OB protein glycosylation alterations. Glycosylation occurs at specific locations along the polypeptide backbone. There are usually two types of glycosylation: O-linked oligosaccharides are attached to serine or threonine residues while N-linked oligosaccharides are attached to asparagine residues when they are a part of the sequence Asn-X-Ser/Thr, where X can be any amino acid except proline, The structures of N-linked and O-linked oligosaccharides and the sugar residues found in each type are different. One type of sugar that is commonly found on both is N-acetylneuraminic acid (hereafter referred co as sialic acid). Sialic acid is usually the terminal residue of both N-linked and O-linked oligosaccharides in mammalian cells and, by virtue of its negative charge, may confer acidic properties to the glycoprotein. The predominate form of naturally occurring human leptin (provided in human cells) is not glycosylated. A variant of the naturally occurring protein having a glutamine absent at position 28 of the mature protein (SEQ ID NO. 2, infra) does contain two sites for glycosylation. These sites are both for O-linked glycosylation. It is believed, however, that this form is produced in only trace amounts in humans, and is not the predominant active form in vivo.

It would be desirable to have a process, which results in a leptin having an increased systemic circulation time, which does not require such second derivatization step as described above. It is further desirable co increase the intrinsic activity, and solubility of leptin, without causing or increasing immunogenicity or other detrimental effects.

WO-A-9720933 discloses mutational variants of mammalian Ob gene proteins. WO-A-9505465 discloses erythropoietin analogs. WO-A-9726916 discloses obesity protein analog compounds and formulations thereof.

Summary of the Invention

The present invention stems from the observation that, as compared to unaltered native recombinant human leptin, glycosylated leptin protein is functional in vivo, and, further, certain forms of glycosylated leptin protein have longer systemic circulation times in vivo, without toxicities.

It has been found, surprisingly and importantly, that a glycosylated human leptin having a single N-linked glycosylation site has in vitro as well as in vivo biological activity. Further, the biological activity is equal to or is slightly more potent than recombinant human native leptin protein. As indicated above, leptin's effect on obesity is thought to be due, in part, by action in the brain. As indicated above, leptin is not a naturally glycosylated molecule (in the Q+28 form, SEQ ID NO. 1, infra, which is believed to be the predominant form in human serum). Further, glycosylated proteins (glycoproteins) generally may not enter the brain because of an inability to cross the blood-brain barrier. Demonstration of equal (or slightly better) biological activity by glycosylated leptin demonstrates either that (a) the glycosylated leptin enters the brain, or (b)if it doesn't, glycosylated leptin is more biologically effective in the peripheral tissues (such as visceral areas of adipose tissue)than native recombinant human leptin.

It has further been observed that a human leptin which is N-glycosylated at three sites has a far longer circulation time and potency than recombinant native human leptin or leptin N-glycosylated at a single site. As set forth in the working examples below, various two-, three-, four-, and five- site glycosylated leptins have been prepared and tested for in vitro, and in some cases, in vivo activity.

The present glycosylated leptins may have a desired, relatively long plasma half life. The present glycosylated leptins which have Stokes' radius of greater than or equal to 30Å have a reduced rate of filterability through the membranes, and thus a reduced rate of degradation in the kidney.

Although Stokes' radii may be determined in a variety of ways, the preferred way herein is to use gel filtration chromatography. See generally, Le Maire et al., Analytical Biochemistry 154: 525-535 (1986) herein incorporated by reference, for gel filtration chromatography for determining the Stokes' radii of various proteins for use as standards. Thus, the present glycosylated leptins are those which have a Stokes' radii of about 30 Å when determined using gel filtration chromatography.

It is preferred that the present glycosylated leptins also substantially avoid clearance in the liver. The liver is known to have receptors which bind galactose. Galactose is a sugar and may be a component of the carbohydrate moiety on the present glycosylated leptins. Sialic acid will typically "cap" the galactose moiety, and prevent its reactivity with galactose receptors in the liver. Additionally, a sialic acid moiety imparts a negative charge. The more negatively charged the present glycosylated leptins are, the more they will "repel" the negatively charged membranes of the liver and kidney. Therefore, the present glycosylated leptin proteins are preferably those having at least a majority of the galactose moieties unavailable for binding to a galactose receptor, and more preferably, having a sialic acid moiety located at least a majority of the sites available for sialation.

As discussed herein, the recombinant human leptin modified to contain sites for N-linked glycosylation at one site or at three sites, demonstrated that glycosylated leptin protein could be functional, and could be as functional as natural human. Glycosylation was accomplished via host cellular machinery, in cell culture, and therefore did not require an extra process step (as required to derivative protein) to attain the desired characteristics of longer serum half life.

Thus, in one aspect, the present invention relates to a glycosylated leptin protein according to the accompanying claims, having a Stokes' radius greater than that of naturally occurring glycosylated human leptin of SEQ ID NO. 2 (rHu-Leptin 1-145, below). In another aspect, the present invention relates to a glycosylated leptin protein having a Stokes' radius greater than that of a glycosylated leptin protein having one N-linked glycosylation moiety. And, in yet another aspect, the present invention relates to a glycosylated leptin protein having a Stokes' radius equal to or greater than 30 Å, as determined by gel filtration.

The present claimed invention also relates to leptin proteins having at least one additional glycosylation site. In yet another way, the present invention relates to a glycosylated leptin protein having five or greater than five sialic acid moieties. The naturally occurring human leptin variant (SEQ. ID NO: 2, below) contains 2 sites for O-linked glycosylation, and therefore may contain 4 sialic acid moieties. The present working examples demonstrate that more heavily glycosylated leptin protein has substantially improved circulation time. Further, the present invention relates to a glycosylated leptin protein having five, six or seven sialic acid moieties.

In other aspects, the present invention relates to a nucleic acid encoding a glycosylated leptin protein as set forth herein, as well as a vector containing a nucleic acid encoding a glycosylated leptin protein according to the disclosure herein.

Thus, in yet other aspects, the present invention relates to a host cell containing a nucleic acid encoding a glycosylated leptin protein according to the present disclosure.

The present disclosure also relates to use of the present nucleic acids for gene therapy. Further, the present invention relates to a method of preparing a glycosylated leptin protein.

The present invention also relates to selective binding molecules, such as antibodies which selectively bind the present glycosylated leptin proteins.

In other aspects more fully described below, the present invention relates to a pharmaceutical compositions comprising a glycosylated lepcin protein of the present invention in a pharmaceutically acceptable carrier. The present disclosure also relates to a method of treatment of a human for a condition selected from among obesity, diabetes, and high blood lipid content effects; said method comprising administering an effective amount of a glycosylated human leptin according to the present invention.

The present invention also relates to improved methods of production of glycosylated leptin proteins, as well as production of glycosylated proteins generally. The present working examples demonstrate that for the present glycosylated leptin proteins, use of a signal peptide other than the native human leptin signal peptide improve glycosylation efficiency. In this case, improved glycosylation efficiency results in the desirable property of both increased number and size of added carbohydrate chains. Thus, the present compositions and methods include the use of signal peptides other than the native leptin signal peptide. Apart from the native human leptin signal, particular signal peptides, both those known to be naturally found (i.e., natural signal peptides are those which have not been genetically manipulated by humans, by any means including homologous recombination, recombinant DNA techniques, or other means known or expected to alter the nucleic acid sequence constituents, although the cell containing them may have been cultured or otherwise removed from its natural in vivo environment), as well as those not found in nature (i.e., non-natural signal peptides are those which have been genetically manipulated by humans as described above), are set forth below.

The present invention further relates to the observation that modification of signal peptides, as well as other peptides which are processed off of the mature protein, improve the yield of glycosylated proteins. Signal peptide modifications include alteration of the peptidase cleavage site to improve cleavage accuracy (and thus produce a larger yield of desired glycosylated proteins having the predicted N-terminal amino acid sequence). Signal peptide modifications may also, or alternatively, largely improve glycosylation efficiency, even in the absence of "correct" cleavage of the mature protein from the presequences. ("Correct" indicating that the first amino acid on the N-terminus is one for the predicted mature protein, not having any amino acids found on the signal peptide or other presequences). Other modifications include the addition of "prosequences" which are also cleaved off but also generate improved glycosylation efficiency. Natural as well as non-naturally occurring signal peptides may be modified as such. Specific examples are provided herein.

Therefore, the present invention also relates to an improved method of manufacturing a glycosylated protein comprising:

(a) culturing, under suitable conditions for expression and glycosylation, a host cell containing a DNA sequence encoding, in the 5' to 3' direction (i) a signal peptide, and (ii) a DNA encoding a glycosylated protein; and
(b) obtaining said glycosylated protein wherein said improvement comprises use of a signal peptide having a peptidase cleavage site optimized for maximizing yield of said glycosylated protein, and, optionally, the addition of a prosequence.

Brief Description of the Figures

FIGURE 1 is a graph showing weight loss relative to buffer control for animals dosed with various doses of a one-site glycosylated leptin ("Glycosylated CHO Leptin) and non-glycosylated rmetHu-Leptinl-146 ("Leptin").
FIGURE 2 is a Western Blot, as further described in Examples 5 and 6, below, showing that alterations in amino acid sequence of the glycosylation site can alter the kind or amount of glycosylation.
FIGURE 3 is a graph of serum leptin concentrations following subcutaneous administration of 1.0 mg/kg rmetHu-Leptin or a three-site glycosylated leptin protein in male CD-1 mice as further described in Example 7.
FIGURE 4 is a graph of serum leptin concentrations following intravenous administration of 1.0 mg/kg rmetHu-Leptin or a three-site glycosylated leptin protein in male CD-1 mice as further described in Example 7.
FIGURE 5 is a graph of weight loss upon administration of a three-site glycosylated leptin protein ("GE-Leptin") as further described in Example 8
FIGURE 6 is a graph of food intake upon administration of a three-site glycosylated leptin protein ("GE-Leptin") as further described in Example 9.
FIGURE 7 is a Western Blot illustrating the effects of various signal peptides on expression and glycosylation of a three-site glycosylated leptin protein, as further described in Example 14.
FIGURE 8 is a Western Blot illustrating the effects of various signal peptides, and other peptides, on the glycosylation of a three-site glycosylated leptin, as further described in Example 14.
FIGURE 9 is a Western Blot illustrating the effects of the peptidase cleavage site on glycosylation of a three-site glycosylated leptin protein as further described in Example 14.
FIGURE 10 is a Western Blot illustrating the effects of various signal peptides and other peptides on the glycosylation of a three-site glycosylated leptin as further described in Example 14.
FIGURE 11 is a Western Blot, as described in Example 15 below, and shows that increasing the number of glycosylation sites, at least up to five sites, increases the amount of glycosylation found on the leptin protein when expressed in CHO cells.

Detailed Description of the Invention

As indicated above, the present invention relates, in one aspect, to glycosylated leptin proteins having a Stokes' radius larger than that of naturally occurring glycosylated human leptin. Preferably for increasing half life of a therapeutic composition in the systemic circulation of the Stoke's radius is of sufficient size to reduce filterability in the kidney. The effect of having a Stokes' radius of this size is to keep the glycosylated leptin protein in the systemic circulation for a longer period of time than would be for a glycosylated, or other leptin protein, not having this effective size. Upon empirical determination of the Stokes' radius for the present glycosylated leptin protein, the size must be greater than or equal to about 30 Å, as determined by methods described in further detail below. When used with reference to an individual glycosylated leptin protein molecule, the term "about" means the average Stokes' radius over a period of time for that individual glycosylated leptin protein molecule.

As provided herein, glycosylated leptin proteins having a Stoke's radius greater than naturally occurring leptin proteins has improved properties. The preferred Stokes' radius for a population of glycosylated leptin protein molecules, such as is present in a therapeutically effective dose, is that which is greater than or equal to about 30 Å. The term "about" here indicating that of any population of glycosylated leptin protein molecules, some may have a greater Stokes' radius, some may have a lower Stokes' radius, but the mean Stokes' radius of a given population of glycosylated leptin protein is greater than or equal to 30 Å.

The higher above 30 Å Stokes' radius, the larger the effective size of the glycosylated leptin protein molecule (s). The larger the effective size, i.e., the larger the hydrodynamic volume attained by the addition of oligosaccharide, the slower the effect movement through basement membranes throughout the body. In order for leptin to reach the kidney tubules where it is degraded it must first pass through the basement membranes of the glomerulus. Thus, increasing the hydrodynamic size slows filtration through the glomerular membrane, and therefore slows degradation, and thus ultimate clearance, of the polypeptide in the proximal tubules. For example, the present 3-glycosylation site leptin, rHu-Leptin 1-146 with glycosylation sites at position 47, 69, and 102 (i.e., having an asparagine residue substituted at positions 47, 69 and 102, and a threonine residue substituted at position 29, 71 and 104) has a mean Stokes' radius of 32.1 Å (based on two gel filtration measurements of 31.9 Å and 32.3 Å). The working example below demonstrates that this glycosylated leptin exhibited a 4- to 5-fold decrease in systemic clearance and increase in half-life compared to rmetHu-Leptin.

Also as indicated above, there are several ways to determine the Stokes' radius of a molecule. The present Stokes' radius, for purposes of the present glycosylated leptin proteins, is determined using gel filtration, see, Le Maire et al., supra, see also, Kyte, Structure in Protein Chemistry, Garland Publishing, Inc., New York and London, 1995 at pages 293-316, herein incorporated by reference. Presently, the gel filtration used to determine Stokes' radius is polymer (agarose) beads to which dextran is covalently bound. Commercial preparations include Superdex[tm] 200 HR 10/30 (Pharmacia) and Sephacryl®S-200 high Resolution (Pharmacia). These two preparations were alternatively used to determine the Stokes' radius of the present glycosylated leptin proteins.

A column may be of any size but a size of approximately 1 x 30 cm is preferred for ease in handling. The instruction manuals for column preparation for each of the above gel filtration substances are herein incorporated by reference in their entirety (Paper number 71-7059-00 Edition AB for Superdex [tm] and 52-2086-00-03, for Sephacryl®).

The buffer to be used should be fairly close to a physiological buffer that does not significantly alter the solution conformation of the molecule and interfere with the size separation of the protein molecules. Phosphate buffered saline is preferred, and was used to determine the Stokes' radius of the present glycosylated leptin proteins.

The process for performing gel filtration should generally follow the instruction manuals as above incorporated. The selected glycosylated leptin protein for which a Stokes' radius is to be determined should be loaded onto the column. Below, for example, a concentration of 0.4 A280/ml, which is about 0.45 mg/ml, was used for a three-site glycosylated leptin (47,49,102). The buffers used herein was PBS, but other buffers may be used. The buffer for the load should be compatible with good gel filtration practices and could in theory contain high salts, and other materials consistent with what one skilled in the art would consider appropriate. The load or storage buffer should not interfere (either by precipitating as it hits the column or by being denaturing and requiring a refold as it elutes) with determination of the Stokes radius. A column of gel filtration substances which has not previously been used, is preferred. The washing buffer, such as phosphate buffered saline, should be applied at a rate of 0.25 ml/min or a linear flow rate of 0.3 cm/min. This value will be determined by the properties of the gel and is basically per the manufacturers instructions. The fractions eluted contain the glycosylated leptin protein molecules which are not trapped in the gel filtration substance.

To determine the Stokes' radius, it is necessary to compare the test glycosylated leptin protein to known proteins used to calibrate the gel filtration column. The methods as in the Gel Filtration Calibration Kit Instruction Manual, (Pharmacia Biotech paper 11-B-033-07, Rev.2), are herein incorporated by reference. Generally, selected proteins of known Stokes' radius are filtered through the column, and the fraction where each eluted is noted. The fraction containing the subject glycosylated leptin protein is compared to the fraction of the calibrated proteins.

Thus, the glycosylated leptin proteins are those having a Stokes' radius (of the glycosylated leptin protein moiety alone, not including any chemical derivatization which may be further performed, as indicated below) of greater than or equal to 30 Å as determined by gel filtration. The gel filtration may be accomplished using dextran-coated agarose gel filtration substances, such as SuperDex™ or Sepharcryl®, as described above.. The buffer may be phosphate buffered saline.

Leptin Amino Acid Sequences

1. Glycosylation sites. Generally, to prepare the present glycosylated leptin composition, one will begin with a selected amino acid sequence, and modify that sequence to include the addition of sites for N-linked or O-linked glycosylation. The following formula is preferred for adding sites for N-linked glycosylation (see generally, Creighton, Proteins, W.H. Freeman and Company, N.Y., (1984) p. 498, plus index at pages 76-78, incorporated herein by reference):

```
N - X - T/S
```

wherein "N" is Asparagine "X" is any amino acid except proline and "T/S" is Threonine or Serine. Preferred is the formula "N - X - T"; whereby the alteration with respect to a starting leptin amino acid sequence is that "X" remains the same as that for starting leptin sequence (preferably SEQ. ID. NOS. 1 or 2, infra), and the amino acid immediately downstream (toward the C-terminus) is threonine. N-linked sites at the outer surface of the protein are preferred. Surface residues suitable for glycosylation can be identified by examination of a three dimensional structure or model, or by nuclear magnetic resonance or crystal structure (as discussed below). Also, it has been determined that a proline at position -1 with respect to the asparagine residue (i.e., toward the N-terminus) in some glycosylation sites may be detrimental, and one may seek to avoid a proline residue at such a site. Working Examples 5 and 6 demonstrate the effect of glycosylation site occupancy of N - X - S versus N - X - T and adjacent amino acids.

O-linked glycosylation sites are found on the outer surface of proteins generally near or adjacent to proline residues. O-linked sites can be found or introduced by including serine or threonine residues near to or adjacent to proline residues. Generally, threonine residues are preferred. For example, SEQ. ID NO: 1 (below) has a proline at position 99, and a threonine at position 100 was introduced. This leptin was expressed in CHO cells and COS cells, and was O-linked glycosylated.

In addition, one may select to combine N-linked and O-glycosylation sites in the present glycosylated leptin proteins. As described above, one may add one or more O-linked glycosylation site, and, in addition, add one or more N-linked glycosylation sites.

2. Sites for Glycosylation. Generally, one will modify the protein backbone using the above formulas to add an N-linked or O-linked glycosylation site.

In order to select a site along the protein backbone for N-glycosylation, the general rule is that the asparagine residue must be located on an external surface of the protein to be available for adding the carbohydrate moiety. For example, with respect to the three dimensional structure of leptin, the asparagine residue should be on a loop, β-turn, or on an outer surface of an alpha helix. This analysis is based on the current structure of leptin and the structure functional relationship of several cytokines.

When selecting the site for glycosylation, one may consider the leptin's three dimensional conformation. The first several amino acids of leptin are disordered, which indicates a certain amount of flexibility. Topologically, the leptin structure (see, Zhang et al., Nature 387: 206-209 (1997) (reporting the crystal structure of obese protein leptin E-100, herein incorporated by reference)) is similar to the structure of the cytokine, granulocyte colony stimulating factor ("G-CSF")(see, e.g., U.S. Patent No. 5,581,476, Osslund, disclosing the 3-D structure of crystalline rmetHuG-CSF).

Given the apparent flexibility and apparent lack of biological significance of helix A, one may choose to modify SEQ. ID NO: 1 to include glycosylation sites at residues Vall or Pro2.

Asp23 (of SEQ. ID NO: 1) is on the last turn of helix A and is considered a good choice as the side chain is at least partially on the outer surface of the protein.

The proline residue at position 47 and the isoleucine residue at position 48 (of SEQ. ID NO: 1) are at the end of the AB loop, only a couple of residues from the beginning of Helix B. They are on the surface of the protein, and may be suitable for glycosylation site insertion.

The proline residue at position 69 is on the surface of the protein, which is a good position for glycosylation.

The phenylalanine residue at position 92 is at the end of the C helix and its side chain is facing opposite to which may be the receptor binding face. This is likely to yield the best result in that there is the least interference from any glycosylation moiety with receptor binding.

Serine at position 102 is at the protein surface in the middle of the CD loop and should be in a relatively flexible portion of the structure, along with positions 101 (alanine) and 103 (glycine) .

Thus, the present invention relates to a glycosylated leptin protein comprising SEQ. ID NO: 1 (rHu-Leptin 1-146, below) or SEQ. ID NO: 2 (rHu-Leptin 1-145, below) having one or more sequence alterations as a site of glycosylation. Said sequence alterations may be selected from among:

01V->N 02P->A 03I->T or S (i.e., altering the first amino acid in SEQ. ID NO: 1, below, which is a valine, to asparagine, altering the second amino acid from proline to any of the other 19 amino acids (such as alanine), and altering the third amino acid from isoleucine to threonine or serine);

02P->N 03I 04Q->T or S (i.e., altering the second amino acid in SEQ. ID NO: 1, below, which is a proline, to asparagine, maintaining the third amino acid as isoleucine, and altering the fourth amino acid from glutamine to threonine or serine);

23D->N 24I 25S->T or maintain as S (i.e., alter the 23rd amino acid in SEQ. ID NO: 1, below, which is an aspartic acid to asparagine, maintaining the 24th amino acid as isoleucine, and for the 25th amino acid, either maintaining as serine or changing to threonine);

47P->N 48I 49L->T or S (i.e., altering the 47th amino acid from proline to asparagine, maintaining the 48th amino acid as isoleucine, and altering the 49th amino acid from leucine to threonine or serine);

48I->N 49L 50T or T->S (i.e., altering the 48th amino acid from isoleucine to asparagine, maintaining the 49th amino acid as leucine, and maintaining the 50th amino acid as threonine, or altering to serine);

69P->N 70S 71R->T or S (i.e., altering the 69th amino acid in SEQ. ID NO: 1, below, from proline to asparagine, maintaining the 70th amino acid as serine, and altering the 71st amino acid from arginine to threonine);

92F->N 93S 94K->T or S (i.e., altering the 92nd amino acid of SEQ. ID NO: 1, below, from phenylalanine to asparagine, maintaining the 93rd amino acid as a serine, and altering the 94th amino acid from lysine to threonine or serine);

101A->N 102S 103G->T or S (i.e., altering the 101st amino acid in SEQ. ID NO: 1, below, from alanine to asparagine, maintaining the 102nd amino acid as serine, and altering the 103rd amino acid from glycine to threonine or serine).

102S->N 103G 104L->T or S (i.e., altering the 102nd amino acid in SEQ. ID NO: 1, below, from tryptophan to asparagine, maintaining the 103rd amino acid as glycine, and altering the 104th amino acid from leucine to threonine or serine).

103G->N 104 L 105E->T or S (i.e., altering the 103rd amino acid in SEQ. ID NO: 1, below, from glycine to asparagine, maintaining the 104th amino acid as leucine, and altering the 105th amino acid from glutamic acid to threonine or serine).

Thus, the above shorthand notations indicate the amino acid location with respect to SEQ. ID NO: 1, and the change from one amino acid ---> to another amino acid. As indicated below, the change in the third amino acid (the amino acid toward the C-terminus of the protein) to threonine is preferred for ease in commercial manufacture, particularly in glycosylation efficiency, although, as indicated above, a serine may also be used at this location. Conventional single letter amino acid abbreviations are used, as in Stryer, Biochemistry, Third Edition (1988), W.H. Freeman and Company, New York, inside back cover, herein incorporated by reference.

In view of above, the present invention also relates to a glycosylated leptin protein comprising SEQ. ID NO: 1 (rHu-Leptin 1-146, below) having one or more sequence alterations as a site of glycosylation selected from among (where "T/S" denotes threonine or serine) :

> (a) 01V->N 02P->X (where X is any amino acid except proline) 03I->T/S
> (b) 02P->N 03I 04Q->T/S
> (c) 23D->N 24I 25S->T/S
> (d) 47P->N 48I 49L->T/S
> (e) 48I->N 49L 50T/S
> (f) 69P->N 70S 71R->T/S
> (g) 92F->N 93S 94K->T/S
> (h) 101A->N 102S 103G->T/S
> (i) 102S->N 103G 104L->T/S
> (j) 103G->N 104L 105E->T/S

The working examples below demonstrate biological activity which at least approximates non-glycosylated rmetHu-Leptin 1-146 (SEQ. ID NO: 1) for single- and double-glycosylation site leptin proteins. Moreover, particular three, four and five-glycosylation site leptin proteins have demonstrated increased biological activity. Thus, the present invention also includes particular glycosylated leptin proteins as set forth in the working examples:

-- a glycosylated leptin protein comprising amino acids 1-146 of SEQ. ID NO: 1, having a glycosylation site located at a position selected from among (with respect to the numbering of SEQ. ID NO: 1: 1, 2, 4, 8, 23, 44, 47, 48, 69, 70, 93, 97, 100, 101, 102, 103, 118 and 141.

-- a glycosylated leptin protein comprising amino acids 1-146 of SEQ. ID NO: 1, having two glycosylation sites, said two sites selected from among (with respect to the numbering of SEQ. ID NO: 1):

> 47 + 69;
> 48 + 69;
> 69 + 101;
> 69 + 102;
> 69 + 103;
> 69 + 118; and,
> 100 + 102.

-- a glycosylated leptin protein comprising amino acids 1-146 of SEQ. ID NO: 1, having three glycosylation sites, said three sites selected from among (with respect to the numbering of SEQ. ID NO: 1):

2 + 47 + 69
23 + 47 + 69;
47 + 69 + 100;
47 + 69 + 102;
48 + 69 + 118;
69 + 102 + 118; and,
69 + 103 + 118.

-- a glycosylated leptin protein comprising amino acids 1-146 of SEQ. ID NO: 1, having four glycosylation sites, said four sites selected from among (with respect to the numbering of SEQ. ID NO: 1):

2 + 47 + 69 + 92;
2 + 47 + 69 + 102;
23 + 47 + 69 + 92;
23 + 47 + 69 + 102; and,
47 + 69 + 100 + 102.

-- a glycosylated leptin protein comprising amino acids 1-146 of SEQ. ID NO: 1, having five glycosylation sites, said five sites selected from among (with respect to the numbering of SEQ. ID NO: 1):

2 + 23 + 47 + 69 + 92
2 + 47 + 69 + 92 + 102
23 + 47 + 69 + 92 + 102.

More particularly, the present invention includes the following glycosylated leptin protein amino acid sequences, DNAs encoding such sequences, and specific DNAs as set forth below:

Glycosylated leptin 2,47,69 (SEQ. ID NO: 25, DNA):

```
  1   GTGAACATCA CAAAAGTCCA AGATGACACC AAAACCCTCA TCAAGACAAT
 51   TGTCACCAGG ATCAATGACA TTTCACACAC GCAGTCAGTC TCCTCCAAAC
101   AGAAAGTCAC CGGTTTGGAC TTCATTCCTG GGCTCCACAA CATCACGACC
151   TTATCCAAGA TGGACCAGAC ACTGGCAGTC TACCAACAGA TCCTCACCAG
201   TATGAATTCC ACAAACGTGA TCCAAATATC CAACGACCTG GAGAACCTCC
251   GGGATCTTCT TCACGTGCTG GCCTTCTCTA AGAGCTGCCA CTTGCCCTGG
301   GCCAGTGGCC TGGAGACCTT GGACAGCCTG GGGGGTGTCC TGGAAGCTTC
351   AGGCTACTCC ACAGAGGTGG TGGCCCTGAG CAGGCTGCAG GGGTCTCTGC
```

```
401   AGGACATGCT GTGGCAGCTG GACCTAAGCC CTGGGTGC
```

Glycosylated leptin 2,47,69 (SEQ. ID NO: 26, protein):

```
  1  VNITKVQDDT  KTLIKTIVTR  INDISHTQSV  SSKQKVTGLD  FIPGLHNITT
 51  LSKMDQTLAV  YQQILTSMNS  TNVIQISNDL  ENLRDLLHVL  AFSKSCHLPW
101  ASGLETLDSL  GGVLEASGYS  TEVVALSRLQ  GSLQDMLWQL  DLSPGC
```

Glycosylated leptin 2,47,69,92 (SEQ. ID NO: 27, DNA):

```
  1  GTGAACATCA  CAAAAGTCCA  AGATGACACC  AAAACCCTCA  TCAAGACAAT
 51  TGTCACCAGG  ATCAATGACA  TTTCACACAC  GCAGTCAGTC  TCCTCCAAAC
101  AGAAAGTCAC  CGGTTTGGAC  TTCATTCCTG  GGCTCCACAA  CATCACGACC
151  TTATCCAAGA  TGGACCAGAC  ACTGGCAGTC  TACCAACAGA  TCCTCACCAG
201  TATGAATTCC  ACAAACGTGA  TCCAAATATC  CAACGACCTG  GAGAACCTCC
251  GGGATCTTCT  TCACGTGCTG  GCCAACTCTA  CCAGCTGCCA  CTTGCCCTGG
301  GCCAGTGGCC  TGGAGACCTT  GGACAGCCTG  GGGGGTGTCC  TGGAAGCTTC
351  AGGCTACTCC  ACAGAGGTGG  TGGCCCTGAG  CAGGCTGCAG  GGGTCTCTGC
401  AGGACATGCT  GTGGCAGCTG  GACCTCAGCC  CTGGGTGC
```

Glycosylated leptin 2,47,69,92 (SEQ. ID NO: 28, protein)

```
  1  VNITKVQDDT  KTLIKTIVTR  INDISHTQSV  SSKQKVTGLD  FIPGLHNITT
 51  LSKMDQTLAV  YQQILTSMNS  TNVIQISNDL  ENLRDLLHVL  ANSTSCHLPW
101  ASGLETLDSL  GGVLEASGYS  TEVVALSRLQ  GSLQDMLWQL  DLSPGC
```

Glycosylated leptin 2,47,69,102 (SEQ. ID NO: 29, DNA):

```
  1  GTGAACATCA  CAAAAGTCCA  AGATGACACC  AAAACCCTCA  TCAAGACAAT
 51  TGTCACCAGG  ATCAATGACA  TTTCACACAC  GCAGTCAGTC  TCCTCCAAAC
101  AGAAAGTCAC  CGGTTTGGAC  TTCATTCCTG  GGCTCCACAA  CATCACGACC
151  TTATCCAAGA  TGGACCAGAC  ACTGGCAGTC  TACCAACAGA  TCCTCACCAG
201  TATGAATTCC  ACAAACGTGA  TCCAAATATC  CAACGACCTG  GAGAACCTCC
251  GGGATCTTCT  TCACGTGCTG  GCCTTCTCTA  AGAGCTGCCA  CTTGCCCTGG
301  GCCAATGGCA  CGGAGACCTT  GGACAGCCTG  GGGGGTGTCC  TGGAAGCTTC
351  AGGCTACTCC  ACAGAGGTGG  TGGCCCTGAG  CAGGCTGCAG  GGGTCTCTGC
401  AGGACATGCT  GTGGCAGCTG  GACCTCAGCC  CTGGGTGC
```

Glycosylated leptin 2,47,69,102 (SEQ. ID NO: 30 protein):

```
  1  VNITKVQDDT KTLIKTIVTR INDISHTQSV SSKQKVTGLD FIPGLHNITT
 51  LSKMDQTLAV YQQILTSMNS TNVIQISNDL ENLRDLLHVL AFSKSCHLPW
101  ANGTETLDSL GGVLEASGYS TEVVALSRLQ GSLQDMLWQL DLSPGC
```

Glycosylated leptin 47,69,102 (SEQ. ID NO: 31, DNA):

```
  1  GTGCCCATCC AAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCTTCTCTA AGAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

Glycosylated leptin 47,69,102 (SEQ. ID NO: 32, protein)

```
  1  VPIQKVQDDT KTLIKTIVTR INDISHTQSV SSKQKVTGLD FIPGLHNITT
 51  LSKMDQTLAV YQQILTSMNS TNVIQISNDL ENLRDLLHVL AFSKSCHLPW
101  ANGTETLDSL GGVLEASGYS TEVVALSRLQ GSLQDMLWQL DLSPGC
```

Glycosylated leptin 2,47,69,92,102 (SEQ. ID NO: 33, DNA):

```
  1  GTGAACATCA CAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCAACTCTA CCAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

Glycosylated leptin 2,47,69,92,102 (SEQ. ID NO: 34, protein):

```
  1  VNITKVQDDT KTLIKTIVTR INDISHTQSV SSKQKVTGLD FIPGLHNITT

 51  LSKMDQTLAV YQQILTSMNS TNVIQISNDL ENLRDLLHVL ANSTSCHLPW

101  ANGTETLDSL GGVLEASGYS TEVVALSRLQ GSLQDMLWQL DLSPGC
```

Glycosylated leptin 47,69,92,102 (SEQ. ID NO: 35, DNA):

```
  1  GTGCCCATCC AAAAAGTCCA AGATGACACC AAAACCCTCA TCAAGACAAT

 51  TGTCACCAGG ATCAATGACA TTTCACACAC GCAGTCAGTC TCCTCCAAAC

101  AGAAAGTCAC CGGTTTGGAC TTCATTCCTG GGCTCCACAA CATCACGACC

151  TTATCCAAGA TGGACCAGAC ACTGGCAGTC TACCAACAGA TCCTCACCAG

201  TATGAATTCC ACAAACGTGA TCCAAATATC CAACGACCTG GAGAACCTCC

251  GGGATCTTCT TCACGTGCTG GCCAACTCTA CCAGCTGCCA CTTGCCCTGG

301  GCCAATGGCA CGGAGACCTT GGACAGCCTG GGGGGTGTCC TGGAAGCTTC

351  AGGCTACTCC ACAGAGGTGG TGGCCCTGAG CAGGCTGCAG GGGTCTCTGC

401  AGGACATGCT GTGGCAGCTG GACCTCAGCC CTGGGTGC
```

Glycosylated leptin 47,69,92,102 (SEQ. ID NO: 36, protein):

```
  1  VPIQKVQDDT KTLIKTIVTR INDISHTQSV SSKQKVTGLD FIPGLHNITT

 51  LSKMDQTLAV YQQILTSMNS TNVIQISNDL ENLRDLLHVL ANSTSCHLPW

101  ANGTETLDSL GGVLEASGYS TEVVALSRLQ GSLQDMLWQL DLSPGC
```

These were the specific amino acid sequences and corresponding DNAs used in the working examples below.

Characterization by Sialic Acid Moieties

Furthermore, the present glycosylated proteins may be characterized by their number of sialic acid moieties. Generally, there may be zero to four sialic acid moieties at an N-linked glycosylation site, and zero to two sialic acid moieties at an O-linked glycosylation site. A typical glycosylated protein preparation will contain a mixture of fully (i.e., having a sialic acid moiety occupying all available sites) and partially (i.e., having a sialic acid moiety occupying less than all available sites) sialated glycosylated proteins molecules.

The number of sialic acid moieties may be determined by methods available to those skilled in the art. For example, one may measure the molecular weight of the protein or preparation thereof before and after treatment with enzymes which remove sialic acid, and calculate the molecular weight of the constituents. Alternatively, one may use isoelectric focusing or other methods to determine the sialic acid content.

For example, human leptin 1-145 (SEQ. ID NO: 2, below) contains two O-linked glycosylation sites, and thus, when fully sialated, four sialic acid moieties. The present working examples with a single_ N-linked glycosylation site contain four sialic acid moieties, when fully sialated. The two-site glycosylated leptin proteins below, when fully glycosylated, contain 8 sialic acid moieties, the three site, 12 sialic acid moieties, the four, 16 sialic acid moieties, and the five site glycosylated leptins, 20 sialic acid moieties. The present invention thus encompasses glycosylated leptin protein preparation wherein each glycosylated leptin protein molecule in said preparation has five or more sialic acid moieties. More preferably, for purposes of enhancing a sustained release effect of a therapeutic protein, the present invention also encompasses a glycosylated leptin protein preparation wherein each glycosylated leptin protein molecule in said

EP 1 151 102 B1

preparation has 8 to 20 sialic acid residues. One may also choose to add additional glycosylation sites, and increase the sialic acid content above 20 accordingly.

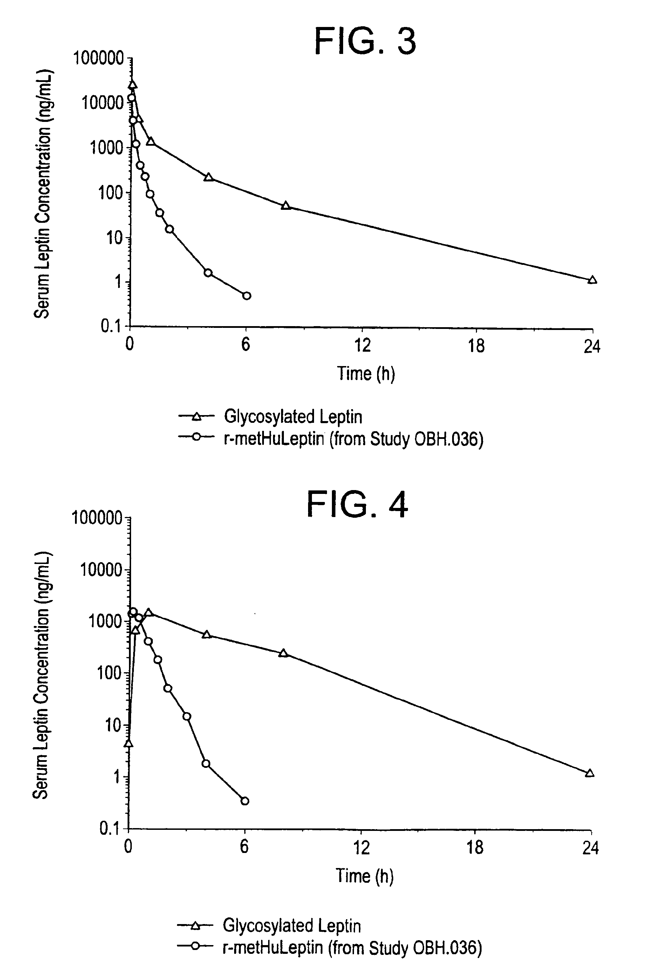

3. Leptin Protein Backbone. The type of leptin used for the glycosylated leptin pharmaceutical compositions may be selected from those described in PCT International Publication Number WO96/05309, as cited above and herein incorporated by reference in its entirety. Figure 3 of that publication (as cited therein SEQ. ID NO: 4) depicts the full deduced amino acid sequence derived for human leptin (also referred to as the human "OB" protein). The amino acids are numbered from 1 to 167. A signal sequence cleavage site is located after amino acid 21 (Ala) so that the mature protein extends from amino acid 22 (Val) to amino acid 167 (Cys). For the present disclosure, a different numbering is used herein, where the amino acid position 1 is the valine residue which is at the beginning of the mature protein.

The amino acid sequence for mature, recombinant human leptin is presented herein as SEQ. ID NO: 1, where the first amino acid of the mature protein is valine (at position 1) (herein called rHu-Leptin 1-146, SEQ. ID NO: 1):

```
              V   P   I   Q   K   V   Q   D   D   T   K   T   L   I   K   T   I   V
      T   R   I   N   D   I   S   H   T   Q   S   V   S   S   K   Q   K   V   T   G
      L   D   F   I   P   G   L   H   P   I   L   T   L   S   K   M   D   Q   T   L
      A   V   Y   Q   Q   I   L   T   S   M   P   S   R   N   V   I   Q   I   S   N
      D   L   E   N   L   R   D   L   L   H   V   L   A   F   S   K   S   C   H   L
      P   W   A   S   G   L   E   T   L   D   S   L   G   G   V   L   E   A   S   G
      Y   S   T   E   V   V   A   L   S   R   L   Q   G   S   L   Q   D   M   L   W
      Q   L   D   L   S   P   G   C
```

Alternatively, one may use a natural variant of human leptin, which has 145 amino acids, and, as compared to rHu-Leptin 1-146, has a glutamine absent at position 28, presented below (herein called rHu-Leptin 1-145, SEQ. ID NO: 2, wherein the blank ("_") indicates no amino acid) :

```
              V   P   I   Q   K   V   Q   D   D   T   K   T   L   I   K   T   I   V
      T   R   I   N   D   I   S   H   T   _   S   V   S   S   K   Q   K   V   T   G
      L   D   F   I   P   G   L   H   P   I   L   T   L   S   K   M   D   Q   T   L
      A   V   Y   Q   Q   I   L   T   S   M   P   S   R   N   V   I   Q   I   S   N
      D   L   E   N   L   R   D   L   L   H   V   L   A   F   S   K   S   C   H   L
      P   W   A   S   G   L   E   T   L   D   S   L   G   G   V   L   E   A   S   G
      Y   S   T   E   V   V   A   L   S   R   L   Q   G   S   L   Q   D   M   L   W
      Q   L   D   L   S   P   G   C
```

For example, for the specific glycosylated leptin proteins recited herein, one may choose to use the "Q-" version of human leptin (1-145, SEQ. ID NO: 2) and modify the corresponding sites enumerated for the 1-146 amino acid human leptin to include glycosylation sites.

Generally, the leptin protein for use herein will be capable of therapeutic use in humans (see also, animal leptins, below). Thus, one may empirically test activity to determine which leptin protein forms may be used. As set forth in WO96/05309, leptin protein in its native form, or fragments (such as enzyme cleavage products) or other truncated forms and analogs may all retain biological activity. Any of such forms may be used to prepare the present glycosylated leptin compositions, although such altered forms should be tested to determine desired characteristics. See also, PCT International Publication Numbers WO96/40912, WO97/06816, WO97/18833, WO97/38014 and WO98/08512, all here incorporated by reference.

One may prepare an analog of recombinant human leptin by altering amino acid residues in the recombinant human sequence, such as substituting the amino acids which diverge from the murine sequence. Murine leptin is substantially homologous to human leptin, particularly as a mature protein and, further, particularly at the N-terminus. Because the recombinant human protein has biological activity in mice, such an analog would likely be active in humans.

14

For example, in the amino acid sequence of native human leptin as presented in SEQ. ID NO: 1, one may substitute with another amino acid one or more of the amino acids at positions 32, 35, 50, 64, 68, 71, 74, 77, 89, 97, 100, 105, 106, 107, 108, 111, 118, 136, 138, 142, and 145. One may select the amino acid at the corresponding position of the murine protein (SEQ. ID NO: 3) or another amino acid.

One may further prepare synthetic molecules based on the rat leptin, called, OB protein, sequence. Murakami et al., Biochem. Biophys. Res. Comm. 209: 944-52 (1995) herein incorporated by reference. Rat OB protein differs from human OB protein at the following positions (using the numbering of SEQ. ID NO:: 1): 4, 32, 33, 35, 50, 68, 71, 74, 77, 78, 89, 97, 100, 101, 102, 105, 106, 107, 108, 111, 118, 136, 138 and 145. One may substitute with another amino acid one or more of the amino acids at these divergent positions. The positions in bold print are those in which the murine OB protein as well as the rat OB protein are divergent from the human OB protein and, thus, are particularly suitable for alteration. At one or more of the positions, one may substitute an amino acid from the corresponding rat OB protein, or another amino acid.

The positions from both rat and murine OB protein which diverge from the mature human OB protein are: 4, 32, 33, 35, 50, 64, 68, 71, 74, 77, 78, 89, 97, 100, 102, 105, 106, 107, 108, 111, 118, 136, 138, 142, and 145. An OB protein according to SEQ. ID NO:: 1 having one or more of the above amino acids replaced with another amino acid, such as the amino acid found in the corresponding rat or murine sequence, may also be effective.

In addition, the amino acids found in rhesus monkey OB protein which diverge from the mature human OB protein are (with identities noted in parentheses in one letter amino acid abbreviation): 8 (S), 35 (R), 48 (V), 53 (Q), 60 (I), 66 (I), 67 (N), 68 (L), 89 (L), 100 (L), 108 (E), 112 (D) and 118 (L). Since the recombinant human OB protein is active in cynomolgus monkeys, a human OB protein according to SEQ. ID NO: 1 having one or more of the rhesus monkey divergent amino acids replaced with another amino acid, such as the amino acids in parentheses, may be effective. It should be noted that certain rhesus divergent amino acids are also those found in the above murine species (positions 35, 68, 89, 100 and 112). Thus, one may prepare a murine/rhesus/human consensus molecule (using the numbering of SEQ. ID NO: 1) having one or more of the amino acids at positions replaced by another amino acid: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145.

Other analogs may be prepared by deleting a part of the protein amino acid sequence. For example, the mature protein lacks a signal sequence (-22 to -1). One may delete a portion of the mature protein, and this deletion may be incident to manufacture, e.g., cleavage of signal peptide or other presequences beyond the first N-terminal amino acid of the mature protein. Also, the N-terminus may contain one or more additional amino acids, which may be incident to use of such presequences, such as, for example, cleavage in the middle of a signal peptide cleavage site, so that a portion of the amino acids of the cleavage site is attached.

One may prepare the following truncated forms of human leptin protein molecules (using the numbering of SEQ. ID NO: 1) :

(a) amino acids 98-146;
(b) amino acids 1-99 and (connected to) 112-146;
(c) amino acids 1-99 and (connected to) 112-146 having one or more of amino acids 100-111 sequentially placed between amino acids 99 and 112.

In addition, the truncated forms may also have altered one or more of the amino acids which are divergent (in the murine, rat or rhesus OB protein) from human OB protein. Furthermore, any alterations may be in the form of altered amino acids, such as peptidomimetics or D-amino acids.

Included are those proteins as set forth above with amino acid substitutions which are "conservative" according to acidity, charge, hydrophobicity, polarity, size or any other characteristic known to those skilled in the art. These are set forth in Table 1, below. See generally, Creighton, Proteins, W.H. Freeman and Company, N.Y., (1984) p. 498, plus index, passim. See, in general Ford et al., Protein Expression and Purification 2: 95-107, 1991, which is herein incorporated by reference.

Table 1 Conservative Amino Acid Substitutions

| Basic: | arginine lysine histidine |
|---|---|
| Acidic: | glutamic acid aspartic acid |
| Polar: | glutamine asparagine |

Table continued

| Hydrophobic: | leucine isoleucine valine |
|---|---|
| Aromatic: | phenylalanine tryptophan tyrosine |
| Small: | glycine alanine serine threonine methionine |

Therefore, the present glycosylated human leptin proteins may be prepared by first starting with a sequence selected from among (according to the amino acid sequence as presented in SEQ. ID NO:: 1 herein):

(a) the amino acid sequence of SEQ. ID NO:: 1, optionally lacking a glutaminyl residue at position 28;
(b) an amino acid sequence of subpart (a) having a different amino acid substituted in one or more of the following positions: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145;
(c) a truncated leptin protein analog selected from among: (using the numbering of subpart (a) above):

(i) amino acids 98-146
(ii) amino acids 1-99 and 112-146
(iii) amino acids 1-99 and 112-146 having one or more of amino acids 100-111 sequentially placed between amino acids 99 and 112; and,
(iv) the truncated leptin analog of subpart (i) having one or more of amino acids 100, 102, 105, 106, 107, 108, 111, 112, 118, 136, 138, 142 and 145 substituted with another amino acid;
(v) the truncated leptin analog of subpart (ii) having one or more of amino acids 4, 8, 32, 33, 35, 48, 50, 53, 60, 64, 66, 67, 68, 71, 74, 77, 78, 89, 97, 112, 118, 136, 138, 142 and 145 replaced with another amino acid;
(vi) the truncated leptin analog of subpart (iii) having one or more of amino acids 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145 replaced with another amino acid; and

(d) a leptin protein of any of subparts (a)-(c) having one or more conserved amino acids substitutions, and then selecting a site, preferably on the external surface of an alpha helix, to insert, by addition or substitution, a glycosylation site. Particular glycosylation sites are recited supra.

Leptin proteins, analogs and related molecules are also reported in the following publications; however, no representation is made with regard to the activity of any composition reported:

U.S. Patent Nos. 5,521,283; 5,525,705; 5,532,336; 5,552,522; 5,552,523; 5,552,524; 5,554,727; 5,559,208; 5,563,243; 5,563,244; 5,563,245; 5,567,678; 5,567,803; 5,569,743; 5,569,744; 5,574,133; 5,580,954; 5,594,101; 5,594,104; 5,605,886; 5,614,379; 5,691,309; 5,719,266 (all assigned to Eli Lilly and Company);
PCT WO96/23513; WO96/23514; WO96/23515; WO96/23516; WO96/23517; WO96/23518; WO96/23519; WO96/23520; WO96/23815; WO96/24670; WO96/27385; EP 725078; EP 725079 (all assigned to Eli Lilly and Company);
PCT WO96/22308 (assigned to Zymogenetics); PCT WO96/29405 (assigned to Ligand
Pharmaceuticals, Inc.); PCT WO96/31526 (assigned to Amylin
Pharmaceuticals, Inc.); PCT WO96/34885 (assigned to Smithkline Beecham PLC);
PCT WO96/35787 (assigned to Chiron);
EP 736599 (assigned to Takeda);
EP 741187 (assigned to F. Hoffman LaRoche).

To the extent these references provide for useful leptin proteins or analogs, or associated compositions or methods, such compositions and/or methods may be used in conjunction with the present glycosylated leptin pharmaceutical compositions, such as for co-administration (together or separately, in a selected dosage schedule). With the above provisos, these publications are herein incorporated by reference.

Nucleic Acids, Vectors, Host Cells and other Expression Systems

Also comprehended by the present invention are nucleic acids encoding the present glycosylated leptin proteins. Such nucleic acids may be prepared by site directed mutagenesis of an existing nucleic acid sequence or by synthetic means, or by other means as are available to those skilled in the art. Methods as disclosed in the Reference Examples below are illustrative.

Vectors include plasmidic as well as viral vectors as are available to those skilled in the art. Vectors may be for cloning or expression, and include plasmids, cosmids, and prokaryotic or eukaryotic-cell infecting viruses. For expression of glycosylated protein, vectors will be useful for expression in a eukaryotic cell. The expression system may be constitutive or inducible, such as systems including an inducible mouse mammary tumor virus LTR promoter. Enhancers, transcription terminators, splice donor and acceptor sites, and other elements may be included in the overall system as is known to those skilled in the art.

Vectors disclosed in the Reference Examples below are illustrative. The present working examples used a modified form of pDSRα2, to express glycosylated leptin proteins.

The host cells may be prokaryotic, such as bacteria used for cloning of the present nucleic acids, for example. Other host cells may be eukaryotic. Eukaryotic host cells may be selected from the phylum Chordata, such as those in the class Mammalia. Primate cells, including human cells (such as Namalwa, HeLa, human hepatocellular carcinoma, such as Hep G2 cells, human embryonic kidney cells, human liver cells, human lung cells or cells cultured from human sources) and COS cells, or other mammalian cells, such as baby hamster kidney cells ("BHK" cells), Chinese hamster ovary cells ("CHO") cells, mouse sertoli cells, canine kidney cells, buffalo rat liver cells, mouse mammary tumor cells may be used. Insect cells may also be used. Lesser host cell organisms, such as yeasts, and fungi, are also included. See generally, Margulis, Five Kingdoms, 2d Edition (1988) W.H. Freeman & Co., New York, for classifications of organisms.

One may seek to co-express more than one desired protein. For example, the present glycosylated leptin protein may be expressed in a eukaryotic host cell along with one or more other desired proteins. The proteins may be separated using a number of available separation techniques, depending on the characteristics of the protein. For example, one may, in a single host cell, such as a CHO cell, express a glycosylated leptin protein, as well as a different protein, such as a different glycosylated protein desired for therapeutic use. One may use, for example, molecular weight to separate the proteins for purification. In this way, economies of manufacture may be achieved, by producing two different proteins from a single cell culture.

One may also use transgenic animals to express the present glycosylated leptin protein. For example, one may use a transgenic milk-producing animal (a cow or goat, for example) and obtain the present glycosylated leptin protein in the milk produced. One may use plants to produce the present glycosylated proteins, however, in general, the glycosylation occurring in plants is different from that produced in mammalian cells, and may result in a glycosylated product which is not suitable for human therapeutic use.

Gene Therapy

The DNA provided herein (or corresponding RNAs) may also be used for gene therapy. A review article on gene therapy is Verma, Scientific American, November 1990, pages 68-84 which is herein incorporated by reference.

Thus, the present invention provides for a population of cells expressing the present glycosylated leptin protein. Such cells are suitable for transplantation or implantation into an individual for therapeutic purposes. One may then implant such cells into an individual. Such cells may, for example, be liver cells, bone marrow cells, or cells derived from umbilical cord. Alternatively, one may wish to use circulating cells such as blood progenitor cells, T cells or other blood cells. For humans, human cells may be used. Cells may be in the form of tissue. Such cells may be cultured prior to transplantation or implantation.

The cells to be transferred to the recipient may be cultured using one or more factors affecting the growth or proliferation of such cells if appropriate. Hematopoietic factors may be used in culturing hematopoietic cells. Such factors include G-CSF, EPO, MGDF, SCF, Flt-3 ligand, interleukins (e.g., IL1-IL13), GM-CSF, LIF, and analogs and derivatives thereof as available to one skilled in the art.

Nerve cells, such as neurons or glia, may also be used, and these may be cultured with neurotrophic factors such as BDNF, CNTF, GDNF, NT3, or others.

Techniques for the encapsulation of living cells are familiar to those of ordinary skill in the art, and the preparation of the encapsulated cells and their implantation in patients may be accomplished without undue experimentation. For example, Baetge et al. (International Publication No. WO 95/05452; International Application No. PCT/US94/09299 the disclosure of which is hereby incorporated by reference) describe membrane capsules containing genetically engineered cells for the effective delivery of biologically active molecules. The capsules are biocompatible and are easily retrievable. The capsules encapsulate cells transfected with recombinant DNA molecules comprising DNA sequences coding for biologically active molecules operatively linked to promoters that are not subject to down regulation in vivo upon implantation into a mammalian host. The devices provide for the delivery of the molecules from living cells to specific sites within a recipient. In addition, see U.S. Patent Numbers 4,892,538, 5,011,472, and 5,106,627, each of which is specifically incorporated herein by reference. A system for encapsulating living cells is described in PCT Application WO 91/10425 of Aebischer et al., specifically incorporated herein by reference. See also, PCT Application WO 91/10470 of Aebischer et al., Winn et al., Exper. Neurol. 113:322-329(1991), Aebischer et al., Exper. Neurol. 111:269-275, (1991); Tresco et al., ASAIO 38:17-23(1992), each of which is specifically incorporated herein by reference.

In vivo and in vitro gene therapy delivery of the present glycosylated leptin protein is also envisioned. In vivo gene therapy may be accomplished by introducing the nucleic acid encoding a present glycosylated leptin protein into cells via local injection of a polynucleotide molecule or other appropriate delivery vectors. (Hefti, J. Neurobiology,. 25: 1418-1435, 1994). For example, a polynucleotide molecule encoding a glycosylated leptin protein may be contained in an adeno-associated virus vector for delivery into the targeted cells (e.g., Johnson, International Publication No. WO 95/34670; International Application No. PCT/US95/07178 the disclosure of which is hereby incorporated by reference). The recombinant adeno-associated virus (AAV) genome contains AAV inverted terminal repeats flanking a DNA sequence encoding the neurotrophic factor operably linked to functional promoter and polyadenylation sequences.

Alternative viral vectors include, but are not limited to, retrovirus, adenovirus, herpes simplex virus and papilloma virus vectors. U.S. 5,672,344 (issued September 30, 1997, Kelley et al., University of Michigan), the disclosure of which is hereby incorporated by reference, describes an in vivo viral-mediated gene transfer system involving a recombinant neurotropic HSV-1 vector. U.S. 5,399,346 (issued March 21, 1995, Anderson et al., Department of Health and human Services), the disclosure of which is incorporated by reference herein, provides examples of a process for providing a patient with a therapeutic protein by the delivery of human cells which have been treated in vitro to insert a DNA segment encoding a therapeutic protein. Additional methods and materials for the practice of gene therapy techniques, the disclosures of which are incorporated by reference herein, are described in U.S. 5,631,236 (issued May 20, 1997, Woo et al., Baylor College of Medicine) involving adenoviral vectors; U.S. 5,672,510 (issued September 30, 1997, Eglitis et al., Genetic Therapy, Inc.) involving retroviral vectors; and U.S. 5,635,399 (issued June 3, 1997, Kriegler et al., Chiron Corporation) involving retroviral vectors expressing cytokines.

Nonviral delivery methods include liposome-mediated transfer, naked DNA delivery (direct injection), receptor-mediated transfer (ligand-DNA complex), electroporation, calcium phosphate precipitation and microparticle bombardment (e.g., gene gun). Gene therapy materials and methods may also include inducible promoters, tissue-specific enhancer-promoters, DNA sequences designed for site-specific integration, DNA sequences capable of providing a selective advantage over the parent cell, labels to identify transformed cells, negative selection systems and expression control systems (safety measures), cell-specific binding agents (for cell targeting), cell-specific internalization factors, transcription factors to enhance expression by a vector as well as methods of vector manufacture. Such additional methods and materials for the practice of gene therapy techniques, the disclosures of which are incorporated by reference herein, are described in U.S. 4,970,154 (issued November 13, 1990, D.C. Chang, Baylor College of Medicine) electroporation techniques; WO 9640958 (published 961219 , Smith et al., Baylor College of Medicine) nuclear ligands; U.S. 5,679,559 (issued October 21, 1997, Kim et al., University of Utah Research Foundation) concerning a lipoprotein-containing system for gene delivery; U.S. 5,676,954 (issued October 14, 1997, K.L. Brigham, Vanderbilt University involving liposome carriers; U.S. 5,593,875 (issued January 14, 1997, Wurm et al., Genentech, Inc.) concerning methods for calcium phosphate transfection; and U.S. 4,945,050 (issued July 31, 1990, Sanford et al., Cornell Research Foundation) wherein biologically active particles are propelled at cells at a speed whereby the particles penetrate the surface of the cells and become incorporated into the interior of the cells. Expression control techniques include chemical induced regulation (e.g., WO 9641865 and WO 9731899), the use of a progesterone antagonist in a modified steroid hormone receptor system (e.g., U.S. 5,364,791), ecdysone control systems (e.g., WO 9637609), and positive tetracycline-controllable transactivators (e.g., U.S. 5,589,362; U.S. 5,650,298; and U.S. 5,654,168).

It is also contemplated that the present gene therapy or cell therapy can further include the delivery of a second therapeutic composition. For example, the host cell may be modified to express and release both a glycosylated leptin protein and native human leptin. Alternatively, they may be expressed in and released from separate cells. Such cells may be separately introduced into the patient or the cells may be contained in a single implantable device, such as the encapsulating membrane described above.

Selective Binding Molecules

The present invention also relates to selective binding moieties of the present glycosylated human leptin proteins. A "selective binding moiety" denotes a substance which selectively binds to the present glycosylated human leptin proteins, in glycosylated or unglycosylated form. Selectivity is determined by whether the binding moiety binds to the subject leptin protein above background (nonselective) levels. The selective binding molecules are antibodies, such as monoclonal, polyclonal, monospecific polyclonal, produced by, for example hybridoma technology or using recombinant nucleic acid means. See, e.g., Huse et al., Science 246: 1275 (1989). Also comprehended herein are nucleic acids, vectors, host cells and other materials and methods used in the recombinant nucleic acid expression of a selective binding moiety, such as a recombinant antibody. One may attach detectable labels to such selective binding moieties, such as chemiluminescent, fluorescent, colorimetric, or radioactive, using materials and methods available to those skilled in the art. One may prepare assays, or kits, containing one or more of these selective binding molecules, for detection or measurement of the present leptin proteins. Illustrative is a kit including monoclonal antibodies selective for a particular glycosylated leptin protein, and means to detect selective binding of said monoclonal antibodies to said glycosylated leptin protein. Other materials and methods for such kits are available to those skilled in the art.

Formulations and Derivatives

In yet another aspect of the present invention, provided are pharmaceutical compositions of the present glycosylated leptin compositions, and derivatives (see below). Such pharmaceutical compositions may,be for administration by injection, or for oral, intrathecal, pulmonary, nasal, transdermal or other forms of administration. In general, comprehended by the invention are pharmaceutical compositions comprising effective amounts of protein or derivative products of the invention together with pharmaceutically acceptable diluents, preservatives, solubilizers, emulsifiers, adjuvants and/or carriers. Such compositions include diluents of various buffer content (e.g., Tris-HCl, acetate, phosphate), pH and ionic strength; additives such as detergents and solubilizing agents (e.g., Tween 80, Polysorbate 80), anti-oxidants (e.g., ascorbic acid, sodium metabisulfite), preservatives (e.g., Thimersol, benzyl alcohol) and bulking substances (e.g., lactose, mannitol); incorporation of the material into particulate preparations of polymeric compounds such as polylactic acid, polyglycolic acid, etc. or into liposomes. See, e.g., PCT WO96/29989, Collins et al., "Stable protein: phospholipid compositions and methods," published October 3, 1996, herein incorporated by reference. Hylauronic acid may also be used, and this may have the effect of promoting sustained duration in the circulation. Such compositions may influence the physical state, stability, rate of in vivo release, and rate of in vivo clearance of the present proteins and derivatives. See, e.g., Remington's Pharmaceutical Sciences, 18th Ed. (1990, Mack Publishing Co., Easton, PA 18042) pages 1435-1712 which are herein incorporated by reference. The compositions may be prepared in liquid form, or may be in dried powder, such as lyophilized form. Implantable sustained release formulations are also contemplated, as are transdermal formulations.

Specifically contemplated are oral dosage forms of the above derivatized proteins. Protein may be chemically modified so that oral delivery of the derivative is efficacious. Generally, the chemical modification contemplated is the attachment of at least one moiety to the protein (or peptide) molecule itself, where said moiety permits (a) inhibition of proteolysis; and (b) uptake into the blood stream from the stomach or intestine. Also desired is the increase in overall stability of the protein and increase in circulation time in the body. See PCT WO95/21629, Habberfield, "Oral Delivery of Chemically Modified Proteins" (published August 17, 1995) herein incorporated by reference, and U.S. Patent No. 5,574,018, Habberfield et al., "Conjugates of Vitamin B12 and Proteins," issued November 12, 1996, herein incorporated by reference. The materials and methods disclosed therein are applicable to the present glycosylated leptin compositions and methods.

Also contemplated herein is pulmonary delivery of the present protein, or derivative thereof. The protein (derivative) is delivered to the lungs of a mammal while inhaling and traverses across the lung epithelial lining to the blood stream. See, PCT WO94/20069, Niven et al., "Pulmonary administration of granulocyte colony stimulating factor," published September 15, 1994, herein incorporated by reference, and PCT WO96/05309, previously incorporated by reference at page 83 et seq., for example. The present glycosylated leptin proteins may be spray-dried into particles having an average size of less than 10 microns, or more preferably, 0.5 to 5 microns. Larger sized particles may be used depending on the density of each particle.

Nasal delivery of the protein (or analog or derivative) is also contemplated. Nasal delivery allows the passage of the protein to the blood stream directly after administering the therapeutic product to the nose, without the necessity for deposition of the product in the lung. Formulations for nasal delivery include those with absorption enhancing agents, such as dextran or cyclodextran. Delivery via transport across other mucous membranes is also contemplated.

The present glycosylated leptin proteins may also be derivatized by the attachment of one or more chemical moieties to the protein moiety. Chemical modification of biologically active proteins has been found to provide additional advantages under certain circumstances, such as increasing the stability and circulation time of the therapeutic protein and decreasing immunogenicity. See U.S. Patent No. 4,179,337, Davis et al., issued December 18, 1979. For a review, see Abuchowski et al., in Enzymes as Drugs. (J.S. Holcerberg and J. Roberts, eds. pp. 367-383 (1891)). A review article describing protein modification and fusion proteins is Francis, Focus on Growth Factors 3: 4-10 (May 1992) (published by Mediscript, Mountview Court, Friern Barnet Lane, London N20, OLD, UK). One may wish to further modify the present glycosylated leptin compositions, such as adding, by chemical modification, a water soluble polymer. The addition of a chemical moiety will likely require an additional manufacturing step, but may result in further benefits in terms of improved product characteristics (with the caveat that under some conditions, chemical derivatization may make the product less desirable, such as by inducing the formation of kidney vacuoles, see supra). The chemical moieties should be attached to the protein with consideration of effects on functional or antigenic domains of the protein. There are a number of attachment methods available to those skilled in the art. E.g., PCT WO96/11953, "N-Terminally Chemically Modified Protein Compositions and Methods," published April 25, 1996, herein incorporated by reference in its entirety, and EP 0 401 384 herein incorporated by reference (coupling PEG to G-CSF). The methods and polymers disclosed therein in the above publications are applicable to the present glycosylated leptin compositions, if derivatization is desired to further improve characteristics of a therapeutic composition, for example.

Fusion proteins may be prepared by attaching polyaminoacids to glycosylated leptin protein moiety. For example, the polyamino acid may be a carrier protein which serves to further increase the circulation half life of the protein. For the present therapeutic or cosmetic purposes, such polyamino acid should be those which do not create neutralizing antigenic response, or other adverse response. Such polyamino acid may be selected from the group consisting of serum album (such as human serum albumin), an antibody or portion thereof (such as an antibody constant region, sometimes called "$F_C$") or other polyamino acids. The location of attachment of the polyamino acid may be at the N-terminus of the glycosylated leptin protein moiety, or other place, and also may be connected by a chemical "linker" moiety to the protein. See, e.g., PCT WO 98/28427, published July 2, 1998, entitled, "Ob Fusion Protein Compositions and Methods", herein incorporated by reference in its entirety. The polyamino acid may be used to aid in detection or purification, such as using a "FLAG" tag, "his" tag, "myc" tag or other polyamino acid tag known to those skilled in the art.

Relatedly, detectable labels may be attached to the present glycosylated leptin proteins. Radioisotopes, light-emitting (e.g., fluorescent or chemiluminescent compounds), enzymatically cleavable compounds, detectable antibody (or modification thereof) or other substances may be used for such labeling of the present proteins. Detecting protein via use of the labels may be useful for identifying the presence or amount of the present proteins, or a compound containing such proteins (such as an antibody/protein complex).

Dosages

One skilled in the art will be able to ascertain effective dosages by administration and observing the desired therapeutic effect. Presently, unmodified rmetHu-leptin 1-146 has been demonstrated to be effective at doses of 0.3 mg protein/kg body weight/day, and has been seen to be less effective at a dose of 0.1 mg protein/kg body weight/day. Greenberg et al., Preliminary safety and efficacy of recombinant methionyl human Leptin administered by SC injection in lean and obese subjects. Poster presented at: Annual Meeting of the American Diabetes Association; June 16, 1998, Chicago, IL. The desired dosage range, to have advantage over the existing rmetHu-leptin 1-146 is the same or lower than the above. Also, a desired dosage range may be one in which the same (or lower) protein load is administered less frequently. The effective dosages may be determined using diagnostic tools over time. For example, a diagnostic for measuring the amount of leptin in the blood (or plasma or serum) may first be used to determine endogenous levels of leptin. Such diagnostic tool may be in the form of an antibody assay, such as an antibody sandwich assay. The amount of endogenous leptin is quantified initially, and a baseline is determined. The therapeutic dosages are determined as the quantification of endogenous and exogenous leptin (that is, protein, analog or derivative found within the body, either self-produced or administered) is continued over the course of therapy. The dosages may therefore vary over the course of therapy, with a relatively high dosage being used initially, until therapeutic or cosmetic benefit is seen, and lower dosages used to maintain the therapeutic or cosmetic benefits.

During an initial course of therapy of an obese person, dosages may be administered whereby weight loss and concomitant fat tissue decrease increase is achieved. Once sufficient weight loss is achieved, a dosage sufficient to prevent re-gaining weight, yet sufficient to maintain desired weight or fat mass may be administered. These dosages can be determined empirically, as the effects of leptin are reversible. E.g., Campfield et al., Science 269: 546-549 (1995) at 547. Thus, if a dosage resulting in weight loss is observed when weight loss is not desired, one would administer a lower dose, yet maintain the desired weight.

Methods of Use

Therapeutic. Therapeutic uses include weight modulation, the treatment or prevention of diabetes, blood lipid reduction (and treatment of related conditions), increasing lean body mass and increasing insulin sensitivity. In addition, the present compositions may be used for manufacture of one or more medicaments for treatment or amelioration of the above conditions.

Cosmetic. For those desiring solely appearance enhancement, the present compositions may be used for weight loss, or weight maintenance which has no concomitant effect on an adverse medical condition. In addition, the present compositions may be used for manufacture of one or more preparations for cosmetic purposes.

Weight Modulation. The present compositions and methods may be used for weight reduction. Viewed another way, the present compositions may be used for maintenance of a desired weight or level of adiposity. As has been demonstrated in murine models (see supra), administration of the present glycosylated leptin proteins results in weight loss. The body mass lost is primarily of adipose tissue, or fat. Such weight loss, or maintenance of a particular weight, can be associated with the prevention or treatment of concomitant conditions, such as those below, and therefore constitute a therapeutic application.

Treatment of Diabetes. The present compositions and methods may be used in the prevention or treatment of Type I or Type II diabetes. As Type II diabetes can be correlated with obesity, use of the present invention to reduce weight (or maintain a desired weight, or reduce or maintain an adiposity level) can also alleviate or prevent the development of diabetes. Moreover, even in the absence of dosages sufficient to result in weight loss, the present compositions may be used to prevent or ameliorate diabetes.

Administration of the present compositions may result in an increased sensitivity to endogenous or exogenous insulin, and allow an individual to reduce or eliminate the amount administration of exogenous insulin required to treat type II diabetes. It is further contemplated that the present compositions may be used in the treatment, prevention or amelioration of Type I diabetes.

Blood Lipid Modulation. The present compositions and methods may be used in the modulation of blood lipid levels. Ideally, in situations where solely reduction in blood lipid levels is desired, or where maintenance of blood lipid levels is desired, the dosage will be insufficient to result in weight loss. Thus, during an initial course of therapy of an obese patient, dosages may be administered whereby weight loss and concomitant blood lipid level lowering is achieved. Once sufficient weight loss is achieved, a dosage sufficient to prevent re-gaining weight, yet sufficient to maintain desired blood lipid levels, or other conditions as set forth herein, for example, may be administered. Thus, if a dosage resulting in weight loss is observed when weight loss is not desired, one would administer a lower dose in order to achieve the desired blood lipid levels, yet maintain the desired weight. See, e.g., PCT Publication WO97/06816 herein incorporated by reference.

Increasing Lean Mass or Insulin Sensitivity.

Ideally, in situations where solely an increase in lean body mass is desired, the dosage will be insufficient to result in weight loss. Thus, during an initial course of therapy of an obese person, dosages may be administered whereby weight loss and concomitant fat tissue decrease/lean mass increase is achieved. Once sufficient weight loss is achieved, a dosage sufficient to prevent re-gaining weight, yet sufficient to maintain desired lean mass increase (or prevention of lean mass depletion) may be administered. For increasing an individual's sensitivity to insulin, similar dosage considerations may be taken into account. Lean mass increase without weight loss may be achieved sufficient to decrease the amount of insulin (or, potentially, amylin, amylin antagonists or agonists, or thiazolidinediones, or other potential diabetes treating drugs) an individual would be administered for the treatment of diabetes. For increasing overall strength, there may be similar dosage considerations. Lean mass increase with concomitant increase in overall strength may be achieved with doses insufficient to result in weight loss. Other benefits, such as an increase in red blood cells (and oxygenation in the blood) and a decrease in bone resorption or osteoporosis may also be achieved in the absence of weight loss. E.g., PCT WO97/18833, published May 29, 1997, herein incorporated by reference in its entirety.

Combination Therapies. The present compositions and methods may be used in conjunction with other therapies, such as altered diet and exercise. Other medicaments, such as those useful for the treatment of diabetes (e.g., insulin and possibly amylin, antagonists or agonists thereof, thiazolidinediones, or other potential diabetes treating drugs), cholesterol and blood pressure lowering medicaments (such as those which reduce blood lipid levels or other cardiovascular medicaments), activity increasing medicaments (e.g., amphetamines), diuretics (for liquid elimination), and appetite suppressants (such as agents which act on neuropeptide $\gamma$ receptors, serotonin reuptake inhibitors or gastric fat uptake inhibitors). Such administration may be simultaneous or may be in seriatim. In addition, the present methods may be used in conjunction with surgical procedures, such as cosmetic surgeries designed to alter the overall appearance of a body (e.g., liposuction or laser surgeries designed to reduce body mass, or implant surgeries designed to increase the appearance of body mass). The health benefits of cardiac surgeries, such as bypass surgeries or other surgeries designed to relieve a deleterious condition caused by blockage of blood vessels by fatty deposits, such as arterial plaque, may be increased with concomitant use of the present compositions and methods. Methods to eliminate gall stones, such as ultrasonic or laser methods, may also be used either prior to, during or after a course of the present therapeutic methods. Furthermore, the present methods may be used as an adjunct to surgeries or therapies for broken bones, damaged muscle, or other therapies which would be improved by an increase in lean tissue mass.

Methods of Manufacture

As indicated above, it has also been observed that particular constructs of signal sequences and mature protein sequences may improve glycosylation efficiency. In this regard, the term "signal sequence" (sometimes referred to in the art as "signal peptide") is use to denote an peptide, found at or near the N-terminus of the mature protein, usually about 15 to about 30 amino acids long, rich in hydrophobic amino acids, which facilitates secretion of the mature protein into the endoplasmic reticulum. It is in the endoplasmic reticulum, or cell membrane region, that initial glycosylation of protein occurs. Signal sequences are cleaved from the mature sequence prior to the secretion of the mature protein. See Watson et al., Molecular Biology of the Gene, 4th Ed., 1987, at page 731, (The Benjamin/Cummings Publishing Company, Inc., Menlo Park, California) herein incorporated by reference. In particular, various signal sequences which are not-naturally found operably linked to a naturally occurring leptin protein have been used, and have been found to improve glycosylation efficiency of multiply glycosylated leptin proteins.

For example, it has been found that, as compared to the native human leptin signal sequence, the signal sequence normally found connected to the tissue plasminogen activator sequence, when used in conjunction with the expression of various of the multiply-glycosylated leptin proteins described herein, results in higher levels of glycosylation (e.g., glycosylation moieties at all suitable sites in a higher proportion of the expressed molecules of mature protein).

Therefore, the present invention also relates to a method of manufacturing a glycosylated leptin protein comprising:

(a) culturing, under suitable conditions for expression, a host cell containing a DNA sequence encoding, in the 5' to 3' direction (i) a signal sequence, and (ii) a DNA encoding a glycosylated leptin protein; and
(b) obtaining said glycosylated leptin protein.

Further, as discussed above, the present invention relates to a method of manufacturing a glycosylated leptin protein wherein said signal is selected from among:

a) (SEQ.ID NO. 3)(native human leptin signal peptide) MHWGTLCGFLWLWPYLFYVQA
(b) (SEQ.ID NO. 4) (modified human leptin signal peptide) MHWGTLCGFLWLWPYLFYVSPS
(c) (SEQ.ID NO. 5) (modified human leptin signal peptide) MHWGTLCGFLWLWPYLFYVSP
(d) (SEQ.ID NO. 6)(modified human leptin signal peptide) MHWGTLCGFLWLWPYLFYVSPA
(e) (SEQ.ID NO. 7)(modified human leptin signal peptide) MHWGTLCGFLWLWPYLFYVSNS
(f) (SEQ.ID NO. 8)(native human tPA signal peptide) MDAMKRGLCCVLLLCGAVFVSPS
(g) (SEQ.ID NO. 9)(native human tPA signal peptide) MDAMKRGLCCVLLLCGAVFVSP
(h) (SEQ.ID NO. 10)(modified tPA signal peptide) MDAMKRGLCCVLLLCGAVFVSNS
(i) (SEQ.ID NO. 11)(modified tPA signal peptide) MDAMKRGLCCVLLLCGAVFVSPA
(j) (SEQ.ID NO. 12)(Leptin/tPA signal peptide) MHWGTLCCVLLLCGAVFVSPS
(k) (SEQ. ID NO. 13)(Leptin/tPA signal peptide) MHWGTLCCVLLLCGAVFVSP

Relatedly, one may use nucleic acid sequences encoding such signal peptides. The below DNA sequences, with the exception of the modified human leptin signal peptide signal sequence (d, SEQ. ID NO: 6) which was not done, were used as described in the working examples below to encode the corresponding signal peptides, as set forth above.
SEQ. ID NO: 14 (native human leptin signal peptide DNA)

```
ATGCATTGGGGAACCCTGTGCGGATTCTTGTGGCTTTGGCCCTATCTTTTCTATG
TCCAAGCT
```

SEQ. ID NO: 15 (modified human leptin signal peptide DNA)

```
ATGCATTGGGGAACCCTGTGCGGATTCTTGTGGCTTTGGCCCTATCTTTTCTATG
TTTCGCCCAGC
```

SEQ. ID NO: 16 (modified human leptin signal peptide DNA)

```
ATGCATTGGGGAACCCTGTGCGGATTCTTGTGGCTTTGGCCCTATCTTTTCTATG
TTTCGCCC
```

SEQ. ID NO: 17 (modified human leptin signal peptide DNA)

```
ATGCATTGGGGAACCCTGTGCGGATTCTTGTGGCTTTGGCCCTATCTTTTCTATG
TTTCGCCCGCT
```

SEQ. ID NO: 18 (modified human leptin signal peptide DNA)

```
ATGCATTGGGGAACCCTGTGCGGATTCTTGTGGCTTTGGCCCTATCTTTTCTATG
TTTCGAACAGC
```

SEQ. ID NO: 19 (native human tPA signal peptide DNA)

```
ATGGATGCAATGAAGAGAGGGCTCTGCTGTGTGCTGCTGCTGTGTGGAGCAGTCT
TCGTTTCGCCCAGC
```

SEQ. ID NO: 20 (native human tPA signal peptide DNA)

```
ATGGATGCAATGAAGAGAGGGCTCTGCTGTGTGCTGCTGCTGTGTGGAGCAGTCT
TCGTTTCGCCC
```

SEQ. ID NO: 21 (modified human tPA signal peptide DNA)

```
ATGGATGCAATGAAGAGAGGGCTCTGCTGTGTGCTGCTGCTGTGTGGAGCAGTCT
TCGTTTCGAACAGC
```

SEQ. ID NO: 22 (modified human tPA signal peptide DNA)

```
ATGGATGCAATGAAGAGAGGGCTCTGCTGTGTGCTGCTGCTGTGTGGAGCAGTCT
TCGTTTCGCCCGCT
```

SEQ. ID NO: 23 (Leptin/tPA signal peptide DNA)

```
ATGCATTGGGGAACCCTGTGCTGTGTGCTGCTGCTGTGTGGAGCAGTCTTCGTTT
CGCCCAGC
```

SEQ. ID NO: 24 (Leptin/tPA signal peptide DNA)

```
ATGCATTGGGGAACCCTGTGCTGTGTGCTGCTGCTGTGTGGAGCAGTCTTCGTTT
CGCCC
```

One may select signal sequences known to be associated with highly glycosylated proteins. Signal sequences which may be used are those native to erythropoietin, Factor VIII, beta-interferon, serum albumin, insulin, von Willebrand's factor, CD11α, IgG, follistatin, intrinsic factor, G-CSF, ceruloplasmin, LAMP-1, secreted hormones, growth factors and other proteins, human or non-human (such as other primate, mouse, rat, or other mammal), which are secreted in eukaryotic cells. For yeast cells, yeast α-factor, and others may be used. Also various other genes have leader sequences which may facilitate secretion of proteins in mammalian cells systems, such as human influenza virus A, human preproinsulin, and bovine growth hormone.

One may also optimize the amino acid compositions of the signal sequences on a trial and error basis to improve glycosylation efficiency, and prepare non-naturally occurring signal sequences. For example one may increase the number of hydrophobic amino acid residues or alter the signal peptidase cleavage site to increase the amount of time the protein spends in the membrane, in order to prolong the time period in which the protein is exposed to the cellular "machinery" which accomplishes glycosylation ("machinery" being a shorthand term for those enzymes and other moieties which perform glycosylation within the membrane region of the cell).

It has also been found that substitution of an existing cleavage site (the site at the carboxy-terminal end of a signal peptide at which the signal peptide is enzymatically cleaved to generate the mature protein) with a different cleavage sites may provide manufacturing advantages, particularly in mammalian cell systems, and increase glycosylation efficiency. Previously, those skilled in the art had altered enzyme cleavage sites of prosequences (see below) relating to signal peptides.

As will be demonstrated in the working examples below, use of the tissue plasminogen activator signal peptide to express a three site glycosylated leptin protein resulted in a higher glycosylation efficiency than use of the native human leptin signal peptide. It was further found that the cleavage site of the tPA (serine-proline-serine) signal peptide, when substituted into the native human leptin signal peptide, conferred improved glycosylation efficiency over the use of the non-modified native human leptin signal peptide.

For particular proteins, the site serine-asparagine-serine ("SNS") may function to improve the glycosylation efficiency. For example, as described herein, substitution of the natural human tPA signal peptide cleavage site with an "SNS" site resulted in a high yield of correctly cleaved glycosylated leptin protein (having glycosylation sites at positions 2, 47, 69, and 92). Other cleavage sites include, serine-proline-serine ("SPS"), serine-asparagine-serine ("SNS"), serine-proline ("SP"), and serine-proline-alanine ("SPA"). A new cleavage site may be substituted into any signal peptide by known methods, including site directed mutagenesis of encoding DNA, DNA synthesis, and alteration of genomic DNA within a cell. One may choose to manufacture a signal peptide, particularly a signal peptide not found in associated with any known secreted protein, such as natural signal peptides, and include a cleavage site as above to optimize or maximize glycosylation efficiency.

Some cleavage sites, such as the serine-proline-serine cleavage site of natural human tissue plasminogen activator ("tPA"), are incompletely cleaved from the N-terminal region of the mature protein. Thus, there leaves a serine residue at the N-terminus of the mature protein, herein referred to as the -1 position. The present invention also includes the present leptin glycosylated proteins having, or optionally having if one chooses to use a subject cleavage site, one or more amino acid residues at the N-terminus of the mature protein sequence.

The present invention also includes, more specifically, glycosylated leptin proteins having

a serine, arginine, proline or alanine residue at the -1 position,
a serine at the -1 position and a proline at the -2 position,
a serine-proline-serine sequence at the -1, - 2, and -3 positions,
a serine at the -1 position and an arginine at the -2 position,
a serine at the -1 position, an arginine at the -2 position and a serine at the -3 position,
an arginine at the -1 position and a serine at the -2 position; and,
an alanine at the -1 position and proline at the -2 positions.

In addition, there may be signal peptide cleavage sites which cleave a portion of the mature protein. Thus, as indicated above, the present invention includes truncated forms of glycosylated leptin protein, such as those having up to and including five amino acid residues deleted from the N-terminus of the mature protein, such as a leptin protein of SEQ. ID NO: 1 or 2, having the subject glycosylation sites.

Therefore, the present.invention also relates to an improved method of manufacturing a glycosylated protein comprising:

(a)culturing, under suitable conditions for expression and glycosylation, a host cell containing a DNA sequence encoding, in the 5' to 3' direction (i) a signal peptide, and (ii) a DNA encoding a glycosylated protein; and
(b) obtaining said glycosylated protein wherein said improvement comprises use of a signal peptide having a peptidase cleavage site optimized for glycosylation efficiency.

The non-naturally occurring cleavage site may be selected from among SPS, SP, SNS, and SPA. The signal peptides and glycosylated leptin proteins as set forth in the specification, including the working examples, are illustrative, although these methods and compositions are broadly applicable to a wide variety of proteins sought to be secreted and/or glycosylated by a eukaryotic cell. Such proteins include but are not limited to tissue plasminogen activator, Factor VIII and other blood clotting factors, erythropoietin and analogs thereof, and other glycosylated proteins.

It was also found that, in conjunction with use of the native leptin leader sequence, use of a "prosequence" may also improved glycosylation efficiency. A "prosequence" is an amino acid sequence optimally having the motif R-X-R/K-R, where "X" is any amino acid (and the one letter abbreviations are those conventionally used, see infra). The prosequence is cleavable (after the final R) with furin-like proteases normally present in CHO cells Watanabe et al., FEBS letters, 320: 215-218 (1993)(herein incorporated by reference). The ability of CHO cells to cleave such prosequences has been shown to be improved when furin expression plasmids are transfected into the cells. Yanagita et al., Endocrinology 133: 639-644 (1993)(herein incorporated by reference). For example, the mature human leptin sequence begins with a valine, which would interfere with removal of the presequence by furin. Better prosequence removal could be achieved by changing this valine to a more preferred amino acid, such as serine or alanine, or inserting such an amino acid before the valine (by, e.g., site-directed mutagenesis or other methods available to those skilled in the art). Therefore, the present methods also optionally include use of such prosequences in conjunction with the natural leptin signal peptide or with other signal peptides.

The present invention also encompasses compositions, such as nucleic acids, vectors, and host cells, such as those recited above and herein incorporated by reference, which contain nucleic acids encoding the present altered signal peptides and/or pro sequences.

EXAMPLES

The following examples are offered to more fully illustrate the invention, but are not to be construed as limiting the scope thereof.

Example 1 demonstrates the Stokes' radius measurement of various glycosylated leptins.

Example 2 demonstrates the in vivo biological activity of a one-site glycosylated leptin, denoted "N48T50". This example demonstrates that this glycosylated leptin has activity at least equal to native recombinant human leptin lacking glycosylation.

Example 3 demonstrates the in vitro biological activity of additional one site glycosylated leptins.

Example 4 demonstrates in vitro biological activity of two-site glycosylated leptin proteins, in terms of a receptor binding assays.

Example 5 demonstrates the effect on glycosylation efficiency of using a threonine residue, rather than a serine residue, in the glycosylation consensus sequence.

Example 6 demonstrates that amino acids adjacent to the consensus sequence affect glycosylation efficiency.

Example 7 that a three site glycosylated leptin protein has a substantially longer systemic circulation time than non-glycosylated leptin.

Example 8 demonstrates in ob/ob mice that a three site glycosylated leptin protein has improved weight loss biological activity as compared to non-glycosylated leptin.

Example 9 demonstrates in ob/ob mice that a three site glycosylated leptin protein has improved appetite suppressant biological activity as compared to non-glycosylated leptin.

Example 10 demonstrates in ob/ob mice that intermittent administration of a three site glycosylated leptin has improved weight loss biological activity as compared to non-glycosylated leptin.

Example 11 provides additional dose response studies using a three site glycosylated leptin on wild type animals, demonstrating that a far lower dose of the three site glycosylated leptin results a substantial weight loss, as compared to non-glycosylated leptin.

Example 12 provides dose frequency studies using a three site glycosylated leptin on wild type mice, and demonstrates that a three site glycosylated leptin may be dosed less frequently than non-glycosylated leptin to obtain the same weight loss response in animals.

Example 13 sets forth additional multiple glycosylation site leptin proteins and in vitro biological activity data.

Example 14 sets forth the expression and glycosylation efficiency of a three site glycosylated leptin protein using a variety of signal peptides and other sequences affecting glycosylation or yield.

Example 15 sets forth additional expression data on a variety of multiple glycosylation site leptin proteins using various signal peptides and other sequences affecting glycosylation or yield.

Reference Examples of methods used herein follow.

EXAMPLE 1

Stokes' Radius of Various Leptins

The present example demonstrates that various leptins have different Stokes' radii, as determined by gel filtration. The present example also demonstrates the consistency of the gel filtration method for determining the Stokes' radius of a single leptin glycosylated protein, as when repeated measurements were taken, the measurements varied by less than 2 Å.

Methods: Gel filtration experiments were carried out on a Pharmacia FPLC system equipped with a Unicorn controller for system control, data acquisition and analysis, a UV-1 detector and a 280 nm filter. Separations were performed at 4°C and at a flow rate of 0.25 ml/min on a SuperDex 200 (HR10/30) column equilibrated in Dulbeccos phosphate buffered saline. Protein samples, dissolved in elution buffer, were applied to the column in 0.25 ml volumes containing 0.1 A280 as determined by a Hewlett Packard Model 8435 Spectrophotometer.

The standard proteins found in the Pharmacia Gel Filtration Calibration Kits, both High Molecular Weight and Low Molecular Weight, were used as recommended by the manufacturer to calibrate the columns. (As recommended by the manufacturer, catalase was not used as a standard.) Additional standards, including human transferrin (36A), soybean trypsin inhibitor (22Å) and horse muscle myoglobin (19Å) were purchased from Sigma Chemicals. Blue Dextran (Pharmacia Gel Filtration Calibration Kit) was used to define the void volume. Values for the Stokes' radius (Rs) for any of the various leptin forms were calculated from a plot of $\sqrt{-\log(Kav)}$ vs Rs where Kav = (Ve-Vo)/Vt-Vo and Ve is the elution volume of the protein, Vo is the void volume, and Vt is the total bed volume of the column.

Results: Using the above methods, and SuperDex 200™ as the gel filtration material, the following Stokes' radii were determined for rHu-Leptin 1-146 (SEQ. ID NO: 1, below) having the following glycosylation sites:

Table 1.1: Stokes' Radius of Various Leptins

| | |
|---|---|
| rmetHu-leptin 1-146 | 18.1 Å |
| rHu-Leptin 1-146 N48T50 | 23Å |
| rHu-leptin 1-146 N33T35 | 24 Å |
| rHu-leptin 1-146 N47, 69, 102 | 31.9 Å |
| rHu-leptin N47, 69, 102 | 32.3 Å |

As can be seen, a population of three site glycosylated leptin protein molecules has a Stokes' radius above 30 Å, as determined by gel filtration. The mean Stokes' radius ((31.9 + 32.3)/2)) is 32.1 Å. The present gel filtration method furthermore demonstrated consistency to one Angstrom. As a comparison, the same glycosylated leptin protein had Stokes' radius of 31.2 Å when determined by sedimentation velocity (using standard methods not detailed here).

The unglycosylated leptin protein (rmetHu-Leptin), as well as the two single site glycosylated leptin proteins, had Stokes' radii less than 30 Å. As will be demonstrated in the working examples below, the N48T50 glycosylated leptin protein had biological activity comparable to rmetHu-Leptin. The three-site glycosylation protein (47, 69, 102) had substantially improved biological activity in terms of increased circulation time (and therefore increase in vivo exposure to drug). This demonstrates the principle that enlarging the effective size (expressed here as the Stokes' radius) prolongs circulation time by decreasing the filterability and ultimate degradation in the kidney.

EXAMPLE 2

In vivo Biological Activity and Serum Circulation Time of a One-Site Glycosylated Leptin, N48T50

This Example demonstrates that a one-site glycosylated leptin has biological activity approximately the same or modestly improved from rmetHu-Leptin 1-146 (SEQ. ID NO: 1). The glycosylation did not hinder activity or prevent receptor binding. Further, there is demonstrated that the serum circulation time of a one-site glycosylated leptin is the same or modestly longer than rmetHu-Leptin 1-146 (SEQ. ID NO: 1).

Animals were administered the one-site glycosylated leptin or rmetHu-Leptin at the same dose daily for 7 days. At the end of 7 days, animals were sacrificed, and fat content examined. As compared to rmetHu-Leptin, administration of the one-site glycosylated leptin resulted in approximately 25% additional fat loss. This demonstrates that the present glycosylated leptin compositions having a non-naturally occurring glycosylation site retains biological activity.

Methods:

1. Leptin Compositions Used. This glycosylated leptin, "N48T50" had the amino acid sequence of native human leptin 1-146 (SEQ. ID NO: 1) with the isoleucine ("I") at position 48 substituted with asparagine ("N"), and the next two amino acids remaining (leucine ("L") and threonine ("T")) without substitution. For daily dosing groups (100 ul injection volume for all): 0.2 mg/ml for 1 mg/kg dose group, 2.0 mg/ml for 10 mg/kg dose group. For day-0-only dosing groups: 5 mg/ml concentration, 400 ul injected, 100 mg/kg dose.

2. Animals:

Number and type. 5 female C57BL/6 mice, from Charles River Laboratories (Wilmington, MA)
Age and weight: Animals were age 8-10 weeks and weighed approximately 20 g each.

3. Administration. At the beginning of each study, mice were weighed and then injected with sample in bolus subcutaneously.

Weighing: Baseline weight was determined in animals allowed to acclimate in animal facility for 1 week prior to study. Baseline weight was taken just before receiving the first dose. Weights were monitored daily throughout each study. After final weights were recorded, animals were sacrificed and the amount of abdominal fat was graded from 0-3, with 0 being no visible fat remaining, and a score of 3 reflecting an amount of visible fat in a normal animal.

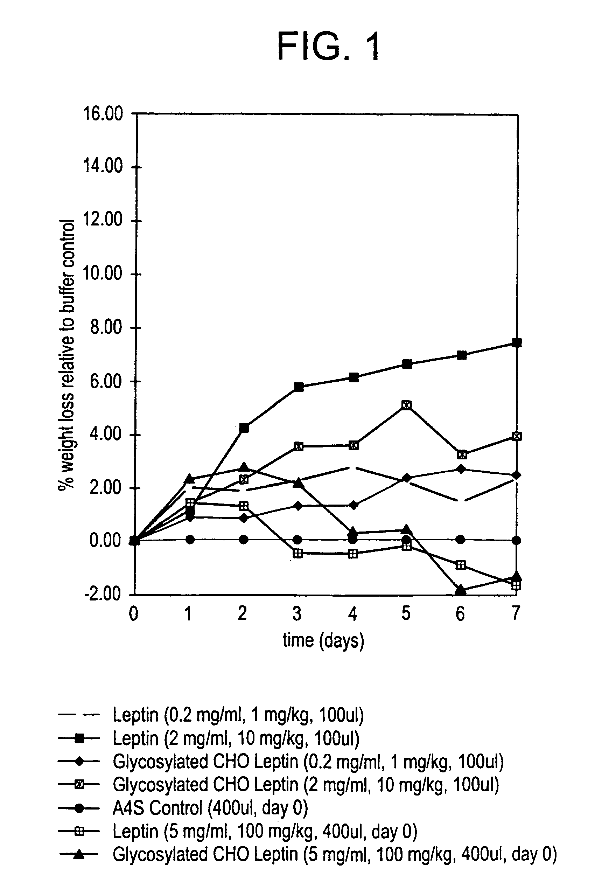

Results: Mice treated daily with rmetHu-leptin (1-146) expressed in E.coli lost weight relative to buffer controls as shown in FIGURE 1. Surprisingly the glycosylated leptin (N48 T50 Leptin) treated mice also lost weight. The amount of weight loss increased with increasing dose (1 mg/kg and 10 mg/kg) for both forms of leptin. When single injections of 100 ug/kg were done, the glycosylated leptin treated mice lost more weight than rmetHu-leptin (1-146) treated mice. In addition the weight loss persisted longer for the glycosylated leptin treated mice than for rmetHu-leptin treated mice. Visual examination of rmetHu-leptin and N48 T50 Leptin treated mice indicated that mice treated with both forms of leptin had reduced amounts of abdominal fat and that the amount of abdominal fat was reduced with increased dose. This indicates that the glycosylated leptin is effective at reducing fat content and it can be administered less frequently than unglycosylated rmetHu-leptin.

Pharmacokinetic Studies of a One-Site Glycosylated Leptin

This study demonstrates that for intravenous administration, a single-site glycosylated leptin has a longer half life than non-glycosylated leptin. For subcutaneous administration, the circulation times were similar for both the glycosylated and non-glycosylated leptin.

Materials:

1. Leptin. A one site glycosylated leptin as above (sites N48 T50) was used, formulated at 10 mg/ml in Dulbecco's Phosphate Buffered Saline without calcium chloride without magnesium chloride. Recombinant methionyl human leptin 1-146 (SEQ. ID NO: 1 with a methionyl residue at position -1), expressed in E. coli was used as a control, formulated at 2.0 mg/ml in buffer.
2. Animals.
Number used/type: 32 (for glycosylated leptin protein) and 81 (for r-metHu-leptin) male CD-1 mice(Charles River Laboratories, Hollister, CA)
Age/weight: Animals were approximately 6-9 weeks old and weighed approximately 30 grams.
Care/handling: Animals were individually housed and fed a diet of laboratory rodent chow ad libitum. All animals were handled in accordance with good animal handling practices.
3. Administration. Animals were injected with glycosylated leptin at a dose of 1.0 mg/kg body weight intravenously (IV) or subcutaneously (SC).
4. Sampling. Animals were anesthetized, and blood samples were collected at designated time points using standard cardiac puncture techniques. The serum concentrations of glycosylated leptin were determined using an immunoassay (as described below).
5. Comparison. The circulation time data were compared to previously obtained data for rmetHu-Leptin, at the same dose, in similarly sized animals, using the same routes of administration.

Results:

Table 2.1 shows the pharmacokinetic parameters of glycosylated Leptin and rmetHu-Leptin in mice. Comparing the IV data, glycosylated Leptin exhibited a lower systemic clearance (500 mL/h/kg vs. 676 mL/h/kg) and a longer terminal half-life (1.24 h vs. 0.733 h). The volumes of distribution at steady-state ($V_{ss}$) were similar between glycosylated Leptin and rmetHu-Leptin. These data indicate that the glycosylated protein was cleared slower than rmetHu-Leptin from the systemic circulation, therefore increasing the half-life and the exposure (AUC estimates of 2000 ng•h/mL vs. 1480 ng•h/mL). Following the SC dose, similar peak serum concentrations ($C_{max}$) were obtained between glycosylated Leptin and rmetHu-Leptin (1230 ng/mL vs. 1380 ng/mL), although there was a delay in the peak time ($t_{max}$) for glycosylated leptin. Similar exposure estimates (based on AUC) were obtained for both molecules. Subcutaneous bioavailability was approximately 60.5% for the glycosylated leptin vs. 79.6% for rmetHu-Leptin.

## Table 2.1

### Pharmacokinetic Parameters of Glycosylated Leptin(N48,T50) and rmetHu-Leptin Following IV and SC Administration

| | Glycosylated Leptin | Leptin | Ratio (Glycosylated Leptin/Leptin) |
|---|---|---|---|
| **SC Dose** | | | |
| $t_{max}$ (h) | 0.5 | 0.167 | --- |
| $C_{max}$ (ng/mL) | 1230 | 1380 | 0.89 |
| AUC (ng•h/mL) | 1210 | 1180 | 1.03 |
| $t_{1/2, lz}$ (h) | 0.552 | 0.541 | 0.96 |
| F (%dose) | 60.5 | 79.6 | 0.760 |
| **IV Dose** | | | |
| AUC (ng•h/mL) | 2000 | 1480 | 1.35 |
| $t_{1/2, lz}$ (h) | 1.24 | 0.733 | 1.69 |
| CL (mL/h/kg) | 500 | 676 | 0.74 |
| $V_{ss}$ (mL/kg) | 149 | 150 | 0.99 |

EXAMPLE 3

In vitro biological activity of other one-site glycosylated leptins

In Table 3.1, below, the amino acid sequence location for alteration to include a glycosylation site is based on the numbering of SEQ. ID NO: 1, above, which is rHu-Leptin.

Protein was expressed as in the Reference Examples below, using the natural human leptin signal peptide and COS cells. The expression products were then put through four types of analysis (methods used are described below):

1. Expression relative to wild type. The yield of protein was compared relative to rHu-Leptin 1-146, as expressed in COS cells. The amount of rHu-Leptin 1-146 was assigned the number of "1.00" under conditions as defined below.

2. Percent glycosylation. The yield of fully glycosylated protein was determined as a percent of total leptin protein by visual inspection of a Western Blot, as described below.

3. Binding, Leptin-R, relative to wild type. In an in vitro competition assay using a preparation of leptin receptor, radio-labeled glycosylated leptin proteins prepared were compared to radio-labeled rHu-Leptin 1-146 in strength of binding to leptin receptor, according to methods described below.

4. In vitro bioactivity relative to wild type. In an in vitro assay using a chimeric leptin receptor, as described below, glycosylated leptin proteins prepared were compared to rHu-Leptin 1-146, according to methods described below. "ND" means that the data are not available because experiments were not done.

Table 3.1

| Summary of COS Single Site Glycosylated Leptin expression, binding, and glycosylation results | | | | | |
|---|---|---|---|---|---|
| Position of N glycosylation 1/ | Sequence Changes | Expression Rel. to WT | % Glycosylation | Receptor binding Rel. to WT. | Bioactivity Rel. to WT. |
| None | wild type | 1 | 0 | 1 | 1 |
| 4 | QKV > NKV | 1.7 | 0 | 1.7 | 0.73 |
| 5 | KVQ > NVT | 0.33 | 65 | 1.7 | 0.05 |
| 7 | QDD > NDT | 0.55 | 5 | 1.25 | 0.04 |
| 8 | DDT > NDT | 1.1 | 15 | 1.2 | 1.2 |
| 23a | DIS > NIT | 7.8 | 60 | ND | 0.53 |
| 23b | DIS > NIS | ND | ND | ND | ND |
| 25 | SHT > NHT | 0.13 | 80 | 0.4 | 0.02 |
| 26 | HTQ > NTT | 1.1 | 70 | 1.5 | 0.01 |
| 27 | TQS > NQT | 0.45 | 30 | 0.7 | 0.13 |
| 29 | SVS > NVT | 0.5 | 70 | 0.6 | 0.5 |
| 33 | KQK > NQT | 1.6 | 95 | 0.9 | 0.04 |
| 35 | KVT > NVT | 0.55 | 95 | 0.5 | 0.13 |
| 37 | TGL > NGT | 1.4 | 95 | 0.4 | 0.043 |
| 38 | GLD > NLT | 0.26 | 45 | 0.2 | 0.036 |
| 43 | PGL > NGT | 1.6 | 85 | 1.5 | 0.014 |
| 44 | GLH > NLT | 1.8 | 10 | 2 | 0.78 |
| 45 | CHP > NHT | 0.52 | 85 | 0.5 | 0.08 |
| 46 | HPI > NPT | 1.4 | 0 | 0.11 | 0.27 |
| 47 | PIL > NIT | 1.06 | 80 | 0.66 | 0.84 |
| 48 | ILT > NLT | 0.92 | 50 | 0.8 | 0.53 |
| 67 | SMP > NMT | 1.1 | 15 | 0.8 | 0.52 |
| 68 | MPS > NAT | 0.5 | 80 | 0.8 | 0.036 |
| 69 | PSR > NST | 0.8 | 75 | 0.6 | 1.1 |
| 70 | SRN > NRT | 1.07 | 10 | 1 | 1 |
| 71 | RNV > NNT | 1.84 | 60 | 1.9 | 0.3 |
| 72 | NVI > NVT | 1.4 | 70 | 1.7 | 0.26 |
| 73 | VIQ > NIT | 0.53 | 45 | 8 | 0.01 |
| ESP77* | SND > NNT | 0.14 | 10 | 2.2 | <0.02 |
| 92 | FSK > NST | 4.8 | 45 | ND | 0.67 |
| 93 | SKS > NKT | 2.4 | 5 | ND | 1.1 |
| 97 | HLP > NLT | 2.6 | 10 | ND | 1.1 |
| 99 | PWA > NWT | 0.45 | 0 | 0.9 | 0.5 |
| 100a | PWAS > SNAT | 0.43 | 35 | 0.6 | 0.9 |
| 100b | WAS > NAS | 1.2 | 0 | 0.7 | 1.46 |

Table continued

| Summary of COS Single Site Glycosylated Leptin expression, binding, and glycosylation results | | | | | |
|---|---|---|---|---|---|
| Position of N glycosylation 1/ | Sequence Changes | Expression Rel. to WT | % Glycosylation | Receptor binding Rel. to WT. | Bioactivity Rel. to WT. |
| 100c | WAS > NAT | 0.5 | 20 | 0.45 | 0.81 |
| 100d | PWAS > ANAT | 2.3 | 35 | 1.45 | 0.26 |
| 100e | WAS > TAS | 1 | 60 (O-linked) | > 1.9 | 0.83 |
| 101 | ASG > NST | 0.59 | 50 | 0.7 | 0.33 |
| 102 | SGL > NGT | 0.79 | 60 | 0.6 | 0.85 |
| 103 | GLE > NLT | 1.6 | 55 | 1 | 0.73 |
| 115 | EAS > NAT | 1.1 | 70 | 0.9 | 0.006 |
| 116 | ASG > NST | 1.1 | 0 | 0.9 | 0.56 |
| 117 | SGY > NGT | 1.33 | 50 | 1.9 | 0.01 |
| 118 | GYS > NYT | 1.44 | 15 | 0.8 | 3.8 |
| 119 | YST > NST | 0.61 | 70 | 0.8 | 0.02 |
| 120 | STE > NTT | 0.9 | 100 | 0.7 | 0.01 |
| 141 | DLS > NLT | 0.18 | 0 | 0.3 | 1 |
| * ESP77 indicates a glycosylation site at position 77 and expression using the signal peptide of erythropoietin, as described in more detail infra | | | | | |

1/ "Position" indicates the amino acid position according to SEQ. ID NO: 1, which is rHu-Leptin 1-146. The particular sequence listed (e.g., "53", "55", etc.) indicates the "N" position in the consensus glycosylation sequence of "N - X - S/T".

Results: As can be seen, with the single-site glycosylation leptin proteins prepared, compared to unglycosylated leptin, most single site glycosylated leptin proteins showed no substantial increase in biological activity as determined by the in vitro assay used herein, except for the protein with a glycosylation site at position 118, which appeared to have an increased amount of activity. Some of these analogs were secreted at normal or higher levels and most had receptor binding activity comparable to rHu-Leptin 1-146 expressed and analyzed in the same manner. Surprisingly some of the glycosylated leptin proteins had low in vitro biological activity even though they retained receptor binding activity. Thus the glycosylated leptin proteins could be divided into 2 classes according to whether they retained in vitro biological activity or not. The glycosylated leptin proteins that had low in vitro biological activity may be leptin antagonists.

EXAMPLE 4

In vitro biological activity of two-site glycosylated leptins

As presented in Table 4.1, various two-site glycosylated leptin proteins were also produced and tested as above for the single site proteins. Notations and abbreviations are the same as those for the Table 3.1 for the one-site proteins.

The glycosylation notations indicate the approximate percent of material which had one chain or two chain, as determined by visual examination of a Western Blot, as described below. For example, for the glycosylated leptin protein 25 + 29, 50% of the material had one chain, and 5% of the material had two chains.

Table 4.1

| Summary of COS Leptin Double Site Expression, Binding, and Glycosylation Results | | | | |
|---|---|---|---|---|
| Position of N-glycosylation | Expression Rel. to WT | Glycosylation | Receptor binding Rel. to WT. | Bioactivity Rel. to WT. |
| None | 1 | 0 | 1 | 1 |
| 25+29 | 1 | 1'-50,2'-5 | 1 | ND |

Table continued

| Summary of COS Leptin Double Site Expression, Binding, and Glycosylation Results | | | | |
|---|---|---|---|---|
| Position of N-glycosylation | Expression Rel. to WT | Glycosylation | Receptor binding Rel. to WT. | Bioactivity Rel. to WT. |
| 25+33 | 1.2 | 1'-40,2'-60 | 1.6 | ND |
| 25+35 | 1.2 | 1'-70,2'-10 | 1 | ND |
| 26+33 | 0.46 | ND | 1.6 | ND |
| 26 + 35 | 0.59 | 1'-45,2'-35 | 1 | ND |
| 27+33 | 0.93 | 1'-60,2'-30 | 1 | ND |
| 27+35 | 1.1 | 1'-50,2'-40 | 0.7 | ND |
| 29+33 | 1.6 | 1'-50,2'-45 | 1.4 | ND |
| 29+35 | 0.33 | 1'-33,2'-33 | 0.6 | ND |
| 33+48 | 0.86 | 1'-35,2'-60 | 0.6 | ND |
| 33+120 | 0.27 | 1'-5,2'-95 | 0.89 | <0.003 |
| 35+48 | 0.46 | 1'-30,2'-40 | 0.6 | ND |
| 47+69 | 1.3 | 1'-10,2'-50 | 1 | 0.69 |
| 47+102 | 2.7 | 1'-50,2'-30 | 0.86 | 0.42 |
| 48+69 | 1.63 | 1'-20,2'-50 | 0.87 | 0.81 |
| 69+101 | 1.2 | 1'-45,2'-20 | 0.42 | 0.66 |
| 69+102 | 1.7 | 1'-40,2'-30 | 0.5 | 0.63 |
| 69+103 | 2.6 | 1'-50,2'-15 | 1.7 | 0.67 |
| 69+118 | 2.9 | 1'-50.2'-5 | 2.2 | 2.3 |
| 102+100e | 1.8 | 2'-60 | 0.62 | 0.97 |

Many glycosylated leptin proteins containing combinations of two glycosylated sites can be made which retain receptor binding and display biological activity.

Example 5

Improvement of Glycosylation Efficiency Using a Threonine Rather Than a Serine in the Consensus Sequence

This example demonstrates that the glycosylation site efficiency is improved using a threonine rather than a serine in the glycosylation consensus sequence. In this Example, all amino acid sequence locations for alterations to include glycosylation sites are based on the numbering of SEQ. ID NO: 1, which is rHu-Leptin 1-146.

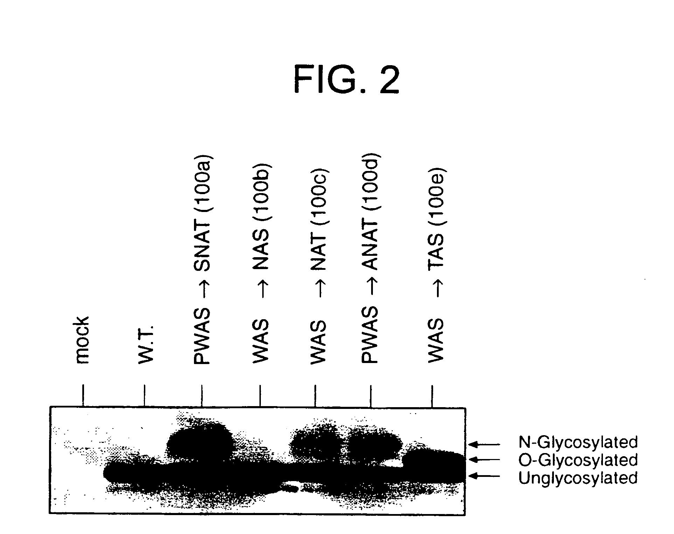

Glycosylated leptin proteins were constructed, expressed, and analyzed using methods in the Reference Examples, below. The results are shown in FIGURE 2. The introduction of a single glycosylation site by the double substitution W100,S102 to N100,T102 resulted in addition of N-linked carbohydrate and the proportion of molecules containing carbohydrate (by SDS PAGE as determined by Western blotting) was substantially more than with a W100,S102 to N100,S102 substitution. This indicates that more of the protein molecules having a threonine in the consensus sequence were glycosylated than those having a serine in the consensus sequence. Thus, the glycosylation efficiency in this expression system is higher using the consensus sequence Asn-Xxx-Thr than when Asn--Xxx-Ser is used. As such, use of a threonine residue is preferred.

EXAMPLE 6

Glycosylation Efficiency Is Effected by Upstream Sequence

This example shows that glycosylation efficiency is affected by both the amino acid in the -1 position (relative to the substituted asparagine residue) as well as substitution of a proline immediately "upstream" (i.e., toward the N-terminus) from the asparagine residue in the consensus sequence.

It was found that rHu-Leptin 1-146 with alterations of: S99, N100, S102 was more efficiently glycosylated than the same alterations lacking the serine substitution at position 99. This indicates that substitutions around the consensus glycosylation site can result in additional improvement in glycosylation site occupancy.

In addition, and surprisingly, a W100 to T100 substitution resulted in O-glycosylation of the leptin presumably at position 100. This indicates that either O-linked or N-linked carbohydrate can be added to the same position depending on the particular substitution that is made. FIGURE 2 is a Western Blot comparing the N-linked glycosylation site to the O-linked glycosylation site, as indicated. As can be seen, use of the sequence "TAS" as indicated (with reference to SEQ. ID NO: 1) results in O-linked glycosylation.

EXAMPLE 7

Improved systemic circulation time of a three site glycosylated leptin

This Example demonstrates that a glycosylated leptin having greater than one glycosylation site has a circulation time which is substantially longer than non-glycosylated recombinant human leptin. As can be seen, glycosylated Leptin exhibited a 4- to 5-fold decrease in systemic clearance and increase in half-life compared to rmetHu-Leptin. Although there was a small decrease in subcutaneous bioavailability (~10% decrease as compared to non-glycosylated), glycosylated leptin still resulted in a higher drug exposure following subcutaneous dosing.

Materials:

1. Leptin. A three site glycosylated leptin as prepared below (sites 47, 69 and 102, SEQ. ID NO: 32) was used, formulated at 1.76 mg/ml in Dulbecco's Phosphate Buffered Saline without calcium chloride without magnesium chloride (Gibco). Recombinant methionyl human leptin 1-146 (SEQ. ID NO: 1 with a methionyl residue at position -1), expressed in E. coli was used as a control, formulated at 2.0 mg/ml in buffer.

2. Animals.
Number used/type: 27 (for glycosylated leptin protein) and 81 (for r-metHu-leptin) male CD-1 mice(Charles River Laboratories, Hollister, CA)
Age/weight: Animals were approximately 6-9 weeks old and weighed approximately 30 grams.
Care/handling: Animals were individually housed and fed a diet of laboratory rodent chow ad libitum. All animals were handled in accordance with good animal handling practices.

3. Administration. Animals were injected with glycosylated leptin at a dose of 1.0 mg/kg body weight intravenously (IV) or subcutaneously (SC).

4. Sampling. Animals were anesthetized, and blood samples were collected at designated time points using standard cardiac puncture techniques. The serum concentrations of glycosylated leptin were determined using an immunoassay (as described below).

5. Comparison. The circulation time data were compared to previously obtained data for rmetHu-Leptin, at the same dose, in similarly sized animals, using the same routes of administration.

Results: Results are illustrated in FIGURES 3 and 4. FIGURE 3 is a graph showing the serum leptin concentration after subcutaneous administration, and FIGURE 4 is a graph showing serum leptin concentration after intravenous administration.

In general, following both IV and SC administration of glycosylated leptin having a Stokes' radius greater than about 30 Å, serum concentrations were higher than those observed for rmetHu-Leptin as well as that for a single-site glycosylated leptin (N48 T50). For rmetHu-Leptin, serum concentrations declined below 1.0 ng/mL within 6 hours after both routes of administration; whereas serum concentrations of glycosylated Leptin remained above 1.0 ng/mL for 24 hours after IV or SC administration.

Table 7.1 shows the pharmacokinetic parameters of glycosylated leptin and rmetHu-Leptin in mice.

Table 7.1

Pharmacokinetic Parameters of Glycosylated Leptin and rmetHu-Leptin Following IV and SC Administration

|  | Glycosylated Leptin | Leptin | Ratio (Glycosylated Leptin/Leptin) |
|---|---|---|---|
| **SC Dose** |  |  |  |
| $t_{max}$ (h) | 1 | 0.167 | --- |
| $C_{max}$ (ng/mL) | 1430 | 1380 | 1.04 |
| AUC (ng•h/mL) | 5800 | 1180 | 4.93 |
| $t_{1/2, lz}$ (h) | 2.21 | 0.541 | 4.09 |
| F (% dose) | 69.5 | 79.6 | 0.873 |
| **IV Dose** |  |  |  |
| AUC (ng•h/mL) | 8350 | 1480 | 5.65 |
| $t_{1/2, lz}$ (h) | 2.76 | 0.733 | 3.77 |
| CL (mL/h/kg) | 120 | 676 | 0.178 |
| $V_{ss}$ (mL/kg) | 157 | 150 | 1.05 |

Comparing the IV data (see FIGURE 4), glycosylated leptin exhibited a lower systemic clearance (120 mL/h/kg vs. 676 mL/h/kg) and a longer terminal half-life (2.76 h vs. 0.733 h). The volumes of distribution at steady-state ($V_{ss}$) were similar between glycosylated leptin and rmetHu-Leptin. These data indicate that the glycosylated protein was cleared slower than rmetHu-Leptin from the systemic circulation, therefore increasing the half-life and the exposure (AUC estimates of 8350 ng•h/mL vs. 1480 ng•h/mL). Following the SC dose, (see FIGURE 3) similar peak serum concentrations ($C_{max}$) were obtained between glycosylated leptin and rmetHu-Leptin (1430 ng/mL vs. 1380 ng/mL), although there was a delay in the peak time ($t_{max}$) for glycosylated leptin. Similar to the results obtained from IV administration, subcutaneously administered glycosylated leptin exhibited an increased terminal half-life (2.21 h vs. 0.541 h) and area under the curve ("AUC") (5800 ng•h/mL vs. 1180 ng•h/mL), probably due to the decreased systemic clearance for glycosylated leptin. Subcutaneous bioavailability was approximately 69.5% for the glycosylated leptin vs. 79.6% for rmetHu-Leptin.

EXAMPLE 8

Improved Weight Loss Activity of Three-Site Glycosylated Leptin

This Example demonstrates that a glycosylated human leptin having a Stokes' radius greater than 30 Å has improved in vivo biological activity as compared to non-glycosylated recombinant human leptin. As can be seen, with daily administration after 7 days, ob/ob mice lost ~6.8 times more weight with the three-site glycosylated leptin here administered than with non-glycosylated leptin.

Methods:

1. Leptin. The three-glycosylation site leptin, prepared as described below (sites 47, 69 and 102, SEQ. ID NO: 32),

was used, formulated at a concentration of 1.9 mg/ml in Dulbecco's phosphate-buffered saline, pH 6.8. Recombinant methionyl human leptin 1-146 (SEQ. ID NO: 1 with a methionyl at position -1) was used as a basis for comparison, formulated at 20 mg/ml in 10 mM sodium acetate with 5% sorbitol, pH 4.0. Ten mM sodium acetate with 5% sorbitol, pH 4.0, was used as a vehicle control.

2. <u>Animals.</u> Animals were housed in a temperature-, light-, and humidity-controlled conditions, with lights on at 0600 hours and lights off at 1800 hours. The Amgen, Inc. animal research facility is approved by the USDA and AAALAC-accredited. Six female *ob/ob* mice (Jackson Laboratories) were used per treatment group. Mice were 2 months old at the time of study, and weighed an average of 45.6 grams. Mice were randomized to treatment groups such that the mean body weights of the groups were equivalent prior to treatment initiation. Animals were housed two per cage and fed standard laboratory rodent chow pellets <u>ad libitum.</u>

3. <u>Administration.</u> All treatment procedures were approved by Amgen's Institutional Animal Care And Use Committee. Glycosylated leptin, r-metHu-Leptin, or placebo were administered daily via subcutaneous injection in the mid-scapular region in a volume of 0.1 ml. The dose of leptin was 0.5 mg/kg body weight/day, for both glycosylated leptin and r-metHu-Leptin. Injections were given on 7 consecutive days, beginning on study day 0, in the late afternoon (within 2 hours of lights off in the colony). Animals were weighed daily at the time of injection. All data are reported as the mean $\pm$ SE.

<u>Results:</u>

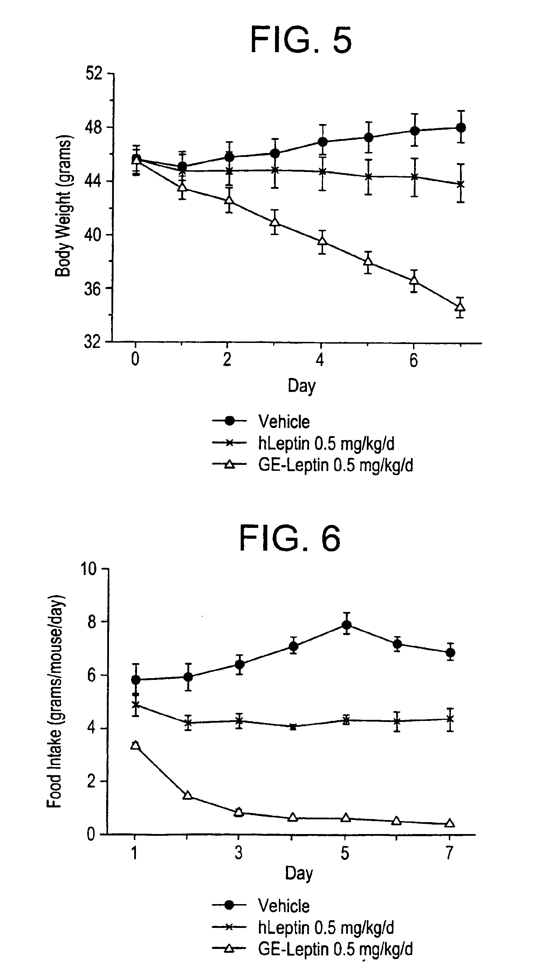

As can be seen in FIGURE 5, the three-site glycosylated leptin ("GE-Leptin") resulted in the largest amount of weight loss, with an average weight loss of 10.8 $\pm$ 0.3 grams (-23.8 $\pm$ 0.5 % of initial body weight). Administration of the same dose of r-metHu-Leptin ("hLeptin") produced an average weight loss of 1.6 $\pm$ 0.4 grams (-3.5 $\pm$ 1.1 % of initial body weight), while administration of placebo resulted in an average weight gain of 2.6 $\pm$ 0.2 grams (5.7 $\pm$ 0.3 % of initial body weight). This Example demonstrates a substantially improved <u>in vivo</u> biological activity of glycosylated leptin as compared to non-glycosylated recombinant human leptin.

<u>EXAMPLE 9</u>

Improved Appetite Suppressant Activity of Three-Site Glycosylated Leptin

This Example demonstrates that a glycosylated human leptin having a Stokes' radius greater than 30 Å has improved <u>in vivo</u> biological activity as compared to non-glycosylated recombinant human leptin. As can be seen, with daily administration after 7 days, *ob/ob* mice ate ~11 times less food with the glycosylated leptin than with non-glycosylated leptin.

<u>Methods:</u>

1. <u>Leptin.</u> The three-glycosylation site leptin, prepared as described below (sites 47, 69 and 102, SEQ. ID NO: 32), was used, formulated at a concentration of 1.9 mg/ml in Dulbecco's phosphate-buffered saline, pH 6.8. Recombinant methionyl human leptin 1-146 (SEQ. ID NO: 1 with a methionyl at position -1) was used as a basis for comparison, formulated at 20 mg/ml in 10 mM sodium acetate with 5% sorbitol, pH 4.0. Ten mM sodium acetate with 5% sorbitol, pH 4.0, was used as a vehicle control.

2. <u>Animals.</u> Animals were housed in a temperature-, light-, and humidity-controlled conditions, with lights on at 0600 hours and lights off at 1800 hours. The Amgen, Inc. animal research facility is approved by the USDA and AAALAC-accredited. Six female *ob/ob* mice (Jackson Laboratories) were used per treatment group. Mice were 2 months old at the time of study, and weighed an average of 45.6 grams. Mice were randomized to treatment groups such that the mean body weights of the groups were equivalent prior to treatment initiation. Animals were housed two per cage and fed standard laboratory rodent chow pellets <u>ad libitum.</u>

3. <u>Administration.</u> All treatment procedures were approved by Amgen's Institutional Animal Care And Use Committee. Glycosylated leptin, r-metHu-Leptin, or placebo were administered daily via subcutaneous injection in the mid-scapular region in a volume of 0.1 ml. The dose of leptin was 0.5 mg/kg body weight/day, for both glycosylated leptin and r-metHu-Leptin. Injections were given on 7 consecutive days, beginning on study day 0, in the late afternoon (within 2 hours of lights off in the colony.

4. <u>Food Measurement.</u> Food intake was measured daily at the time of injection by weighing the amount of food in each animal's cage each day. Food intake is reported as the grams eaten per mouse per day, and was calculated as follows: (weight of the food in the cage the previous day - weight of the food that day)/the number of mice per cage (two). All data are reported as the mean $\pm$ SE.

<u>Results:</u>

As can be seen in FIGURE 6, administration of the three-site glycosylated leptin resulted in the largest reduction of food intake, with an average food intake of 0.4 $\pm$ 0.04 grams/mouse/day for the final 24-hour period after the seventh dose. Administration of recombinant methionyl human leptin produced a reduction in food intake to 4.4 $\pm$ 0.4 grams/ mouse/day, in comparison to the food intake of vehicle-treated controls (7.0 $\pm$ 0.3 grams/mouse/day), for the same 24-hour period. This Example demonstrates a substantially improved <u>in vivo</u> biological activity of glycosylated leptin as compared to non-glycosylated recombinant human leptin.

EXAMPLE 10

Improvement in Weight Loss Activity of Three-Site Glycosylated Leptin Administered Intermittently

This Example demonstrates that the improved <u>in vivo</u> biological activity of a three site glycosylated human leptin having a Stokes' radius greater than 30 Å is maintained when the material is administered on an intermittent basis. As can be seen, *ob/ob* mice lost significantly more weight with either daily or every-other-day administration of glycosylated leptin, than when treated with a 10-fold higher dose of non-glycosylated leptin.

<u>Methods:</u>

1. <u>Leptin</u>. The three-glycosylation site leptin, prepared as described below (sites 47, 69 and 102, SEQ. ID NO: 32), was used, formulated at a concentration of 1.9 mg/ml in Dulbecco's phosphate-buffered saline, pH 6.8. Recombinant methionyl human leptin 1-146 (SEQ. ID NO: 1 with a methionyl at position -1) was used as a basis for comparison, formulated at 20 mg/ml in 10 mM sodium acetate with 5% sorbitol, pH 4.0. Ten mM sodium acetate with 5% sorbitol, pH 4.0, was used as a vehicle control.

2. <u>Animals</u>. Animals were housed under temperature-, light-, and humidity-controlled conditions, with lights on at 0600 hours and lights off at 1800 hours. The Amgen, Inc. animal research facility is approved by the USDA and AAALAC-accredited. Six female *ob/ob* mice (Jackson Laboratories) were used per treatment group. Mice were 4.5 months old at the time of study, and weighed an average of 66.6 grams. Mice were randomized to treatment groups such that the mean body weights of the groups were equivalent prior to treatment initiation. Animals were housed two per cage and fed standard laboratory rodent chow pellets <u>ad libitum.</u>

3. <u>Administration</u>. All treatment procedures were approved by Amgen's Institutional Animal Care And Use Committee. Glycosylated leptin, r-metHu-Leptin, or placebo were administered either daily or every-other-day via subcutaneous injection in the mid-scapular region in a volume of 0.1 ml. The dose of leptin was 0.25 or 2.5 mg/kg body weight/day for mice injected daily with glycosylated leptin or r-metHu-Leptin. The dose of leptin was 1 or 10 mg/kg body weight/day for mice injected every-other-day with glycosylated leptin or r-metHu-Leptin. Injections were given on 7 consecutive days, beginning on study day 0, in the late afternoon (within 2 hours of lights off in the colony). Mice injected every other day with leptin received injections of vehicle on alternate days. Animals were weighed daily at the time of injection. Percent weight loss is calculated as: ((Body weight on day 7 - Body weight on day 0)/Body weight on day 0) multiplied by 100. All data are reported as the mean $\pm$ SE.

<u>Results:</u>

As shown in Table 10.1, mice injected daily with 0.25 mg/kg body weight/day glycosylated leptin lost more weight than did mice receiving either the same dose or a ten-times higher dose of recombinant methionyl human leptin.

Table 10.1: Weight loss (expressed as a % of initial body weight) after 7 days of daily dosing of Glycosylated Leptin or recombinant methionyl human leptin.

|  | Dose | |
|---|---|---|
| Injectate | 0.25 mg/kg/d | 2.5 mg/kg/d |
| r-metHu-Leptin | -8.2 $\pm$ 1.0% | -15.2 $\pm$ 0.5% |
| Three-Site Glycosylated Leptin | -21.4 $\pm$ 0.6% | not done |

As shown in Table 10.2, mice injected every-other-day with 1 mg/kg body weight/day glycosylated leptin lost more weight than did mice receiving either the same dose or a ten-fold higher dose of recombinant methionyl human leptin.

Table 10.2: Weight loss (expressed as a % of initial body weight) after 7 days of daily dosing of glycosylated leptin or recombinant methionyl human leptin.

|  | Dose | |
| --- | --- | --- |
| Injectate | 1.0 mg/kg/d | 10 mg/kg/d |
| r-metHu-Leptin | -5.7 $\pm$ 0.8% | -10.6 $\pm$ 0.8% |
| Three-Site Glycosylated Leptin | -16.9 $\pm$ 1.0% | not done |

This example demonstrates that the enhanced biological activity of glycosylated leptin, relative to non-glycosylated leptin, is preserved when the protein is administered intermittently to obese mice.

EXAMPLE 11

Dose Response Studies of a Three-Site Glycosylated Leptin on Wild Type Mice

This example demonstrates that the present three-site glycosylated leptin having a Stokes' radius greater than 30 Å has biological activity in non-obese mice. Moreover, the present example confirms in wild type mice, that a far lower dose of the glycosylated leptin results in substantial weight loss, compared to non-glycosylated leptin

1. Leptin. The three-glycosylation site leptin, prepared as described below (sites 47, 69 and 102, SEQ. ID NO: 32), was used, formulated at a concentration of 5.1 mg/ml in Dulbecco's phosphate-buffered saline, pH 6.8. Recombinant methionyl human leptin 1-146 (SEQ. ID NO: 1 with a methionyl at position -1) was used as a basis for comparison, formulated at 20 mg/ml in 10 mM sodium acetate with 5% sorbitol, pH 4.0. Ten mM sodium acetate with 5% sorbitol, pH 4.0, was used as a vehicle control.

2. Animals. Animals were housed under temperature-, light-, and humidity-controlled conditions, with lights on at 0600 hours and lights off at 1800 hours. The Amgen, Inc. animal research facility is approved by the USDA and AAALAC-accredited. Six female C57Bl/6J mice (Jackson Laboratories) were used per treatment group. Mice were 2.5 months old at the time of study, and weighed an average of 20.0 grams. Mice were randomized to treatment groups such that the mean body weights of the groups were equivalent prior to treatment initiation. Animals were housed two per cage and fed standard laboratory rodent chow pellets ad libitum.

3. Administration. All treatment procedures were approved by Amgen's Institutional Animal Care And Use Committee. Glycosylated leptin, r-metHu-Leptin, or placebo were administered either daily via subcutaneous injection in the mid-scapular region in a volume of 0.1 ml. The dose of leptin was 1 or 10 mg/kg body weight/day. Injections were given on 7 consecutive days, beginning on study day 0, in the late afternoon (within 2 hours of lights off in the colony. Animals were weighed daily at the time of injection. Percent weight loss is calculated as: ((Body weight on day 7 - Body weight on day 0)/Body weight on day 0) multiplied by 100. All data are reported as the mean $\pm$ SE.

Results:

As shown in Table 11.1, mice injected daily with 1 mg/kg body weight/day glycosylated leptin lost more weight than did mice receiving either the same dose or a ten-times higher dose of recombinant methionyl human leptin.

Table 11.1: Weight loss (expressed as a % of initial body weight) after 7 days of daily dosing of Glycosylated Leptin or recombinant methionyl human leptin.

|  | Dose | |
| --- | --- | --- |
| Injectate | 1 mg/kg/d | 10 mg/kg/d |
| r-metHu-Leptin | -2.2 $\pm$ 1.0% | -3.9 $\pm$ 0.6% |
| Three-Site Glycosylated Leptir | -8.6 $\pm$ 0.6% | not done |

This example demonstrates that the enhanced biological activity of glycosylated leptin, relative to non-glycosylated leptin, is also present in non-obese mice.

EXAMPLE 12

Improvement in Weight Loss Activity of Three-Site Glycosylated Leptin Administered Intermittently to Wild Type Mice

The present example demonstrates that the three-site glycosylated leptin having a Stokes' radius greater than 30 Å has a improved biological activity as compared to r-metHu-Leptin 1-146. Further, the example demonstrates that the improved biological activity is sustained when glycosylated leptin is administered on a less frequent dosing schedule than r-metHu-Leptin 1-146, in wild type mice.

1. <u>Leptin</u>. The three-glycosylation site leptin, prepared as described below (sites 47, 69 and 102, SEQ. ID NO: 32), was used, formulated at a concentration of 5.1 mg/ml in Dulbecco's phosphate-buffered saline, pH 6.8. Recombinant methionyl human leptin 1-146 (SEQ. ID NO: 1 with a methionyl at position -1) was used as a basis for comparison, formulated at 20 mg/ml in 10 mM sodium acetate with 5% sorbitol, pH 4.0. Ten mM sodium acetate with 5% sorbitol, pH 4.0, was used as a vehicle control.

2. <u>Animals</u>. Animals were housed under temperature-, light-, and humidity-controlled conditions, with lights on at 0600 hours and lights off at 1800 hours. The Amgen, Inc. animal research facility is approved by the USDA and AAALAC-accredited. Six female C57Bl/6J mice (Jackson Laboratories) were used per treatment group. Mice were 2.5 months old at the time of study, and weighed an average of 20.0 grams. Mice were randomized to treatment groups such that the mean body weights of the groups were equivalent prior to treatment initiation. Animals were housed two per cage and fed standard laboratory rodent chow pellets <u>ad</u> <u>libitum.</u>

3. <u>Administration</u>. All treatment procedures were approved by Amgen's Institutional Animal Care And Use Committee. Glycosylated leptin, r-metHu-Leptin, or placebo were administered either daily via subcutaneous injection in the mid-scapular region in a volume of 0.1 ml. The dose of leptin was 1, 5, or 10 mg/kg body weight, injected every-other-day. Leptins were injected every-other-day for 7 consecutive days, beginning on study day 0, in the late afternoon (within 2 hours of lights off in the colony). Mice received injections of vehicle on alternate days. Animals were weighed daily at the time of injection. Percent weight loss is calculated as: ((Body weight on day 7 - Body weight on day 0)/Body weight on day 0) multiplied by 100. All data are reported as the mean ± SE.

Results:

As shown in Table 12.1, mice injected every-other-day with 1, 5, or 10 mg/kg body weight glycosylated leptin lost more weight than did mice receiving the same of recombinant methionyl human leptin every-other-day.

Table 12.1: Weight loss (expressed as a % of initial body weight) after 7 days of every-other-day dosing of glycosylated leptin or recombinant methionyl human leptin.

|  | Dose | | |
|---|---|---|---|
| Injectate | 1 mg/kg | 5 mg/kg | 10 mg/kg |
| r-metHu-Leptin | 0.8 ± 1.4% | -1.0 ± 0.8% | -1.5 ± 0.9% |
| Three-Site Glycosylated Leptin | -1.2 ± 2.1% | -6.7 ± 1.2% | -9.1 ± 0.5% |

This example demonstrates that the enhanced biological activity of glycosylated leptin, relative to non-glycosylated leptin, is preserved when the protein is administered intermittently to non-obese mice.

EXAMPLE 13

Additional Multiple Glycosylation Site Leptin Proteins

Presented below in Table 13.1 are additional glycosylated human leptin proteins which were also prepared. The table columns present: (1) the position of the N-glycosylation (with respect to the numbering of SEQ. ID NO 1., rHu-Leptin 1-146)(unless otherwise noted); (2) the expression yield as compared to "wild type" ("WT", here, rHu-Leptin 1-146 as in SEQ. ID NO: 1); (3) the glycosylation species which were detected; (4) the receptor binding relative to "WT"; (5) the bioactivity relative to "WT". Methods used are described below. The term "ND" means not determined. (The specific sequences used herein for expression are more fully set forth in the Example below pertaining to expression of glycosylated leptin protein 47 + 69 + 102)

Table 13.1

| Summary of COS Leptin Three Site Glycosylation expression, binding, and glycosylation results | | | | |
|---|---|---|---|---|
| Position of N-glycosylation | Expression Rel. to WT | Glycosylation | Receptor binding Rel. to WT. | Bioactivit y Rel. to WT. |
| None | 1 | 0 | 1 | 1 |
| 2+69+92 | 1.07 | 1'-25,2'-45,3'-10 | 0.7 | 0.41 |
| 2+69+92RRR* | 0.36 | 1'-10,2'-25,3'-60 | ND | ND |
| 26+33+48 | 0.25 | 1'-30,2'-30,3'-20 | ND | ND |
| 26+35+48 | 0.88 | 1'-30,2'-30,3'-20 | 1.3 | ND |
| 26+33+115 | 0.17 | 1'-30,2'-30,3'-20 | ND | ND |
| 26+35+115 | 0.16 | 1'-30,2'-30,3'-20 | ND | ND |
| 27+35+115 | 0.25 | 1'-30,2'-30,3'-20 | 0.8 | ND |
| 29+33+115 | 0.83 | 1'-30,2'-30,3'-20 | 1.1 | ND |
| 33+48+115 | 0.27 | 1'-25,2'-25,3'-40 | 0.7 | ND |
| 35+48+115 | 0.47 | 1'-30,2'-30,3'-20 | 1 | ND |
| 47+69+100e | 2.66 | 1'-5,2'-80(50/O-link) | 0.66 | 1.3 |
| 47+69+102 | 1.8 | 1'-30,2'-50,3'-10 | 0.62 | 0.86 |
| 47+69+103 | 0.57 | 1'-20,2'-45,3'-5 | 0.44 | 0.11 |
| 48+69+101 | 2.2 | 1'-10,2'-60,3'-10 | 0.95 | 0.15 |
| 48+69+102 | 3.3 | 1'-20,2'-45,3'-20 | 1.04 | 0.46 |
| 48+69+103 | 2.8 | 1'-15,2'-60,3'-5 | 0.52 | 0.36 |
| 48+69+118 | 1.2 | 1'-20,2'-45,3'-5 | 0.09 | 1.6 |
| 69+102+118 | 1.1 | 1'-50,2'-30,3'-0 | 0.11 | 3.5 |
| 69+103+118 | 0.72 | 1'-50,2'-20,3'-0 | 0.55 | 1.5 |
| Abbreviations and notations are the same as those used above, see, e.g., Table 4.1.<br>* "RRR" denotes the use of three C-terminal arginine residues on the glycosylated leptin protein. | | | | |

Additionally, the following multiple site glycosylated leptin proteins have been made and tested as further described in Example 15, below.

2+23+47+69+92
2+47+69+92+102
23+47+69+92+102
2+47+69+92
2+47+69+102
23+47+69+102
23+47+69+92
Q-47+69+92+102 ("Q-" indicates that SEQ. ID NO: 2, rHu-Leptin 1-145, was used as the protein backbone to which glycosylation sites were added)
47+69+100e+102

In addition, the present invention also encompasses a glycosylated leptin protein having glycosylation sits at position 47, 69, 92, and 102.

EXAMPLE 14

Expression of a Three Site Glycosylated Leptin Protein Using a Variety of Signal Sequences and Other Sequences Affecting Glycosylation

This example illustrates the differences in glycosylation of a three-site glycosylated leptin protein having sites available for glycosylation located at positions 47, 69, and 102 of rHu-Leptin 1-146 (SEQ. ID NO: 1 having the noted glycosylation sites using the formula N-X-S/T, prepared as described herein), also as described above. Expression in two cell types, COS cells, and CHO cells, was generally according to methods in the Reference Examples, below. Glycosylation was determined according to the methods set forth in the Reference Examples, below. The degree of glycosylation was scored on a scale of 1 to 5, with 5 having the appearance of the maximum occupancy of glycosylation sites. The term "ND" means "not determined."

A variety of signal sequences were used. The amino acid sequences of these signal sequences are presented below in Table 14.1. The following terms were used to denote what signal sequences or other amino acid sequences used to express the present glycosylated leptin:

| |
|---|
| Leptin - the naturally occurring human leptin signal sequence |
| Leptin/TPA(L/T) - the first five n-terminal of human leptin followed by 15 human tPA signal amino acids ending with SP |
| TPA/Leptin(T/L) - the seven N-terminal amino acids from human tissue plasminogen activator followed by 16 amino acids of the human leptin signal sequence beginning at LCG |
| Leptin(SP)- the naturally occurring human leptin signal sequence, except having the last two c-terminal amino acid replaced with serine-proline |
| Leptin(SPS)- the naturally occurring human leptin signal sequence, except having the two c-terminal amino acids replaced with serine-proline-serine |
| Leptin(SNS)-the naturally occurring leptin signal sequence from human leptin, except having the two c-terminal amino acids replaced with serine-asparagine-serine |
| Leptin-pro- the naturally occurring human leptin signal sequence, plus an additional "pro" sequence at the c-terminus |
| Leptin-modified(LGDVMT)- the naturally occurring human leptin signal sequence, except with six c-terminal amino acids substituted with LGDVMT |
| Leptin + RRR @ c-term - the naturally occurring human leptin signal sequence, and additionally, at the c-terminus of the glycosylated leptin protein, three arginine residues |
| Leptin R81, R85(analog)- the naturally occurring human leptin signal sequence, and glycosylated leptin protein being the amino acid sequence of SEQ. ID NO: 1 with arginine at positions 81 and 85, and glycosylation sites at positions 47, 69 and 102 |
| Thrombopoietin (TPO)- the naturally occurring human thrombopoietin signal sequence |
| Tissue Plasminogen Activator (TPA)- the naturally occurring human tissue plasminogen activator signal sequence |
| TPA(SNS)- the naturally occurring human tissue plasminogen activator signal sequence having the three c-terminal amino acids being the amino acids serine-asparagine-serine |
| TPA(SPA)- the naturally occurring human tissue plasminogen activator signal sequence having the three c-terminal amino acids being the amino acids serine-proline-alanine |
| TPA(SP)- the naturally occurring human tissue plasminogen activator signal sequence having the two c-terminal amino acids being the amino acids serine-proline |
| TPA(SFS)- the naturally occurring human tissue plasminogen activator signal sequence having the three c-terminal amino acids being the amino acids serine-phenylalanine-serine |
| TPA(SWS)- the naturally occurring human tissue plasminogen activator signal sequence having the three c-terminal amino acids being the amino acids serine-tryptophan-serine |
| TPA(INS)- the naturally occurring human tissue plasminogen activator signal sequence having the three c-terminal amino acids being the amino acids isoleucine-asparagine-serine |

Table continued

| |
|---|
| TPA(INA)- the naturally occurring human tissue plasminogen activator signal sequence having the three c-terminal amino acids being the amino acids isoleucine-asparagine-alanine |
| TPA-A2- the naturally occurring human tissue plasminogen activator signal sequence having additional c-terminal amino acids of arginine-glycine-arginine-phenylalanine-arginine-arginine |
| TPA(SP)-A2 the naturally occurring human tissue plasminogen activator signal sequence having the last serine of the naturally occurring sequence eliminated and |
| having additional c-terminal amino acids of arginine-glycine-arginine-phenylalanine-arginine-arginine |
| TPA-A4- the naturally occurring human tissue plasminogen activator signal sequence additional c-terminal amino acids of glutamine-glutamic acid-isoleucine-arginine-glycine-arginine-phenylalanine-arginine-arginine |
| TPA-A5- the naturally occurring human tissue plasminogen activator signal sequence having additional c-terminal amino acids of glutamine-glutamic acid-isoleucine-histidine-alanine-arginine-phenylalanine-arginine-arginine |
| Intrinsic factor- the naturally occurring human intrinsic factor signal sequence |
| Serum albumin(pre-pro)- the naturally occurring human serum albumin signal sequence and prosequence |
| G-CSF- the naturally occurring human granulocyte colony stimulating factor signal sequence |
| von Willebrand's factor (vW)- the naturally occurring human von Willebrand's factor signal sequence |
| MAC-1 (CD11 alpha)- the naturally occurring human CD11α signal peptide |
| Tie (receptor)- the naturally occurring human Tie receptor signal sequence |
| Factor VIII - the naturally occurring human Factor VIII signal sequence |
| IgG-1, murine - the naturally occurring murine IgG-1 signal sequence |
| Follistatin (FS)- the naturally occurring human follistatin signal sequence |
| LAMP-1 - the naturally occurring human LAMP-1 signal peptide |
| Ceruloplasmin (CP)- the naturally occurring human ceruloplasmin signal peptide |
| EPO (or "ESP" denoting erythropoietin signal peptide)-the naturally occurring human erythropoietin signal sequence |
| EPO(ESP)RRR@c-term - the naturally occurring human erythropoietin signal sequence and also having, at the c-terminus of the amino acid sequence for the glycosylated leptin protein, three arginine residues |
| EPO-HSApro - the naturally occurring human erythropoietin signal sequence having at the c-terminus the "pro" sequence from human serum albumin |
| EPO-modifiedHSApro - the naturally occurring human erythropoietin signal sequence having at the c-terminus a modified "pro" sequence from human serum albumin (modified as indicated in the table below) |
| EPO-modifiedHSApro +furin - the naturally occurring human erythropoietin signal sequence having at the c-terminus a modified "pro" sequence from human serum albumin (modified as indicated in the table below) coexpressed with furin |
| EPO-NT3 pro- the naturally occurring human erythropoietin signal sequence having at the c-terminus a "pro" sequence from NT-3 |
| EPO-HSApro (leptin NH2 VtoA)- the naturally occurring human erythropoietin signal sequence having at the c-terminus a "pro" sequence from human serum albumin and with Val1 of the leptin sequence changed to Ala to improve cleavage of the prosequence by furin. |

Table 14.1 Expression of Leptin 47+69+102 Glycosylated Protein

| Signal | Sequence of leader peptide | Glycosylation 1-5, 5-Best | |
|---|---|---|---|
| | | COS | CHO |
| | | | |
| | | | |

EP 1 151 102 B1

Table continued

| Signal | Sequence of leader peptide | Glycosylation 1-5, 5-Best | |
|---|---|---|---|
| | | COS | CHO |
| Leptin | MHWGTLCGFLWLWPY LFYVQA | 1 | 1 |
| | | | |
| Leptin/TPA(L/T) | MHWGT/LCCVLLLCG AVFVSP | 4 | 5 |
| | | | |
| TPA/Leptin(T/L) | MDAMKRG/LCGFLWL WPYLFYVQA | 1 | 0.5 |
| | | | |
| Leptin(SP) | MHWGTLCGFLWLWPY LFYVSP | 4 | 3 |
| | | | |
| Leptin(SPS) | MHWGTLCGFLWLWPY LFYVSPS | 4.5 | 4.5 |
| | | | |
| Leptin(SNS) | MHWGTLCGFLWLWPY LFYVSNS | ND | ND |
| | | | |
| Leptin-pro | MHWGTLCGFLWLWPY LFYVQA-RGRFRR | 3 | 4.5 |
| | | | |
| Leptin-modified(LGDVMT) | MHWGTLCGFLWLWPY LGDVMT | 3 | 2 |
| | | | |
| Leptin + RRR @ c-term | MHWGTLCGFLWLWPY LFYVQA | 5 | 5 |
| | | | |
| Leptin R81, R85(analog) | MHWGTLCGFLWLWPY LFYVQA | 0.5 | ND |
| | | | |
| Thrombopoietin (TPO): | MELTELLLVV MLLLTARLTL S | 1 | 1 |
| | | | |
| Tissue Plasminogen Activator (TPA) | MDAMKRGLCCVLLLC GAVFVSPS | 4.5 | 4.5 |
| | | | |
| TPA(SNS): | MDAMKRGLCCVLLLC GAVFVSNS | 4 | 4 |
| | | | |
| TPA(SPA) | MDAMKRGLCCVLLLC GAVFVSPA | 4.5 | 4 |
| | | | |
| TPA(SP) | MDAMKRGLCCVLLLC GAVFVSP | 3 | 3 |
| | | | |
| TPA(SFS) | MDAMKRGLCCVLLLC GAVFVSFS | 3.5 | 4 |
| | | | |
| TPA(SWS) | MDAMKRGLCCVLLLC GAVFVSWS | 3.5 | 4 |
| | | | |
| TPA(INS) | MDAMKRGLCCVLLLC GAVFVINS | 3.5 | 4 |
| | | | |

41

Table continued

| Signal | Sequence of leader peptide | Glycosylation 1-5, 5-Best | |
|---|---|---|---|
| | | COS | CHO |
| TPA(INA) | MDAMKRGLCCVLLLC GAVFVINA | 3.5 | 3 |
| | | | |
| TPA-A2 | MDAMKRGLCCVLLLC GAVFVSPS-RGRFRR | 5 | 4.5 |
| | | | |
| TPA(SP)-A2 | MDAMKRGLCCVLLLC GAVFVSP-RGRFRR | 5 | 5 |
| | | | |
| TPA-A4 | MDAMKRGLCCVLLLC GAVFVSPS-QEIRGRFRR | 4.5 | 4 |
| | | | |
| TPA-A5 | MDAMKRGLCCVLLLC GAVFVSPS-QEIHARFRR | 4.5 | 4 |
| | | | |
| Intrinsic factor | MAWFALYLLS LLWATAGT | 3 | no expression |
| | | | |
| Serum albumin(pre-pro) | MKMVTFISLLFLFSS AYSRG-RGVFRR | 1 | ND |
| | | | |
| G-CSF | MAGPATQSPMKLMAL QLLLWHSALWTVQEA | 1 | 1 |
| | | | |
| von Willebrand's factor (vW) | MIPARFAGVLLALAL ILPGTLC | 1.5 | 3 |
| | | | |
| MAC-1 (CD11 alpha) | MALRVLLLTALTLCH G | 2 | 2 |
| | | | |
| Tie (receptor) | MVWRVPPFLLPILFL ASHVGA | 1 | 1 |
| | | | |
| Factor VIII | MQIELSTCFFLCLLR FCFS | 1 | 1 |
| | | | |
| IgG-1, murine | MKCSWVIFFLMAVVT GVDS | 1 | 3 |
| | | | |
| Follistatin (FS) | MVRARHQPGG LCLLLLLLCQ FMEDRSAQA | 1 | 1 |
| | | | |
| LAMP-1 | MAPRSARRPL LLLLPVAAAR PHALSSA | low expression | no expression |
| | | | |
| Ceruloplasmin (CP) | MKILILGIFL FLCSTPAWA | 1 | 3 |
| | | | |

Table continued

| Signal | Sequence of leader peptide | Glycosylation 1-5, 5-Best | |
| --- | --- | --- | --- |
| | | COS | CHO |
| EPO (ESP): | MGVHECPAWL WLLLSLLSLP LGLPVLG | 3 | 1.5 |
| | | | |
| EPO(ESP)RRR@c-term | MGVHECPAWL WLLLSLLSLP LGLPVLG | 5 | 5 |
| | | | |
| EPO-HSApro | MGVHECPAWLWLLLS LLSLPLGLPVLG-RGVFRR | 4 | 4.5 |
| | | | |
| EPO-modifiedHSApro | MGVHECPAWLWLLLS LLSLPLGLPVLG-RGRFRR | 4 | 4.5 |
| | | | |
| EPO-modifiedHSApro +furin | MGVHECPAWLWLLLS LLSLPLGLPVLG-RGRFRR | 4 | 4.5 |
| | | | |
| EPO-NT3 pro | MGVHECPAWLWLLLS LLSLPLGLPVLG-NRTSRRKR | 3.5 | 3 |
| | | | |
| EPO-HSApro (leptin NH2 VtoA) | MGVHECPAWLWLLLS LLSLPLGLPVLG-RGVFRR | 4 | 4 |

The various TPA signal peptides, particularly those with modified c-termini (such as the addition of a prosequence) appeared to have the greatest glycosylation efficiency in both CHO and COS cells.

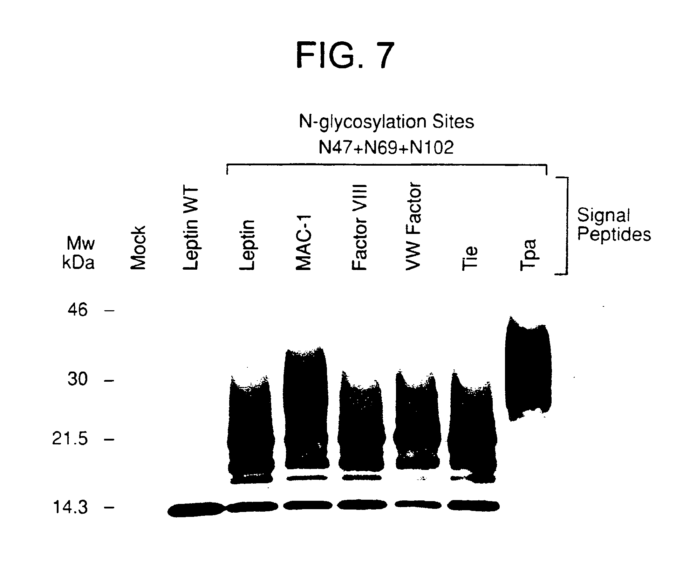

This was confirmed in a Western Blot (FIGURE 7). As can be seen, the use of the signal peptide for tPA resulted in the highest molecular weight, and therefore the most highly glycosylated, sample.

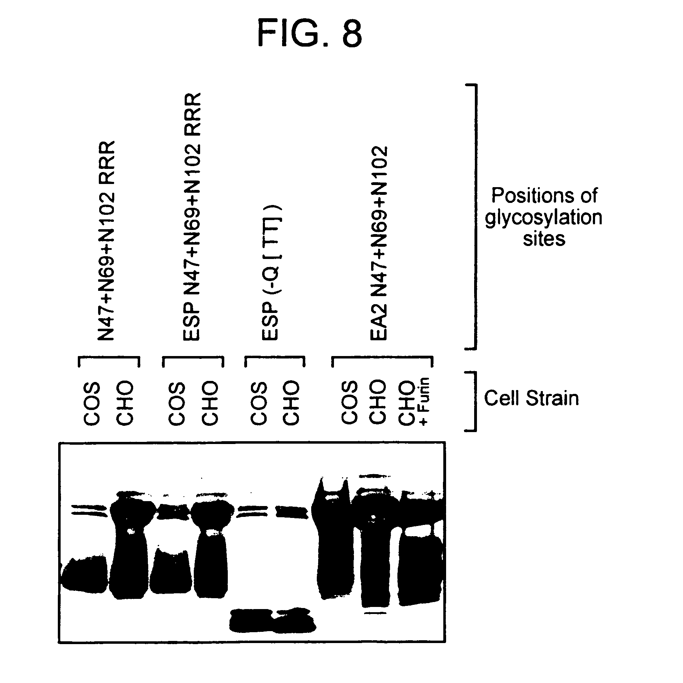

FIGURE 8 is a Western blot which shows the results of a comparison of the various expression conditions for the three-site glycosylated leptin protein 47 + 69 + 102 as above. Beginning at the left hand side of the Western Blot in the FIGURE, the lanes are loaded as follows:

Lane 1: molecular weight standards;

Lane 2: 47+69+102 having a c-terminal amino acid sequence of three arginines, expressed in COS cells using the native human leptin signal peptide;

Lane 3: Same as lane 2, expressed in CHO cells

Lane 4: Same as lane 2, expressed using a native human erythropoietin signal peptide;

Lane 5: "QTT COS ESP", rHu-Leptin 1-145 (SEQ. ID NO: 2) having the amino acid change 27T29S > 27T29T, expressed in COS cells using a native human erythropoietin signal peptide;

Lane 6: "QTT CHO ESP", same as lane 5, expressed in CHO cells using a native human erythropoietin signal peptide;

Lane 7: "EA2 47 + 69 + 102 COS", same as Lane 2, lacking the C-terminal arginines, expressed in COS cells, using the erythropoietin signal peptide and a modified human albumin prosequence, as indicated in this Example;

Lane 8: "EA2 47 + 69 + 102 CHO", same as lane 7, expressed in CHO cells;

Lane 9: "EA2 47 + 69 + 102 + Furin in CHO" same as lane 8, using the furin construct as indicated in this Example.

As can be seen by noting the density of high molecular weight bands (indicating glycosylation), the triple glycosylated leptin protein (lanes 2, 3, 4, 7, 8, an 9) has more glycosylation than the modified rHu-Leptin 1-145, having a single O-linked site (lanes 5 and 6). Use of CHO cells resulted in an increased amount of glycosylation as compared to COS cells (lane 2 versus lane 4, for example), and the use of the erythropoietin signal peptide appeared also to improve glycosylation (lanes 3 versus lane 5, for example).

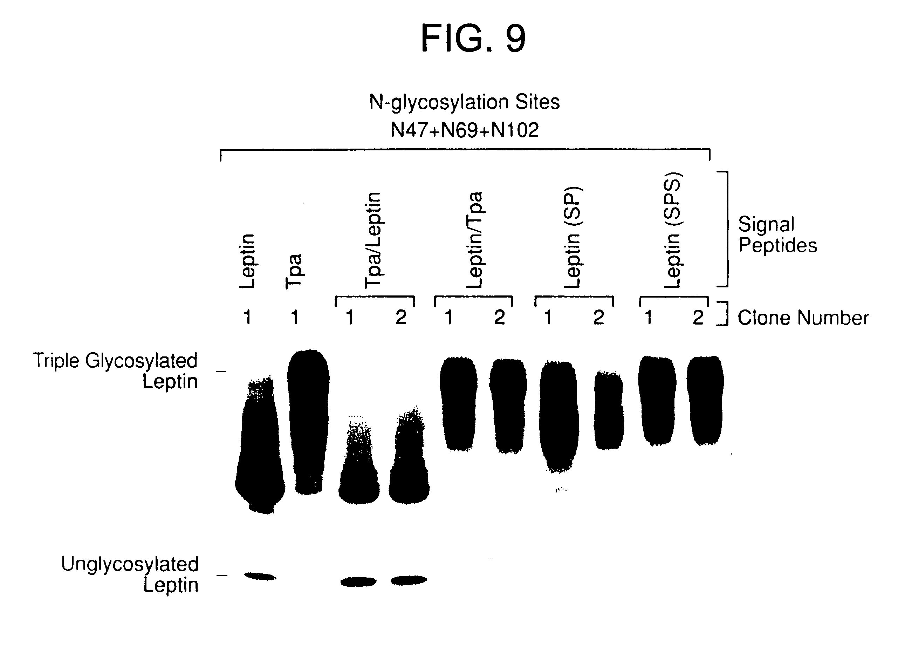

FIGURE 9 is a Western Blot comparing the use of either the leptin signal peptide or the tPA signal peptide, or use of either one with a substituted enzyme cleavage site of the other. Use of the tPA signal peptide resulted in greater glycosylation than use of the leptin signal peptide (left two lanes). When the c-terminal portion of the leptin signal peptide containing its peptidase cleavage site was used with the N-terminal portion of the tPA signal peptide, glycosylation efficiency decreased ("Tpa/Leptin" lanes). But when the C-terminal portion of the tPA signal peptide containing its cleavage site was used with the N-terminal portion of the leptin signal peptide, glycosylation efficiency increased ("Leptin /Tpa" lanes). Good glycosylation efficiency was found when only the tPA cleavage site was introduced into the leptin signal peptide("Leptin(SPS)"). It had greater glycosylation efficiency than substitution of a partial cleavage site sequence ("Leptin(SP)").

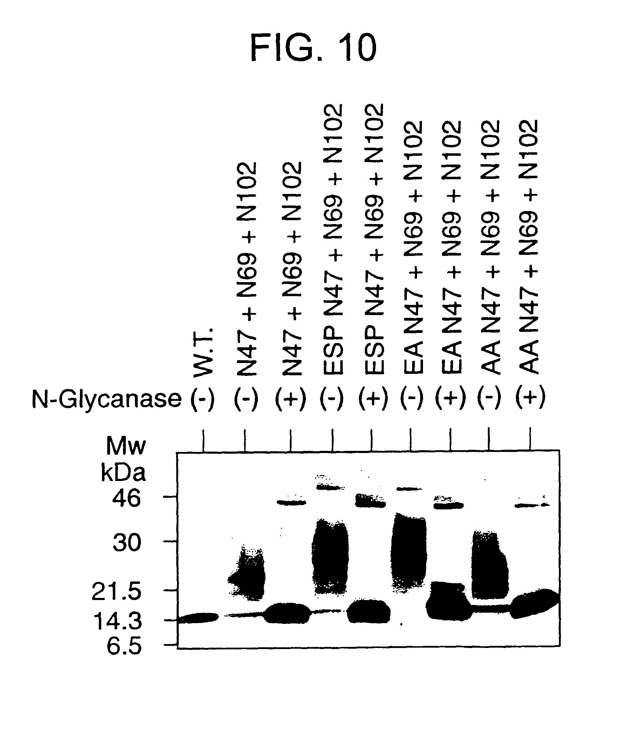

FIGURE 10 is a Western Blot comparing glycosylation efficiency by observation of results of removal of the carbohydrate moiety by N-glycanase. As can be seen, in the lanes which have carbohydrate removed (indicated by the "+"), the leptin protein migrates to the same molecular weight as non-glycosylated leptin. Comparing the apparent amount of carbohydrate in the lanes without N-glycanase ("-"), use of the erythropoietin signal peptide ("ESP" or "E" in combination with another notation, as used above) appears to more efficiently glycosylated the three site glycosylated leptin protein tested.

EXAMPLE 15

Additional Expression Studies of Multiple site Glycosylated Leptin Proteins Using a Variety of Signal Peptides and Other Sequences Affecting Glycosylation

This example presents additional data of expression of single and multiple glycosylation site leptin proteins, using a variety of signal peptides and other sequence. Unless otherwise indicated, the glycosylated leptin proteins below refer to glycosylation sites added to SEQ. ID NO: 1, using the preferred formula of N-X-S/T. Expression was in COS cells.

The percent("%") glycosylation means that percent of the molecules containing any carbohydrate. This was determined by visual examination of a Western Blot (as further described below in the Reference Examples)and determining subjectively that proportion of glycosylated protein of total leptin protein visualized.

Controls

Presented below in Table 15.1 are the data for various leptin proteins. The human leptin 1-145 (SEQ. ID NO: 2, herein denoted to be "Q-") was expressed as a glycosylated protein. When expressed in COS cells using its native glycosylation site, using a signal peptide from erythropoietin ("ESP" as in the Examples above), there was 25% glycosylation (indicating that 25% of the available sites for glycosylation were glycosylated in a population of expressed protein molecules). This was above the 10% glycosylation seen when the native human leptin signal peptide (as above) was used. When one of the glycosylation sites was changed to have a threonine at position 29 rather than a serine (as shown in the Table), the results were doubled, indicating that a "T" is better than an "S" for O-glycosylation. Both the "T" and the "S" of the natural O-linked site may each be glycosylated with a mixture of one and two carbohydrate chains. When the site is changed to have a "T" at position 29, the percent of these two sites having 1 and/or two chains is increased.

Table 15.1:

| Position of glycosylation | Sequence Change | Expression Rel. to WT | % Glycosylation | Receptor binding Rel. to WT. | Bio activity Rel. to WT. |
|---|---|---|---|---|---|
| WT | none | 1 | 0 | 1 | 1 |
| ESP Ob+ | EPO sp | 2 | 0 | 1 | 0.95 |
| (-Q) Ob+ | (-)Q @ 28 | 1.3 | 10 | 1.3 | 0.14 |
| ESP (-Q) Ob+ | EPO sp | 1.1 | 25 | 0.6 | 0.36 |
| ESP (-Q[TT])Ob+ | 27T29S > 27T29T | 0.72 | 60 | 0.71 | 0.3 |
| EA Ob+ | | 0.13 | 0 | 3.5 | 1.8 |
| "WT" denotes rHu-Leptin 1-146 (SEQ. ID NO: 1) | | | | | |

Table continued

| | |
|---|---|
| "ESP" denotes the native human erythropoietin signal peptide, as set forth in Example 14, above "ESP Ob+" denotes use of the above with the native human erythropoietin signal peptide |
| "(-Q)Ob+" denotes rHu-Leptin 1-145 (SEQ. ID NO:2) |
| "EA" denotes use of the native human erythropoietin signal peptide with the human albumin prosequence, as in Example 14, above. |

Expression of Single Site Glycosylated Leptin Proteins

Site 2 Comparisons

As can be seen from Table 15.2, below, the addition of three terminal arginines resulted in improved glycosylation efficiency.

Table 15.2

| Position of glycosylation | Expression Rel. to WT | % Glycosylation | Receptor binding Rel. to WT. | Bio-activity Rel. to WT. |
|---|---|---|---|---|
| ESP2 | 0.3 | 60 | 0.9 | 0.16, 0.012 |
| EA V>A2 | 0.45 | 35 | 1, 0.7 | 0.53, 0.73 |
| 2RRR | ND | 90 | ND | ND |

Where two assays were done, both results are presented. The abbreviations are the same as those for Example 14, above. "ND" as used in all Tables, means not determined.

Site 23 comparisons

As indicated in Table 15.3, the highest expression levels and the highest bioactivity occurred with use of the native leptin signal peptide (first row, 23a). Expression was in COS cells.

Table 15.3

| Position of glycosylation | Sequence Changes | Expression Rel. to WT | % Glycosylation | Receptor binding Rel. to WT. | Bio-activity Rel. to WT. |
|---|---|---|---|---|---|
| 23a | DIS > NIT | 7.8 | 50 | ND | 0.53 |
| ESP* 23a | EPO sp | 0.49 | 30 | 0.5 | 0.79 |
| ESP (-Q) 23 | EPO sp | 0.78 | 45 | 0.87 | <0.03 |
| ESP (-Q[TT]) 23 | EPO sp | 0.32 | 60 | ND | <0.004 |

The abbreviations are the same as those for Example 14, above. "ND" as used in all Tables, means not determined.

Site 47 Comparisons

As can be seen in Table 15.4, below, the addition of three terminal arginine residues ("RRR") resulted in additional glycosylation for the glycosylated leptin protein with a glycosylation site at position 47.

Table 15.4

| Position of glycosylation | Expression Rel. to WT | % Glycosylation | Receptor binding Rel. to WT. | Bio-activity Rel. to WT. |
|---|---|---|---|---|
| 47 | 1.06 | 80 | 0.66 | 0.84 |

Table continued

| Position of glycosylation | Expression Rel. to WT | % Glycosylation | Receptor binding Rel. to WT. | Bio-activity Rel. to WT. |
|---|---|---|---|---|
| 47RRR | 0.07 | 95 | ND | 0.86 |

Where two assays were done, both results are presented. The abbreviations are the same as those for Example 14,above. "ND" as used in all Tables, means not determined.

Site 48 Comparisons

As presented in Table 15.5, below, the highest level of expression for the position 48 single site glycosylated leptin protein using COS Cells was with the signal peptide from erythropoietin combined with the prosequence from human serum albumin. As can be seen, this single site protein had a biological activity higher than non-glycosylated leptin.

Table 15.5

| Position of N-glycosylation | Sequence Changes | Expression Rel. to WT | % Glycosylation | Receptor binding Rel. to WT. | Bio-activity Rel. to WT. |
|---|---|---|---|---|---|
| 48 | ILT > NLT | 0.92 | 50 | 0.8 | 0.53 |
| ESP 48 | EPO sp | 0.54 | 75 | 0.8 | <0.001 |
| EA 48 | EPO sp + HSA pro | 1.8 | 80 | 1 | 1.2 |
| AA 48 | HSA sp + HSA pro | 1 | 90 | 1 | 0.78 |

The abbreviations used are the same as those used for the above Examples. Percent glycosylation is expressed in the same terms as Table 15.1, above.

Site 69 Comparisons

As can be seen from Table 15.6, use of the tPA signal peptide plus three terminal arginines resulted in the highest glycosylation efficiency.

Table 15.6

| Position of N-glycosylation | Expression Rel. to WT | % Glycosylation | Receptor binding Rel. to WT. | Bio-activity Rel. to WT. |
|---|---|---|---|---|
| 69 | 0.8 | 75 | 0.6 | 1.1 |
| T 69 | ND | 85 | ND | ND |
| 69RRR | 0.07 | 65 | ND | ND |
| T 69RRR | 0.04 | 95 | ND | ND |

The abbreviations used are the same as those used for the above Examples. Percent glycosylation is expressed in the same terms as Table 15.1, above.

Site 92 Comparisons

As can be seen in Table 15.7, the addition of three terminal arginines improved glycosylation efficiency.

Table 15.7

| Position of N-glycosylation | Expression Rel. to WT | % Glycosylation | Receptor binding Rel. to WT. | Bio-activity Rel. to WT. |
|---|---|---|---|---|
| 92 | 4.8 | 45 | 1.6 | 0.8 |
| 92RRR | 0.03 | 95 | ND | ND |
| The abbreviations used are the same as those used for the above Examples. Percent glycosylation is expressed in the same terms as Table 15.1, above. | | | | |

Site 102 Comparisons

As can be seen in Table 15.8, the addition of three C-terminal arginine residues resulted in improved glycosylation efficiency.

Table 15.8

| Position of N-glycosylation | Expression Rel. to WT | % Glycosylation | Receptor binding Rel. to WT. | Bio-activity Rel. to WT. |
|---|---|---|---|---|
| 102 | 1.8 | 70 | 0.5 | 0.66 |
| 102RRR | 0.07 | 95 | ND | 0.33 |
| The abbreviations used are the same as those used for the above Examples. Percent glycosylation is expressed in the same terms as Table 15.1, above. | | | | |

Expression of Two Site Glycosylated Leptin Protein Site 47+69 Comparisons

As can be seen in Table 15.9, for site 47 + 69 leptin glycosylated protein (as described above), use of the native leptin signal peptide gave the highest expression levels. Use of the erythropoietin signal peptide with the human serum albumin prosequence, or use of the human serum albumin signal peptide and prosequence, gave higher bioactivity results.

Table 15.9

| Position of N-glycosylation | Sequence Changes | Expression Rel. to WT | Glycosylation | Receptor binding Rel. to WT. | Bio-activity Rel. to WT. |
|---|---|---|---|---|---|
| 47+69 | 47+69 | 1.3 | 1'-10,2'-50 | 1 | 0.69 |
| ESP 47+69 | EPO sp | 0.35 | 1'-10,2'-60 | 0.6 | <0.002 |
| EA 47+69 | EPO sp + HSA pro | 0.3 | 1'-5,2'-85 | 0.3 | 1.8 |
| AA 47+69 | HSA sp + HSA pro | 0.82 | 1'-20,2'-50 | 1 | 1.7 |
| Abbreviations are the same a those used above. Glycosylation is expressed in the same terms as used above, see, e.g., Table 4.1. | | | | | |

Site 69+102 Comparisons

As can be seen in Table 15.10, use of the erythropoietin signal peptide in COS Cells apparently had a detrimental effect on expression of the 69 + 102 two site glycosylated leptin (as described above).

Table 15.10

| Position of N-glycosylation | Sequence Changes | Expression Rel. to WT | % Glycosylation | Receptor binding Rel. to WT. | Bio-activity Rel. to WT. |
|---|---|---|---|---|---|
| 69+102 | 69+102 | 1.7 | 1'-40,2'-30 | 0.5 | 0.63 |

Table continued

| Position of N-glycosylation | Sequence Changes | Expression Rel. to WT | % Glycosylation | Receptor binding Rel. to WT. | Bio-activity Rel. to WT. |
|---|---|---|---|---|---|
| ESP 69+102 | EPOsp /69+102 | 0.6 | 1'-20,2'-50 | 1 | <0.001 |
| 47+102 | 47+102 | 2.7 | 1'-50.2'-25 | 1.08 | 0.42 |
| Abbreviations are the same as those used above, <u>see, e.g.,</u> Example 14 for more details. Glycosylation is expressed in the same terms as used above, <u>see, e.g.,</u> Table 4.1. | | | | | |

Expression of Three Site Glycosylated Leptin Protein

As can be seen in Table 15.11, the use of the erythropoietin signal peptide had varying effects on various glycosylated leptin proteins expressed in COS cells. Interestingly, the glycosylated leptin protein with the highest bioactivity had the highest receptor binding (the lower the number the higher the affinity for the receptor).

Table 15.11

| Position of glycosylation | Sequence Changes | Expression Rel. to WT | Glycosylation | Receptor binding Rel. to WT. | Bio-activity Rel. to WT. |
|---|---|---|---|---|---|
| ESP 2+47+69 | EPO sp | 0.2 | 1'-5,2'-70,3'-20 | 0.7 | 0.24 |
| L/T 2+47+69 | (seeEx14) | 0.21 | 1'-5,2'-60,3'-30 | 1.25 | ND |
| L(SNS) 2+47+69 | (seeEx14) | 0.31 | ND | 1.63 | ND |
| T 2+47+69 | (seeEx14) | 0.1 | 1'-5,2'-50,3'-40 | ND | ND |
| ESP 23+47+69 | EPO sp | 0.51 | 1'-5,2'-10,3'-45 | 0.5 | 1.4 |
| ESP 47+69+77 | EPO sp | 0.51 | 1'-10,2'-75,3'-5 | 4.2 | <0.006 |
| ESP 47+69+92 | EPO sp | 0.39 | 1'-15,2'-50,3'-15 | 0.71 | 0.47 |
| ESP (-Q) 47+69+92 | EPO sp | 0.76 | 1'-15,2'-50,3'-15 | 0.8 | 0.88 |
| Abbreviations are the same as those used above, <u>see, e.g.,</u> Example 14 for more details. Glycosylation is expressed in the same terms as used above, <u>see, e.g.,</u> Table 4.1. | | | | | |

Expression of Four Site Glycosylated Leptin Protein

As can be seen in Table 15.12, for quadruple site glycosylated leptin protein expression in COS cells, various expression levels were obtained, and the resultant glycosylated proteins had various degrees of receptor binding and bioactivity. For this group of quadruple site leptins, the 23 +47 + 69 + 92 and 2<u>3</u> + 47 + 69 + 102 site leptins had the highest bioactivity relative to wild type (<u>i.e.,</u> rmetHu-Leptin 1-146, SEQ. ID NO: 1).

Table 15.12

| Summary of COS Leptin Glycosylation Quadruple Site Expression, <u>B</u>inding, and Glycosylation Results | | | | |
|---|---|---|---|---|
| Position of glycosylation | Expression Rel. to WT | Glycosylation | Receptor binding Rel. to WT. | Bioactivity Rel. to WT. |
| None | 1 | 0 | 1 | 1 |
| ESP2+47+69+92RRR | 0.004 | 1'-5,2'-5,3'-30,4'-70 | ND | ND |
| 2+47+69+92RRR | 0.13 | 1'-5,2'-5,3'-60,4'-25 | ND | 0.2 |

Table continued

| Summary of COS Leptin Glycosylation Quadruple Site Expression, Binding, and Glycosylation Results | | | | |
|---|---|---|---|---|
| Position of glycosylation | Expression Rel. to WT | Glycosylation | Receptor binding Rel. to WT. | Bioactivity Rel. to WT. |
| T 2+47+69+92 | 0.052 | 1'-5,2'-5,3'-45,4'-40 | ND | ND |
| T 2+47+69+92RRR | 0.01 | 1'-52'-5,3'-35,4'-50 | ND | ND |
| T(SNS) 2+47+69+92 | 0.15 | 1'-5,2'-20,3'-45,4'-25 | 1.1 | <0.1 |
| T(-S)2+47+69+92 | 0.14 | 1'-5,2'-20,3'-45,4'-25 | 1.1 | <0.01 |
| EA2 2+47+69+92 | 0.24 | 1'-5,2'-10,3'-50,4'-30 | ND | ND |
| TA4 2+47+69+92 | 0.24 | 1'-10,2'-25,3'-15,4'-45 | 0.6 | 0.49 |
| TA5 2+47+69+92 | 0.13 | 1'-10,2'-25,3'-15,4'-45 | 0.8 | 0.88 |
| L/T 2+47+69+92 | 0.2 | 1'-5,2'-25,3'-45,4'-25 | 1.2 | ND |
| L(SNS) 2+47+69+92 | 0.28 | ND | 0.75 | ND |
| T 2+47+69+102 | 0.16 | ND | ND | ND |
| L(SNS) 2+47+69+102 | 0.28 | ND | 1.1 | ND |
| ESP 23+47+69+102 | 0.48 | 1'-20,2'-20,3'-20,4'-10 | 0.5 | 1.8 |
| ESP 23+47+69+92 | 0.41 | 1'-20,2'-20,3'-20,4'-5 | 0.5 | 2.3 |
| ESP(-Q) 47+69+92+102 | 0.32 | 1'-10,2'-40,3'-40 | 0.57 | 0.13 |
| 47+69+100e+102 | 1.5 | 1'-25.2'-40,3'-20 | 0.7 | 0.66 |
| Abbreviations are the same as those used above, see, e.g., Example 14 for more details. Glycosylation is expressed in the same terms as used above, see, e.g., Table 4.1. | | | | |

Expression of Five Site Glycosylated Leptin Protein

As presented in Table 15.13, the expression levels of various quintuple site glycosylated proteins was fairly low using the indicated signal peptides. Some data were not determined ("ND").

Table 15.13

| Summary of COS Leptin Glycosylation Quintuple Site Expression, Binding, and Glycosylation Results | | | | |
|---|---|---|---|---|
| Position of N-glycosylation | Expression Rel. to WT | Glycosylation | Receptor binding Rel. to WT. | Bioactivity Rel. to WT. |
| None | 1 | 0 | 1 | 1 |
| 2+23+47+69+92 RRR | 0.11 | 1'-5,2'-5,3'-20,4'-40,5'-25 | 1 | ND |
| T 2+23+47+69+92 | 0.045 | 2'-5,3'-20,4'-40,5'-25 | ND | ND |

Table continued

| Summary of COS Leptin Glycosylation Quintuple Site Expression, Binding, and Glycosylation Results | | | | |
|---|---|---|---|---|
| Position of N-glycosylation | Expression Rel. to WT | Glycosylation | Receptor binding Rel. to WT. | Bioactivity Rel. to WT. |
| T 2+23+47+69+92RRR | 0.01 | 2'-5,3'-10,4'-45,5'-30 | ND | ND |
| T 2+47+69+92+102 | 0.19 | 2'-20,3'-30,4'-30,5'-15 | .83 | ND |
| ESP 23+47+69+92+102 | 0.34 | 1'-20,2'-20,3'-20,4-20,5'-5 | 0.3 | 1.3 |
| L/T 2+47+69+92+102 | 0.29 | 2'-30,3'-30,4'-30,5'-5 | 0.75 | ND |
| L(SPS) 2+47+69+92+1 02 | 0.18 | 1'-5,2'-10,3'-20,4-30,5'-25 | 1.42 | ND |
| T(SNS) 2+47+69+92+1 02 | 0.16 | 2'-30.3'-30,4-30,5'-5 | 0.5 | ND |
| T(SPA) 2+47+69+92+1 02 | 0.19 | 2'-20,3'-30,4-30,5'-15 | 0.58 | ND |
| L(SNS) 2+47+69+92+1 02 | 0.21 | ND | 0.38 | ND |
| Abbreviations are the same as those used above, see, e.g., Example 14 for more details. Glycosylation is expressed in the same terms as used above, see, e.g., Table 4.1. | | | | |

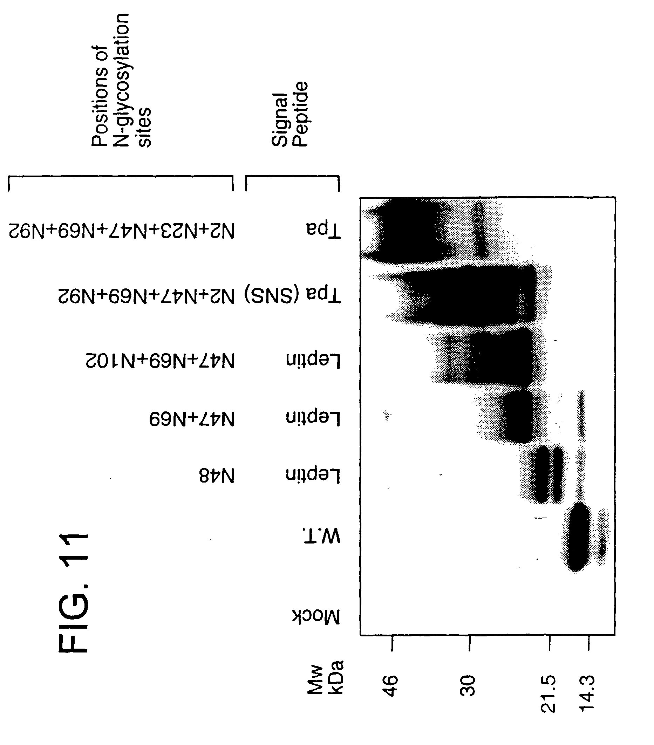

In addition, Western Blots of the present glycoslated leptin proteins also illustrates that differences in expression conditions and compositions results in different glycosylation efficiencies. FIGURE 11 shows that increasing the number of glycosylation sites, at least up to five sites, increases the amount of glycosylation found on the leptin protein when expressed in CHO cells. The samples are as follows:

Lane 0: "MOCK" is non-leptin containing cell culture supernatant;
Lane 1: "ESP(W.T.)", rHu-Leptin 1-146 (SEQ. ID NO: 1) expressed using an erythropoietin signal peptide;
Lane 2: "ESP (N48)", same as above, using the single site glycosylated leptin protein indicated (as described above);
Lane 3: "ESP (N47 + N69)", same as above, using the two-site glycosylated leptin protein indicated (as described above);
Lane 4: "ESP (N47 + N69 + N102)", same as above, using the three site glycosylated leptin protein indicated (as described above);
Lane 5: "Tpa(SNS) N2 + N46 + N69 + N92" indicates the four site glycosylated leptin protein as described above, expressed using a human tPA signal peptide having an enzyme cleavage site of SNS (see Example 14 for sequence information);
Lane 6: "Tpa N2 + N23 + N47 + N69 + N92" indicates the five site glycosylated leptin protein as described above, expressed using the natural human tPA signal peptide (see Example 14 for sequence information).

As can be seen, the molecular weight increases with the increase in glycosylation sites (compare lane 1 to lane 6). This indicates that the sites are adding carbohydrate and that up to five chains can be added to leptin simultaneously.

N-terminal amino acids/peptides

The below Table 15.14 presents a comparison of the different N-terminal amino acids of the subject glycosylated leptin protein incident to the use of various substituted enzyme cleavage sites for various signal peptides.

Table 15.14

| Signal Peptide | Glycosylation | Amino Terminus N-terminal extension |
|---|---|---|
| Leptin | + | Correct (V) |
| TPA | ++++ | S+ SPS |
| TPASP | ++ | 30% SP |
| **TPA(SNS)** | +++ | 96% |
| **Leptin(SPS)** | ++++ | S |
| Leptin(SP) | +++ | Not Determined |
| Leptin/tPA | +++ | S |

As can be seen, the most highly glycosylated leptins having the highest yield of correct N-terminal amino acid was produced using the signal peptide tPA(SNS). Leptin(SNS) also was highly glycosylated, and produced a leptin having an N-terminal amino acid of serine (e.g., serine at the -1 position of SEQ. ID NO: 1 in a glycosylated leptin with a modified amino acid sequence of SEQ. ID NO: 1).

REFERENCE EXAMPLES

The following reference examples provide methods which were used in the above Working Examples.

Preparation of DNAs, Vectors and Host Cells

1. Construction of the Human Leptin (1-146) expressing vector. These methods result in an expression vector for rHu-Leptin 1-146 (SEQ. ID NO: 1) in mammalian cells. The DNA encoding rHu-Leptin 1-146 including its signal peptide was also used as a template for preparing glycosylated leptin proteins of the present invention.

A DNA encoding rHu-Leptin amino acids 1-146 plus a signal sequence as in Zhang et al., Nature 372: 425-432 (1994) at 430, Figure 6b, herein incorporated by reference in its entirety, was cloned from human adipose cDNA by polymerase chain reaction (PCR). The signal sequence cloned encoded the following amino acid sequence:

```
M H W G T L C G F L   W L W P Y L F Y V Q   A
```

Primers. The 5' (forward) flanking primer encoded the amino terminus of the rHu-Leptin signal peptide, an XbaI restriction enzyme site, and an optimized Kozak sequence (TCT ATC TAG ACC ACC ATG CAT TGG GGA ACC CTG T). The 3' (reverse) flanking primer sequence (GAG AGT CGA CTA TCA GCA CCC AGG GCT GA) contained the complement of the carboxyl terminus of rHu-Leptin(1-146) and termination codons, as well as a SalI restriction site.

Vector Preparation. The PCR amplification product was digested with XbaI and SalI restriction enzymes, electrophoresed on an agarose gel, then isolated from the gel using the Promega® Wizard® kit procedure (Promega® Corporation, Madison, WI). The purified product was ligated to XbaI and SalI cut pDSRα2 expression vector modified slightly from that described in WO 90/14363 1990, e.g., at Figure 12, herein incorporated by reference in its entirety. The pDSRα2 used herein maintained the same functional elements, but was slightly modified from that set forth in WO 90/14363. The sequence at the Hind III site was modified to AAGCTTCTAGA to generate an XbaI site, and the sequence at the NcoI site was modified to GTCGACCTAGG to generate a SalI site, with sufficient DNA sequence ("stuffer DNA") in between the two sites to allow efficient cutting by both XbaI and SalI to produce the cut plasmid for directional cloning of the present leptin protein expression construct. The resulting pDSRα2/leptin plasmid was used for mammalian cell expression as below, and as a template for in vitro site directed mutagenesis.

2. Construction of Glycosylation Sites into Leptin by site-directed mutagenesis. Glycosylation sites were introduced into rHu-Leptin 1-146 (SEQ. ID NO: 1, above) sequence by site-directed mutagenesis using overlap extension PCR methods similar to those described by Ho et al., Gene Vol.77, pp. 51-59 (1989). The pDSRa2 Leptin plasmid, prepared as above, was utilized as a PCR template for the initial steps of site directed mutagenesis.

PCR. PCR procedures were performed in two successive steps.

Step 1: Two reactions (PCR1 and PCR2) were performed on leptin template DNA using a total of four oligonucleotides: the 5' (forward) flanking rHu-Leptin primer, a reverse mutagenic primer, a forward mutagenic primer (complementary at least in part to the reverse mutagenic primer) and the 3' (reverse) flanking rHu-Leptin primer. The mutagenic primers contained the desired nucleotide changes as well as 6-20 nucleotides exactly matching the template on each side of the changes. PCR1 used the 5' (forward) flanking primer and the reverse mutagenic primer. PCR2 used the 3' (reverse) flanking primer and the forward mutagenic primer. The DNA products of PCR1 and PCR2 contained overlapping sequences at and on both sides of the mutations. The amplified DNA fragments were separated by agarose gel electrophoresis. Small pieces of agarose containing DNA fragments of the correct size were excised from the gel.

Step 2: The DNA fragments from PCR1 and PCR2 were combined together and a third PCR reaction was performed using only the 5' forward and 3' reverse flanking primers. Annealing of the complementary 3'-terminal regions of the appropriate strands of the PCR1 and PCR2 products and subsequent strand elongation resulted in formation of full-length leptin DNA fragments. Thus, a full length DNA segment containing the desired mutations was amplified.

PCR for expression in COS and CHO cells. For expression in the human embryonic kidney cell line 293 (such as that commercially available from the American Type Culture Collection) the 5' (forward) primer contained sequences which introduced a stop codon, a KpnI site and a Kozak sequence (ACCACC) in front of the leptin signal peptide coding region (5'-TCTGGTACCTAGACCACCATGCATTGGGGAACCCTGT-3'). The 3' (reverse) primer (5'-GAAGCGGCCGCCTATCAGCACCCAGGGCTGA-3') contained sequences which introduced two stop codons (TGA TAG) and a NotI restriction site at the end of the glycosylated leptin protein coding region. For COS and CHO cell expression the 3' (reverse) primer contained sequences that introduced a stop codon followed by a SalI restriction site (GAGAGTCGACTATCAGCACCCAGGGCTGA). The 5' forward reaction primer (TCTATCTAGACCACCATGCATTGGGGAACCCTGT) had an XbaI restriction site followed by a Kozak sequence upstream of the leptin initiator codon (ATG).

PCR Methods. Polymerase chain reactions were performed using either of two procedures interchangeably.

In one method, used in some of the constructions for 293 expression, PCR reactions were performed using a protocol adapted from Cheng et. al., PNAS 91: 5695 (1994)(herein incorporated by reference in its entirety): 4 $\mu$l each of forward and reverse primers (5 pm/$\mu$l), 1 $\mu$l template (50 ng), 10 $\mu$l of 5X LP buffer (100 mM Tricine, pH 8.7/25% glycerol/425 mM KOAc), 2 $\mu$l dNTP stock (1 mM each of dATP, dTTP, dCTP, dGTP), 0.8 $\mu$l rtTh polymerase (Perkin Elmer®; 2.5 U/$\mu$l), and 2 $\mu$l Vent polymerase (NEB; 0.01 U/$\mu$l after 1:100 fresh dilution in 1X LP buffer). $H_2O$ was added to bring the final volume to 50 $\mu$l. All the components were added together in the order shown and the PCR was started when the temperature during the first cycle was above 60°C by adding 1 $\mu$l of 10 mM MgOAc. Reaction conditions were: 2 cycles of 94°C, 10 sec/45°C, 1 min/68°C, 5 min followed by 25 cycles of 94°C, 10 sec/55°C, 1 min/ 68°C, 5 min. The amplified fragments were separated by agarose gel electrophoresis and the correct sized DNA fragment was purified using a Geneclean™ kit and procedures supplied by the manufacturer (Bio 101, Inc.) herein incorporated by reference. The purified DNA was digested with NotI and KpnI, then it was purified again using the Geneclean™ kit. Digestion conditions were 20 Units KpnI in "M" buffer (22 $\mu$L final volume) (Boehringer Mannheim) followed by addition of 3 $\mu$l of "H" buffer, 20 units NotI in 52 $\mu$l final volume. The fragment was then ligated into plasmid pBCB cut with KpnI and NotI. Plasmid pBCB was derived from pRC/CMV (Invitrogen®, Carlsbad, California) by deletion of the region of pRC/CMV comprising the f1 origin, SV40 origin, neomycin resistance gene and SV40 polyadenylation site. Ligated DNA was precipitated with 2 volumes of ethanol in 0.3M NaOAc pH 5.2 in the presence of carrier tRNA and transformed into E. coli. Glycosylated leptin protein DNAs were initially screened by colony PCR to identify clones containing the correctly sized and type of DNA insert. With this procedure, cells containing plasmids were placed into PCR tubes in the presence of leptin forward and reverse primers. The mixture was then subjected to PCR using the reaction conditions described above. Plasmids from positive clones were then prepared and the glycosylated leptin protein insert was sequenced to confirm the presence of the desired glycosylation sites and to ensure that no additional amino acid changes were introduced.

A slightly different PCR strategy was used in the remainder of the constructions. PCR was performed using Taq DNA Polymerase (Boehringer Mannheim) or preferably, the proofreading DNA polymerase Pfu polymerase (Stratagene), which unlike Taq polymerase does not tend to add an extra untemplated nucleotide at the 3' terminus of the extended strands. In Taq polymerase PCRs the DNA template was combined with 2.5 $\mu$l 10X Taq PCR buffer, 2.5 $\mu$l 1mM dNTPs, 5 pmol of each primer, and water in a final volume of 25 $\mu$l. 0.5 units of Taq polymerase was added after the PCR mixture reached 94°C. PCR reactions were then carried out for 25 cycles at 94°C for 30 seconds, 60°C for 30 seconds, and 72°C for 30 seconds. In Pfu polymerase PCRs, the DNA template was combined with 5 ul 10xPfu buffer (Stratagene), 5 $\mu$l 1mM dNTPs, 10 pmol of each primer, and water in a final volume of 50 $\mu$l and 1.2 U Pfu polymerase were added. Four cycles of PCR with an annealing temperature of 48°C were performed followed by 20 cycles with an annealing temperature of 66°C. In each cycle denaturation was performed for 30 sec at 94°C, annealing was performed for 30 sec, and elongation was at 74°C for 30 sec. Following the two PCR reactions of the first step described earlier, product bands of the correct sizes were excised from an agarose gel following electrophoresis and the gel slices containing the products of PCR 1 and PCR 2 were either added directly to a PCR tube for the second step of PCR, or first combined in a tube with 300 $\mu$l of water and boiled to melt the agarose before adding to the PCR tube. PCRs were performed with Taq or Pfu polymerase as described earlier using the forward and reverse flanking primers. After the final cycle of PCR, the tubes were allowed to incubate an extra 5 minutes at the elongation temperature. The resulting PCR products for each analog were cleaned using the Promega® Wizard® PCR Cleanup kit. The purified DNA was digested in a 50 ul restriction digest with XbaI and SalI restriction enzymes (Boehringer Mannheim) at 37°C for 1 hour. The digests were cleaned by Promega® Wizard® Cleanup kit. The digested fragment was then ligated into Xbal and Sall digested pDSRa2 vector. A 1 μl aliquot of the ligation reaction, containing pDSRa2 Leptin analog plasmid, was used to transform DH10B cells by electroporation. A single colony for each analog was grown overnight in liquid culture and plasmid was isolated using the Qiagen® Maxi DNA® plasmid isolation kit. DNA for each pDSRa2 Human Leptin analog was resuspended in water and sequenced to ensure that the correct sequence was present.

Multiple glycosylation sites. Two or more glycosylation site mutations were combined by introducing a new substitution into DNA already containing a change, using the same PCR process. To construct double glycosylation site leptin analog genes, single site glycosylated leptin plasmids (produced as described above) were used as PCR templates, and an additional glycosylation site was introduced by site directed mutagenesis with the appropriate primers. Similarly, plasmids encoding leptin analogs with three glycosylation sites were constructed using double N-glycosylation site leptin DNAs as template, and the process could be iterated for introduction of further glycosylation sites. Alternatively, new combinations of glycosylation site mutations can be produced by using two different DNA templates, containing different glycosylation sites, in PCR1 and PCR2. For example, a DNA encoding an analog with glycosylation sites at positions 2, 47, 69 and 102 could be produced by the mutagenic primer pair for position 47 glycosylation, by using a DNA template with a glycosylation site at position 2 in PCR1 and a DNA with glycosylation sites at positions 69 and 102 in PCR2.

These general procedures were used to construct plasmids for expression of the glycosylated leptin proteins shown in the Examples above The DNA sequence changes for each of the forms are shown.

Chimeric signal peptides. Constructs for expression of leptin or a leptin analog with a non-leptin signal peptide were prepared by overlap extension PCR methods for gene splicing (Horton et al., Gene 77: 61-68 (1989)) similar to those used for site-directed mutagenesis. In a preliminary step, a DNA encoding the signal peptide of the exogenous gene, for example tissue plasminogen activator (TPA), was obtained by cloning methods or by a combination of chemical and enzymatic gene synthesis. This DNA was used as template in a PCR to generate a DNA fragment encoding the exogenous signal peptide preceded by a consensus Kozak sequence and immediately followed by the first part of the coding region of mature rHu-leptin (or leptin analog). The primers used in this PCR reaction were a 5' (forward) flanking primer for the exogenous gene and a reverse primer whose 5' portion (10-25 nucleotides) was complementary to the sequence encoding the amino terminal portion of mature leptin (or leptin analog). A second PCR reaction was performed with leptin or leptin analog DNA as template, using the leptin 3'(reverse) flanking primer and a forward primer encoding the region of the junction of the exogenous signal peptide and the mature leptin (or leptin analog) sequence. The forward primer in this reaction was designed to overlap the DNA generated by the first PCR, usually over a length of 15-35 nucleotides. The products of the two PCR reactions were gel purified as described earlier, mixed, and were annealed and amplified in a PCR with just the exogenous gene 5' (forward) flanking primer and the 3' (reverse) flanking leptin primer.

Host Cells

1. Expression of Glycosylated Leptin Proteins in 293 cells. DNA was transfected into the human embryonic kidney cell line, "293",(such as that commercially available from the American Type Culture Collection) using the lipofectamine method. 293 cells were grown to 40-70% confluency in tissue culture plates(P100) in 293 medium (DMEM (Difco®)/20mM HEPES/1X Pen-Strep-Glutamine/20% FBS). 20 μg of plasmid DNA encoding the glycosylated leptin protein in 1 ml of DMEM was filter sterilized with a 0.45 μm Acrodisc® membrane (Gelman Sciences). 100 μL of lipofectamine (Gibco®/BRL®) was added and the DNA-lipofectamine mix was incubated for 20 minutes at RT. Medium was removed from plates containing 293 cells and 4 mL of DMEM and the DNA/lipofectamine solution was added. After 4-6 hours at 37°C, 5 ml of DMEM with 20% fetal bovine serum was added and the cultures incubated overnight. The next day the cells were rinsed with 293 medium and 5 ml of fresh 293 medium was added. The conditioned medium was collected after 3 days, aliquoted and stored at -70°C.

2. Expression of glycosylated leptin proteins in COS cells. cDNA clones of glycosylated leptin proteins were transferred into COS-1 cells (ATCC No. CRL-1650) by electroporation. COS-1 cells were harvested from semi-confluent dishes, washed with medium (DMEM containing 10% fetal bovine serum and 1% L-glutamine/penicillin/ streptomycin (Irvine Scientific)) and resuspended at 6 x 106 cells/ml. One half ml of cells was transferred to a 0.2 cm electroporation cuvette (Bio-Rad®) and electroporated with a BTX Electroporation System Electrocell Manipulator 600® at 650 uF and 130 volts on the low voltage setting with 25 μg of plasmid DNA encoding the glycosylated leptin protein. The electroporated cells were plated on 100 mm tissue culture dish in 7 ml of medium. The conditioned medium was collected 3 days after electroporation, filtered using a 0.45 μm Acrodisc® membrane (Gelman Sciences)and stored at minus 80oC.

3. Expression of glycosylated leptin protein in CHO cells. Stable expression of rHu-Leptin 1-146 or glycosylated leptin protein was performed by transformation of dihydrofolate reductase deficient (DHFR⁻) Chinese Hamster Ovary (CHO) cells with pDSRα2 with the selected glycosylated leptin protein DNA as above followed by isolation and testing of individual clones. A 60 mm tissue culture dish was plated with 1x10⁶ CHO DHFR- cells grown in CHO D- medium (DMEM-high glucose, 10% fetal bovine serum, 1% penicillin/streptomycin/glutamine, 1% nonessential amino acids (Gibco®) and 1% HT supplement (Gibco®)) the day before transfection. A 10 μg DNA precipitate was then formed and added to the plates dropwise as per the Mammalian Cell Transfection Kit instructions (Specialty Media, incorporated herein by reference). After 24 hours in a tissue culture incubator, the medium was replaced with fresh CHO D- medium. Twenty four hours later the cells were split into six 100 mm culture dishes with CHO select medium (D-MEM high glucose, 10% dialyzed fetal bovine serum, 1% penicillin /streptomycin /glutamine, 1% nonessential amino acids (Gibco®). Medium was changed weekly until colonies appeared. After 10-14 days colonies were picked using 5 mm cloning discs (Labcore®) soaked in 1X trypine-EDTA (Life Technologies®) and cultured in 24 well tissue culture plates with CHO select medium. After 1-2 weeks glycosylated leptin protein expression was determined using a leptin EIA assay described below. The best expressing clones (i.e., those which demonstrated the most intense response using the EIA) were expanded and frozen in cryogenic storage.

In some circumstances a more rapid protocol was used to express analogs in CHO cells. In this case electroporation was used to transfect cells and individual clones were not isolated. Electroporation experiments used 200 $\mu$g of pDSR$\alpha$2 with the glycosylated leptin protein insert as described above, and 200 $\mu$g of herring sperm carrier DNA. The DNAs were phenolchloroform extracted and ethanol precipitated, then resuspended in 800 $\mu$l 1X HEBS along with 2 X 10$^7$ DHFR-Chinese Hamster Ovary (CHO) cells grown in CHO D-medium. The cells and DNA were incubated at room temperature for 10 minutes. Electroporation was carried out at 290 volts, and 960 ufarads using a BIO RAD Gene Pulser™ in 0.4 cm electroporation cuvettes. Cells were then incubated for 10 minutes at room temperature, washed with 10 ml CHO D- media, spun for 10 minutes at 1000 rpm in a Damon®/IEC Division IEC HN-SII Centrifuge, then resuspended in 20 ml CHO D-media and added to two 10 centimeter dishes. Cells were grown for 2 days at 37°C, then split 1:4 into CHO selection media and grown to ~70% confluency. Cells were then split 1:2 into selection media plus 6 nM methotrexate and grown at 37°C until clones were visible (approximately 2 weeks). Pools were generated from plates containing at least 4 colonies and were grown in selection media with 6 nM methotrexate until confluent (approximately 1 week). The pools were then frozen in cryogenic storage.

Expression and purification of N48 T50 Leptin (single glycosylation site leptin protein).

CHO cells were transformed with DNA expressing N48 T50 Leptin as described in above. Cells were expanded in spinner culture in growth medium (DMEM/F12 (1:1), 365 mg/l L-Glutamine, 1X MEM Non-Essential Amino Acids, 5% FBS). Roller bottles with breathable caps were then inoculated with 2e7 cells/bottle in 400 ml of growth medium and gassed for 10 seconds with 10% $CO_2$ in air. After 5 days, the bottles were shifted to serum free production medium (400 ml/bottle, DMEM/F12 (1:1), 365 mg/l (1X) L-Glutamine, 1X MEM Non-Essential Amino Acids, 10$\mu$M CUSO4, 1.5g/l additional glucose). Serum-free conditioned medium from three successive harvests were collected (180 Liters) and filtered through a 0.45 $\mu$m filter, concentrated about 30 fold, and diafiltered into 1 mM CHAPS, 10 mM Tris, pH 7.5 using a tangential-flow ultrafiltration system (Amicon®) with a 10,000 molecular weight cutoff membrane. The diafiltered media was stored at -20°C.

The following steps were performed at 2 to 8°C. The DFM was applied to a Q-Sepharose Fast Flow column (Pharmacia®, 8 cm x 14 cm) equilibrated in 10 mM Tris, pH 7.9 and washed with about two column volumes of 10 mM Tris to elute all non-binding species. N48 T50 leptin, which remains bound to the column, was then eluted by a twelve column volume gradient from 10mM Tris, pH 7.9 to 200 mM NaCl, 10mM Tris, pH 7.9 collected into fractions. Fractions containing fully glycosylated N48 T50 leptin, as determined by Western blot analysis, were combined then diluted with one volume of water to reduce the sodium chloride concentration. The sample was then applied to a Bio-Gel® HT column (Bio-Rad®, 10 cm x 7 cm) equilibrated in 10mM Tris, pH 7.9 then washed with about four column volumes of 10mM Tris, pH 7.9. Fractions of the non-binding species were collected and those containing N48 T50 leptin, as determined by Western blot analysis, were combined.

A one third volume of 3 M $(NH_4)_2SO_4$, 10mM Tris, pH 7.9 was added to the N48 T50 leptin pool from the Bio-Gel® HT column. The pool, now in 1 M $(NH_4)_2SO_4$, was applied to a Source 15PHE column (Pharmacia®, 10 cm x 1.6 cm) equilibrated in 1 M $(NH_4)_2SO_4$, 10mM Tris, pH 7.9 then washed with about two column volumes of 1 M $(NH_4)_2SO_4$, 10 mM Tris, pH 7.9. F3, which remains bound to the column, was then eluted by a 40 column volume gradient from 1 M $(NH_4)_2SO_4$, 10mM Tris, pH 7.9 to 10mM Tris, pH 7.9 collected into fractions. Fractions containing F3, as determined by SDS-PAGE analysis, were combined.

Solid ammonium sulfate was added to the N48 T50 leptin pool from the Source 15PHE column to a final concentration of about 2.5 M and incubated overnight. The overnight precipitate was harvested by centrifugation.

The harvested ammonium sulfate precipitate was resolubilized in water, titrated to pH 4.5 with acetic acid, applied to a Source 15S column (Pharmacia®, 5.5 cm x 1.6 cm) equilibrated in 10mM NaCH$_2$COOH, pH 4.5, then washed with about two column volumes 10mM NaCH$_2$COOH, pH 4.5. N48 T50 leptin which remains bound to the column was then eluted by a 72 column volume gradient from 50 mM NaCl, 10mM NaCH$_2$COOH, pH 4.5 to 150 mM NaCl, 10mM NaCH$_2$COOH, pH 4.5 collected into fractions. Fractions containing N48 T50 leptin, as determined by SDS-PAGE analysis, were combined and titrated to pH 7.5.

The N48 T50 leptin pool from the Source 15S column was concentrated to about 1 mg/ml, diafiltered into Dulbecco's PBS (Gibco®), then concentrated to about 5 mg/ml using a stirred cell ultrafiltration system (Amicon®) with a 10,000 molecular weight cutoff membrane (Filtron®). The N48 T50 leptin was further concentrated to 10 mg/ml using centrifugal ultrafiltration (Centricon 10, Amicon®). The concentrated N48 T50 leptin was filtered (0.22 $\mu$m) and stored at 2 to 8°C.

II. Analytical Methods

The following analytical methods were used herein to characterize the present glycosylated leptin proteins.

A. In vitro Assays

1. Receptor Binding Assay. In this assay, membrane-bound leptin receptor was used as a target to measure the amount of binding by radioactively labeled test glycosylated leptins.

Chinese Hamster Ovary ("CHO") cells were engineered to stably express a human leptin receptor by transfecting them with human leptin receptor DNA (short form; Tartaglia et al., Cell 83: 1271 et seq.(1995) herein incorporated by reference in its entirety; the entire article is herein incorporated by reference). Leptin receptor expressing cells were grown and collected by low speed centrifugation. The pelleted cells (approximately 50 mg wet weight) were resuspended in 0.32 M sucrose/25 mM HEPES and homogenized in glass homogenizing tubes using a Glas-col® motor. The cell membranes were washed two times by centrifugation (48,000 x g), dispersion using a polytron homogenizer(Tissue Tearor®), and resuspension in cold binding buffer(MEM,Gibco BRL®/ 25 mM HEPES, Gibco BRL®/0.1 % BSA/ 0.5 mg/ml Bacitracin®(Sigma®)/ 0.1 mg/mL STI, Boehringer Mannheim/ 0.1 mg/mL AEBSF, Boehringer Mannheim). After the second wash, the membrane preparation was resuspended at a final concentration of 2-3 mg wet weight/mL in cold binding buffer.

Competition binding was performed by incubating 400 uL membrane solution, 50 uL of 2 nM 125I-Leptin (Amersham) and 50 uL sample or Leptin standard(10-6 M rHu-Leptin 1-146) for 2-3 hours at room temperature in 12mm x 75mm tubes. Bound 125I-Leptin was separated from unbound 125I-Leptin by filtration through glass fiber filters and 3 washes with cold PBS using a Brandel cell harvester. Bound radioactivity was determined with a gamma counter. The affinity of each analog for the leptin receptor was determined by calculation of the midpoint of the cold displacement curve (IC50) for each analog.

2. In vitro biological activity. In this assay, in vitro biological activity was determined using a chimeric leptin receptor, having an extracellular domain of a leptin receptor, a transmembrane and intracellular domains of an erythropoietin receptor. Upon activation of the intracellular erythropoietin receptor domain by binding to the extracellular leptin domain, the cells exhibited a biological activity of proliferation, measured by $H^3$-thymidine uptake.

Interleukin-3 (IL-3) dependent 32D (clone3) murine myeloid progenitor cells (Greenberger et al., PNAS-USA 80: 2931 (1983) herein incorporated by reference) were grown in RPMI 1640 (Gibco®) supplemented with 10% fetal bovine serum and 10 ng/mL IL-3 (Biosource®). A chimeric leptin receptor-EPO receptor (OBR-EPOR) was constructed by standard techniques and subcloned into an expression vector containing the transcriptional promoter of Moloney murine sarcoma virus resulting in the vector OBR-EPOR/pLJ. The chimeric receptor contained the coding regions for the extracellular domain of a human leptin receptor (amino acids 1-839; Tartaglia et al., Cell: 83: 1271 (1996) (herein incorporated by reference in its entirety) and transmembrane and intracellular domains of murine erythropoietin receptor (amino acids 250-507; D'Andrea et al., 57: 277 (1989) herein incorporated by reference in its entirety. The chimeric receptor was then transfected into 32D cells by electroporation. Transfected cells were initially selected on G418 (750 ug/ml). Leptin responsive cells were then selected in RPMI 1640 (Gibco®) supplemented with 10% fetal bovine serum and 25 ng/ml Hu Leptin, resulting in 32D-OBECA cells. 32D cells which were not transfected with the chimeric receptor remained unresponsive to leptin.

32D-OBECA cells were grown in 1640 RPMI medium (1x liquid, without L-Glutamine, Gibco®) containing 10% fetal bovine serum (Hyclone Laboratories®) and 1.0 ng/ml of recombinant murine IL-3 (Biosource®) to a density of approximately 5.0E+05 cells/ml. Cells were collected by centrifugation (approximately 270 X G), washed twice in sterile 1X PBS (Gibco®) and then resuspended to 1.0E+05 cells/ml in a media consisting of 20% DMEM Medium (DMEM+10%FBS) plus 80% assay medium (RPMI + 2% FBS) plus 10ng/mL pan-specific anti-TGFβ neutralizing antibody. An extended twelve point rmetHu-Leptin 1-146 standard curve was prepared using assay medium at a range of approximately 0.1 to 200ng/ml. Test samples were diluted in assay medium and typically run as extended multiple point curves or at ranges falling within the linear range of the standard curve. A volume of 100$\mu$l of each sample was added to appropriate wells of 96 well microtiter tissue culture plates. Cells with sample or standard were grown at 10,000 cells per well (in 100$\mu$l) for approximately 48 hours at $5\pm1\%$ $CO_2$ and $37\pm2°C$ high humidity incubator. 3H-Thymidine(0.5 $\mu$Ci per well, Dupont®) was then added and the plates were incubated for an additional 18 hours and their DNA harvested onto preprinted glass fiber filtermats (Pharmacia®) using a cell harvester (Tomtek 96 Mach II®). Filters were dried in a microwave oven, bagged in LBK® sample bags plus 10ml of scintillation fluid (LKB®), then counted in a Betaplate® scintillation counter (LKB®). Cell response (in the form of average CPMs-background) was plotted vs. mass (ng/well) of a rHu-Leptin 1-146 standard. The bioactivity of a sample of rmetHu-Leptin or glycosylated leptin was determined from regression analysis of the standard curve. Specific activity was calculated by dividing the assayed biological activity (ng/ml) by the concentration as determined by leptin ELISA.

B. Characterization of glycosylated leptin proteins

1. Enzyme Immuno Assay ("EIA").

Polyclonal antibodies. Anti-rmetHu-Leptin 1-146 (SEQ. ID NO: 1 with a methionyl residue at the -1 position) polyclonal antibodies were raised in New Zealand white rabbits by repeated subcutaneous injections of rmetHu-Leptin 1-146 conjugated to keyhole limpet hemocyanin (KLH), and mixed with the adjuvant Titermax$^{tm}$, or with Freunds complete adjuvant (primary injection)and Freunds incomplete adjuvant (subsequent injections). The resulting rabbit sera were tested for reactivity with rmetHu-Leptin 1-146, and sera from those rabbits with the highest titer were pooled and affinity purified over Actigel-ALD Superflow® (Sterogene #2701-S-01) coupled to rmetHu-Leptin. An aliquot of the purified polyclonal antibody was coupled to horseradish peroxidase (HRP, Sigma® P-8415) to be used as a detecting antibody in the sandwich enzyme immunoassay (EIA) or Western.

Monoclonal antibodies. Anti-rmetHu-Leptin 1-146 monoclonal antibodies were developed from Lou rats that were injected multiple times with KLH conjugated rmetHu-Leptin 1-146, mixed with Freunds complete adjuvant (primary injection) or with Freunds incomplete adjuvant (subsequent injections). Rat sera were tested for reactivity with rmetHu-Leptin 1-146, and spleen cells from those with the highest titers were fused to rat myeloma line Y3Ag 1.2.3 by standard hybridoma techniques. The hybrid cells were plated in 96-well plates, allowed to form colonies, assayed for anti-rmetHu-Leptin 1-146 activity, and single-cell cloned. Monoclonal antibody from a rat hybridoma was used as one component of the leptin sandwich EIA.

Sandwich Assay. Microtiter plates (96-well standard [Immulon®] or half-well [Costar®]) were coated with 75μl or 30μl, respectively, of either polyclonal (1.5 μg/ml) or monoclonal (2.0 μg/ml) antibody in carbonate/bicarbonate buffer (NaHCO$_3$ 0.029M, Na$_2$CO$_3$ 0.015M, pH 9.6). The plates were blocked (1% bovine serum albumin [BSA], 5% sucrose) and samples, diluted appropriately in 2% BSA in phosphate buffered saline (PBS) were added, in duplicate, to the wells. To each microtiter plate was also added, in triplicate, a set of r-metHu-Leptin standards, or glycosylated leptin standards, covering the range of 90 to 4580 pg/ml. The plates were incubated at 4°C for 18 hours, aspirated and washed three times with wash buffer (Tris 50mM, NaCl 0.15M, EDTA 10mM, Tween® 20 0.05%, pH 7.35 [TEN]) and HRP-conjugated polyclonal anti-rmetHu-Leptin 1-146 was added, -70 ng/ml in 2% BSA in PBS with 0.05% Tween® 20. The plates were incubated at room temperature for 3 hours, washed five times with TEN and color developed with TMB substrate (tetramethyl benzidine) according to the manufacturer's instructions (Kirkegaard and Perry #50-76-00, Gaithersburg, MD 20879). Absorbance was measured at 450nm in a microplate reader. Leptin concentrations were calculated from a standard curve constructed for each plate, after subtraction of background color. Assay sensitivity was approximately 90 pg/ml; the inter- and intra-assay variations, calculated from controls included on each microplate, were 7.5% and 5.4%, respectively.

2. Carbohydrate Analysis by Western Blotting.

Generally, the larger the molecular weight _of a glycosylated leptin protein of the present invention, the more heavily glycosylated. Thus the present Western-blot type analysis was used to determine the amount of carbohydrate present on the expressed glycosylated leptin proteins.

A volume of supernatant containing approximately 400-600 pg of glycosylated leptin protein from COS or CHO cells transfected with glycosylated leptin protein cDNAs as described above was mixed with SDS-PAGE 3X sample buffer (0.1875 M Tris-HCl pH 6.8, 6% SDS, 30% glycerol, 15% 2-mercaptoethanol). The samples were analyzed by 14% acrylamide Tris-Glycine SDS-polyacrylamide gel electrophoresis (Novex®) and transferred to 0.45 μm nitrocellulose membrane(Novex®). The nitrocellulose membrane was rinsed, blocked with TBST (Tris 20mM, NaCl 137mM, Tween® 20 0.08%) containing 10% FBS and 2% BSA and incubated with HRP-conjugated polyclonal anti-rmetHu-Leptin 1-146 as prepared above (140 ng/ml, in TBST containing 5% FBS and 1% BSA) for 3-5 hours. After washing with TBST, five times, five minutes each, the membrane was developed with ECL reagent (Amersham®), according to the manufacturer's directions. The membrane was exposed to X-Omat® AR film (Kodak®) for ten to sixty seconds, and developed as for standard x-ray film. Specific protein bands were visualized, and sizes estimated from their positions relative to the molecular weight markers. The larger the size, the more carbohydrate moiety connected to the protein.

Treatment with N-glycanase. N-glycanase treatment removes N-linked carbohydrate resulting in an increase in mobility that is equal to that of unglycosylated leptin. Treatment of glycosylated leptin proteins with N-glycanase resulted in a molecular weight similar to unglycosylated leptin. This confirms that the increased size of glycosylated leptin protein is due to N-linked carbohydrate.

<u>Methods</u>. COS cell conditioned medium containing 400 pg of glycosylated leptin protein (1-3ul) was mixed with 10 μl 0.5% SDS and each sample was boiled for 3 minutes. Then 10.5μl of a 0.5M NaP04 pH 8.6 + 7.5% nonidet P40 was added with 3 μl of 250 unit/ml N-glycanase (Genzyme®). Each sample was incubated overnight at 37ºC and the reaction was stopped by the addition of SDS-PAGE sample buffer and subjected to SDS-PAGE Western analysis as described above. These results indicate that the reduced mobility on SDS PAGE observed is due to addition of N-linked carbohydrate. The fact that numerous glycosylated leptin proteins were identified indicates that there are multiple positions in leptin that can support N-linked carbohydrate addition. Similar results were obtained when the analogs were expressed in 293 cells. Similarly, when multiple-glycosylation site leptin proteins were treated with N-glycanase, their mobility also changed to that of unglycosylated leptin indicating that the mobility differences are due to the presence of N-linked carbohydrate.

While the invention has been described in what is considered to be its preferred embodiments, it is not to be limited to the disclosed embodiments, but on the contrary, is intended.to cover various modifications and equivalents included within the appended claims, which scope is to be accorded the broadest interpretation so as to encompass all such modifications and equivalents.

**Claims**

1. A glycosylated leptin protein comprising SEQ. ID NO: 1 having one or more sequence alterations as a site of glycosylation selected from among (where "T/S" denotes threonine or serine):

    (a) 01V->N 02P->X (where X is any amino acid except proline) 03I->T/S
    (b) 02P->N 03I 04Q->T/S
    (c) 23D->N 24I 25S->T/S
    (d) 47P->N 48I 49L->T/S
    (e) 48I->N 49L 50T/S
    (f) 69P->N 70S 71R->T/S
    (g) 92F->N 93S 94K->T/S
    (h) 101A->N 102S 103G->T/S
    (i) 102S->N 103G 104L->T/S
    (j) 103G->N 104L 105E->T/S

2. A glycosylated leptin protein comprising amino acids 1-146 of SEQ. ID NO: 1, having two glycosylation sites, said two sites selected from among (with respect to the numbering of SEQ. ID NO: 1):

    47 + 69;
    48 + 69;
    69 + 101;
    69 + 102;
    69 + 103;
    69 + 118; and,
    100 + 102.

3. A glycosylated leptin protein comprising amino acids 1-146 of SEQ. ID NO: 1, having three glycosylation sites, said three sites selected from among (with respect to the numbering of SEQ. ID NO: 1):

    2 + 47 + 69
    23 + 47 + 69;
    47 + 69 + 100;
    47 + 69 + 102;
    48 + 69 + 118;
    69 + 102 + 118; and,
    69 + 103 + 118.

4. A glycosylated leptin protein comprising amino acids 1-146 of SEQ. ID NO: 1, having four glycosylation sites said four sites selected from among (with respect to the numbering of SEQ. ID NO: 1):

    2 + 47 + 69 + 92;
    2 + 47 + 69 + 102;
    23 + 47 + 69 + 92;

23 + 47 + 69 + 102; and,
47 + 69 + 100 + 102.

**5.** A glycosylated leptin protein comprising amino acids 1-146 of SEQ. ID NO: 1, having five glycosylation sites, said five sites selected from among (with respect to the numbering of SEQ. ID NO: 1):

2 + 23 + 47 + 69 + 92
2 + 47 + 69 + 92 + 102
23 + 47 + 69 + 92 + 102.

**6.** Glycosylated leptin 2, 47, 69, according to claim 3, comprising the amino acid sequence (SEQ. ID NO: 26):

```
  1   VNITKVQDDT KTLIKTIVTR INDISHTQSV SSKQKVTGLD FIPGLHNITT
 51   LSKMDQTLAV YQQILTSMNS TNVIQISNDL ENLRDLLHVL AFSKSCHLPW
101   ASGLETLDSL GGVLEASGYS TEVVALSRLQ GSLQDMLWQL DLSPGC
```

**7.** Glycosylated leptin 2,47,69,92, according to claim 4, comprising the amino acid sequence (SEQ. ID NO: 28)

```
  1   VNITKVQDDT KTLIKTIVTR INDISHTQSV SSKQKVTGLD FIPGLHNITT
 51   LSKMDQTLAV YQQILTSMNS TNVIQISNDL ENLRDLLHVL ANSTSCHLPW
101   ASGLETLDSL GGVLEASGYS TEVVALSRLQ GSLQDMLWQL DLSPGC
```

**8.** Glycosylated leptin 2, 47, 69, 102, according to claim 4 comprising the amino acid sequence(SEQ. ID NO: 30):

```
  1   VNITKVQDDT KTLIKTIVTR INDISHTQSV SSKQKVTGLD FIPGLHNITT
 51   LSKMDQTLAV YQQILTSMNS TNVIQISNDL ENLRDLLHVL AFSKSCHLPW
101   ANGTETLDSL GGVLEASGYS TEVVALSRLQ GSLQDMLWQL DLSPGC
```

**9.** Glycosylated leptin 47,69,102, according to claim 3 comprising the amino acid sequence (SEQ. ID NO: 32)

```
  1   VPIQKVQDDT KTLIKTIVTR INDISHTQSV SSKQKVTGLD FIPGLHNITT
 51   LSKMDQTLAV YQQILTSMNS TNVIQISNDL ENLRDLLHVL AFSKSCHLPW
101   ANGTETLDSL GGVLEASGYS TEVVALSRLQ GSLQDMLWQL DLSPGC
```

**10.** Glycosylated leptin 2, 47, 69, 92, 102, according to claim 5, comprising the amino acid sequence (SEQ. ID NO: 34):

```
  1   VNITKVQDDT KTLIKTIVTR INDISHTQSV SSKQKVTGLD FIPGLHNITT
 51   LSKMDQTLAV YQQILTSMNS TNVIQISNDL ENLRDLLHVL ANSTSCHLPW
101   ANGTETLDSL GGVLEASGYS TEVVALSRLQ GSLQDMLWQL DLSPGC
```

**11.** Glycosylated leptin 47, 69, 92, 102, according to claim 4, comprising the amino acid sequence (SEQ. ID NO: 36):

```
  1   VPIQKVQDDT KTLIKTIVTR INDISHTQSV SSKQKVTGLD FIPGLHNITT

 51   LSKMDQTLAV YQQILTSMNS TNVIQISNDL ENLRDLLHVL ANSTSCHLPW

101   ANGTETLDSL GGVLEASGYS TEVVALSRLQ GSLQDMLWQL DLSPGC
```

**12.** A glycosylated leptin protein according to claim 1 having an N-terminal residue sequence selected from among:

a serine, arginine, proline or alanine residue at the -1 position,
a serine at the -1 position and a proline at the -2 position,
a serine-proline-serine sequence at the -1, -2, and -3 positions,
a serine at the -1 position and an arginine at the -2 position,
a serine at the -1 position, an arginine at the -2 position and a serine at the -3 position,
an arginine at the -1 position and a serine at the -2 position; and,
an alanine at the -1 position and proline at the -2 positions.

**13.** A nucleic acid encoding a glycosylated leptin protein according to claim 1.

**14.** A nucleic acid encoding glycosylated leptin 2,47,69 comprising the nucleic acid sequence (SEQ. ID NO: 25):

```
  1   GTGAACATCA CAAAAGTCCA AGATGACACC AAAACCCTCA TCAAGACAAT

 51   TGTCACCAGG ATCAATGACA TTTCACACAC GCAGTCAGTC TCCTCCAAAC

101   AGAAAGTCAC CGGTTTGGAC TTCATTCCTG GGCTCCACAA CATCACGACC

151   TTATCCAAGA TGGACCAGAC ACTGGCAGTC TACCAACAGA TCCTCACCAG

201   TATGAATTCC ACAAACGTGA TCCAAATATC CAACGACCTG GAGAACCTCC

251   GGGATCTTCT TCACGTGCTG GCCTTCTCTA AGAGCTGCCA CTTGCCCTGG

301   GCCAGTGGCC TGGAGACCTT GGACAGCCTG GGGGGTGTCC TGGAAGCTTC

351   AGGCTACTCC ACAGAGGTGG TGGCCCTGAG CAGGCTGCAG GGGTCTCTGC

401   AGGACATGCT GTGGCAGCTG GACCTAAGCC CTGGGTGC
```

**15.** A nucleic acid encoding glycosylated leptin 2,47,69,92 comprising the nucleic acid sequence (SEQ. ID NO: 27):

```
  1   GTGAACATCA CAAAAGTCCA AGATGACACC AAAACCCTCA TCAAGACAAT

 51   TGTCACCAGG ATCAATGACA TTTCACACAC GCAGTCAGTC TCCTCCAAAC

101   AGAAAGTCAC CGGTTTGGAC TTCATTCCTG GGCTCCACAA CATCACGACC

151   TTATCCAAGA TGGACCAGAC ACTGGCAGTC TACCAACAGA TCCTCACCAG

201   TATGAATTCC ACAAACGTGA TCCAAATATC CAACGACCTG GAGAACCTCC

251   GGGATCTTCT TCACGTGCTG GCCAACTCTA CCAGCTGCCA CTTGCCCTGG

301   GCCAGTGGCC TGGAGACCTT GGACAGCCTG GGGGGTGTCC TGGAAGCTTC

351   AGGCTACTCC ACAGAGGTGG TGGCCCTGAG CAGGCTGCAG GGGTCTCTGC

401   AGGACATGCT GTGGCAGCTG GACCTCAGCC CTGGGTGC
```

**16.** A nucleic acid encoding glycosylated leptin 2, 47, 69, 102 comprising the nucleic acid sequence (SEQ. ID NO: 29):

```
  1  GTGAACATCA CAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCTTCTCTA AGAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

**17.** A nucleic acid encoding glycosylated leptin 47,69,102 comprising the nucleic acid sequence (SEQ. ID NO: 31):

```
  1  GTGCCCATCC AAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCTTCTCTA AGAGCTGCCA CTTGCCETGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

**18.** A nucleic acid encoding glycosylated leptin 2,47,69,92,102 comprising the nucleic acid sequence (SEQ. ID NO: 33):

```
  1  GTGAACATCA CAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG

201  TATGAATTCC ACAAACGTGA TCCAAATATC CAACGACCTG GAGAACCTCC
251  GGGATCTTCT TCACGTGCTG GCCAACTCTA CCAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

**19.** A nucleic acid encoding glycosylated leptin 47, 69, 92, 102 comprising the nucleic acid sequence (SEQ. ID NO: 35);

```
  1  GTGCCCATCC AAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCAACTCTA CCAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

**20.** A vector containing a nucleic acid encoding a glycosylated leptin protein according to any of claims 13-19.

**21.** A vector according to claim 20 which is an expression vector.

**22.** A host cell containing a nucleic acid encoding a glycosylated leptin protein according to claim 1.

**23.** A host cell of claim 22 selected from among prokaryotic and eukaryotic cells.

**24.** A prokaryotic host cell of claim 23 which is a bacterial cell.

**25.** A eukaryotic host cell of claim 23 which is selected from among mammalian cells, yeast cells, and insect cells.

**26.** A mammalian host cell according to claim 25 selected from among human cells, monkey cells, BHK cells and CHO cells.

**27.** A method of preparing a protein according to claim 1 comprised of:

(a) culturing a cell containing a nucleic acid encoding said glycosylated leptin protein under suitable conditions for expression; and,
(b) obtaining said protein.

**28.** A pharmaceutical composition for parenteral injection, intravenous injection, subcutaneous injection, intrathecal administration, nasal administration, pulmonary administration, and osmotic pump administration comprising a glycosylated leptin protein according to any of claims 1-12 in a pharmaceutically acceptable carrier.

**29.** A glycosylated human leptin according to claim 1 for use in a method of treatment of a human for a condition selected from among obesity, diabetes, and high.blood lipid content effects; said method comprising administering an effective amount of said leptin.

**30.** A leptin according to claim 29 wherein said effective amount of said glycosylated human leptin is administered by gene therapy.

**31.** A selective binding molecule which is selective for a glycosylated leptin protein according to claim 1, which molecule is selected from among a polyclonal antibody, a monoclonal antibody, and a recombinant antibody.

**32.** A selective binding molecule according to claim 31, wherein the molecule is a monoclonal antibody.

**33.** A method of manufacturing a glycosylated leptin protein according to claim 27 comprising:

(a)culturing, under suitable conditions for expression, a host cell containing a DNA sequence encoding, in the 5' to 3' direction (i) a signal peptide, and (ii) a DNA encoding a glycosylated leptin protein according to claim 1; and
(b) obtaining said glycosylated leptin protein.

**34.** A method of claim 33 wherein said signal peptide is selected from among:

a) (SEQ. ID NO: 3) (native human leptin signal peptide) MHWGTLCGFLWLWPYLFYVQA
(b) (SEQ. ID NO: 4) (modified human leptin signal peptide) MHWGTLCGFLWLWPYLFYVSPS
(c) (SEQ. ID NO: 5) (modified human leptin signal peptide) MHWGTLCGFLWLWPYLFYVSP
(d) (SEQ. ID NO: 6) (modified human leptin signal peptide) MHWGTLCGFLWLWPYLFYVSPA
(e) (SEQ. ID NO: 7) (modified human leptin signal peptide) MHWGTLCGFLWLWPYLFYVSNS
(f) (SEQ. ID NO: 8) (native human tPA signal peptide) MDAMKRGLCCVLLLCGAVFVSPS
(g) (SEQ. ID NO: 9) (native human tPA signal peptide) MDAMKRGLCCVLLLCGAVFVSP
(h) (SEQ. ID NO: 10) (modified tPA signal peptide) MDAMKRGLCCVLLLCGAVFVSNS
(i) (SEQ. ID NO: 11) (modified tPA signal peptide) MDAMKRGLCCVLLLCGAVFVSPA
(j) (SEQ. ID NO: 12) (Leptin/tPA signal peptide) MHWGTLCCVLLLCGAVFVSPS
(k) (SEQ. ID NO: 13) (Leptin/tPA signal peptide) MHWGTLCCVLLLCGAVFVSP

**35.** A method of claim 33 wherein said signal peptide is selected from among the signal peptide for:

erythropoietin, Factor VIII, beta-interferon, serum albumin, insulin, von Willebrand's factor, CD11$\alpha$, IgG, follistatin, intrinsic factor, G-CSF, ceruloplasmin, and LAMP-1.

**Patentansprüche**

**1.** Glykosyliertes Leptin-Protein, umfassend SEQ ID NR. 1, das eine oder mehrere Sequenzveränderungen als eine Stelle der Glykosylierung aufweist, ausgewählt aus (wobei "T/S" Threonin oder Serin angibt):

(a) 01V → N 02P → X (worin X jede Aminosäure außer Prolin ist) 03I → T/S
(b) 02P → N 03I 04Q → T/S
(c) 23D → N 24I 25S → T/S
(d) 47P → N 48I 49L → T/S
(e) 48I → N 49L 50T/S
(f) 69P → N 70S 71R → T/S
(g) 92F → N 93S 94K → T/S
(h) 101A → N 102S 103G → T/S
(i) 102S → N 103G 104L → T/S
(j) 103G → N 104L 105E → T/S.

**2.** Glykosyliertes Leptin-Protein, umfassend Aminosäuren 1-146 von SEQ ID NR. 1, das zwei Glykosylierungsstellen aufweist, wobei die zwei Stellen ausgewählt sind aus (mit Bezug auf die Numerierung von SEQ ID NR. 1):

47 + 69;
48 + 69;
69 + 101;
69 + 102;
69 + 103;
69 + 118; und
100 + 102.

**3.** Glykosyliertes Leptin-Protein, umfassend Aminosäuren 1-146 von SEQ ID NR. 1, das drei Glykosylierungsstellen aufweist, wobei die drei Stellen ausgewählt sind aus (im Hinblick auf die Numerierung von SEQ ID NR. 1):

2 + 47 + 69
23 + 47 + 69;
47 + 69 + 100;
47 + 69 + 102;
48 + 69 + 118;
69 + 102 + 118; und
69 + 103 + 118.

**4.** Glykosyliertes Leptin-Protein, umfassen Aminosäuren 1-146 von SEQ ID NR. 1, das vier Glykosylierungsstellen aufweist, wobei die vier Stellen ausgewählt sind aus (im Hinblick auf die Numerierung von SEQ ID NR. 1):

2 + 47 + 69 + 92;
2 + 47 + 69 + 102;
23 + 47 + 69 + 92;
23 + 47 + 69 + 102; und
47+69+100+102.

**5.** Glykosyliertes Leptin-Protein, umfassen Aminosäuren 1-146 von SEQ ID NR. 1, das fünf Glykosylierungsstellen aufweist, wobei die fünf Stellen ausgewählt sind, aus (im Hinblick auf die Numerierung von SEQ ID NR. 1):

2 + 23 + 47 + 69 + 92
2 + 47 + 69 + 92 + 102
23 + 47 + 69 + 92 + 102.

**6.** Glykosyliertes Leptin 2, 47, 69 nach Anspruch 3, umfassend die Aminosäuresequenz (SEQ ID NR. 26):

```
  1  VNITKVQDDT KTLIKTIVTR INDISHTQSV SSKQKVTGLD FIPGLHNITT
 51  LSKMDQTLAV YQQILTSMNS TNVIQISNDL ENLRDLLHVL AFSKSCHLPX
101  ASGLETLDSL GGVLEASGYS TEVVALSRLQ GSLQDMLWQL DLSPGC
```

**7.** Glykosyliertes Leptin 2, 47, 69, 92 nach Anspruch 4, umfassend die Aminosäuresequenz (SEQ ID NR. 28):

```
  1  VNITKVQDDT KTLIKTIVTR INDISHTQSV SSKQKVTGLD FIPGLHNITT
 51  LSKMDQTLAV YQQILTSMNS TNVIQISNDL ENLRDLLHVL ANSTSCHLPX
101  ASGLETLDSL GGVLEASGYS TEVVALSRLQ GSLQDMLWQL DLSPGC
```

**8.** Glykosyliertes Leptin 2, 47, 69, 102 nach Anspruch 4, umfassen die Aminosäuresequenz (SEQ ID NR. 30):

```
  1  VNITKVQDDT KTLIKTIVTR INDISHTQSV SSKQKVTGLD FIPGLHNITT
 51  LSKMDQTLAV YQQILTSMNS TNVIQISNDL ENLRDLLHVL AFSKSCHLPX
101  ANGTETLDSL GGVLEASGYS TEVVALSRLQ GSLQDMLWQL DLSPGC
```

**9.** Glykosyliertes Leptin 47, 69, 102 nach Anspruch 3, umfassen die Aminosäuresequenz (SEQ ID NR. 32):

```
  1  VPIQKVQDDT KTLIKTIVTR INDISHTQSV SSKQKVTGLD FIPGLHNITT
 51  LSKMDQTLAV YQQILTSMNS TNVIQISNDL ENLRDLLHVL AFSKSCHLPW
101  ANGTETLDSL GGVLEASGYS TEVVALSRLQ GSLQDMLWQL DLSPGC
```

**10.** Glykosyliertes Leptin 2, 47, 69, 92, 102 nach Anspruch 5, umfassend die Aminosäuresequenz (SEQ ID NR. 34):

```
  1   VNITKVQDDT KTLIKTIVTR INDISHTQSV SSKQKVTGLD FIPGLHNITT

 51   LSKMDQTLAV YQQILTSMNS TNVIQISNDL ENLRDLLHVL ANSTSCHLPW

101   ANGTETLDSL GGVLEASGYS TEVVALSRLQ GSLQDMLWQL DLSPGC
```

**11.** Glykosyliertes Leptin 47, 69, 92, 102 nach Anspruch 4, umfassend die Aminosäuresequenz (SEQ ID NR. 36):

```
  1   VPIQKVQDDT KTLIKTIVTR INDISHTQSV SSKQKVTGLD FIPGLHNITT

 51   LSKMDQTLAV YQQILTSMNS TNVIQISNDL ENLRDLLHVL ANSTSCHLPW

101   ANGTETLDSL GGVLEASGYS TEVVALSRLQ GSLQDMLWQL DLSPGC
```

**12.** Glykosyliertes Leptin-Protein nach Anspruch 1, das eine N-termiale Reste-Sequenz ausgewählt aus:

einem Serin-, Arginin-, Prolin- oder Alaninrest an der -1 Position,
einem Serin an der -1 Position und einem Prolin an der -2 Position,
einer Serin-Prolin-Serin Sequenz an den -1, -2 und -3 Positionen,
einem Serin an der -1 Position und einem Arginin an der -2 Position,
einem Serin an der -1 Position, ein Arginin an der -2 Position und ein Serin an der-3 Position,
ein Arginin an der -1 Position und einem Serin an der -2 Position; und
einem Alanin an der -1 Position und Prolin an der -2 Position aufweist.

**13.** Nukleinsäure, kodierend für ein glykosyliertes Leptin-Protein nach Anspruch 1.

**14.** Nukleinsäure, die für ein glykosyliertes Leptin 2, 47, 69 kodiert, umfassend die Nukleinsäuresequenz (SEQ ID NR. 25):

```
  1   GTGAACATCA CAAAAGTCCA AGATGACACC AAAACCCTCA TCAAGACAAT

 51   TGTCACCAGG ATCAATGACA TTTCACACAC GCAGTCAGTC TCCTCCAAAC

101   AGAAAGTCAC CGGTTTGGAC TTCATTCCTG GGCTCCACAA TATCACGACC

151   TTATCCAAGA TGGACCAGAC ACTGGCAGTC TACCAACAGA TCCTCACCAG

201   TATGAATTCC ACAAACGTGA TCCAAATATC CAACGACCTG GAGAACCTCC

251   GGGATCTTCT TCACGTGCTG GCCTTCTCTA AGAGCTGCCA CTTGCCCTGG

301   GCCAGTGGCC TGGAGACCTT GGACAGCCTG GGGGGTGTCC TGGAAGCTTC

351   AGGCTACTCC ACAGAGGTGG TGGCCCTGAG CAGGCTGCAG GGGTCTCTGC

401   AGGACATGCT GTGGCAGCTG GACCTAAGCC CTGGGTGC
```

**15.** Nukleinsäure, die für ein glykosyliertes Leptin 2, 47, 69, 92 kodiert, umfassend die Nukleinsäuresequenz (SEQ ID NR. 27):

```
  1  GTGAACATCA CAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCAACTCTA CCAGCTGCCA CTTGCCCTGG
301  GCCAGTGGCC TGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

**16.** Nukleinsäure, die für ein glykosyliertes Leptin 2, 47, 69, 102 kodiert, umfassend die Nukleinsäuresequenz (SEQ ID NR. 29):

```
  1  GTGAACATCA CAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCTTCTCTA AGAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

**17.** Nukleinsäure, die für ein glykosyliertes Leptin 47, 69, 102 kodiert, umfassend die Nukleinsäuresequenz (SEQ ID NR. 31):

```
  1  GTGCCCATCC AAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCTTCTCTA AGAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

18. Nukleinsäure, die für ein glykosyliertes Leptin 2, 47, 69, 92, 102 kodiert, umfassend die Nukleinsäuresequenz (SEQ ID NR. 33):

```
  1  GTGAACATCA CAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG

201  TATGAATTCC ACAAACGTGA TCCAAATATC CAACGACCTG GAGAACCTCC
251  GGGATCTTCT TCACGTGCTG GCCAACTCTA CCAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

19. Nukleinsäure, die für ein glykosyliertes Leptin 47, 69, 92, 102 kodiert, umfassend die Nukleinsäuresequenz (SEQ ID NR. 35):

```
  1  GTGCCCATCC AAAAAGTCCA AGATGACACC AAAACCCTCA TCAAGACAAT
 51  TGTCACCAGG ATCAATGACA TTTCACACAC GCAGTCAGTC TCCTCCAAAC
101  AGAAAGTCAC CGGTTTGGAC TTCATTCCTG GGCTCCACAA CATCACGACC
151  TTATCCAAGA TGGACCAGAC ACTGGCAGTC TACCAACAGA TCCTCACCAG
201  TATGAATTCC ACAAACGTGA TCCAAATATC CAACGACCTG GAGAACCTCC
251  GGGATCTTCT TCACGTGCTG GCCAACTCTA CCAGCTGCCA CTTGCCCTGG
301  GCCAATGGCA CGGAGACCTT GGACAGCCTG GGGGGTGTCC TGGAAGCTTC
351  AGGCTACTCC ACAGAGGTGG TGGCCCTGAG CAGGCTGCAG GGGTCTCTGC
401  AGGACATGCT GTGGCAGCTG GACCTCAGCC CTGGGTGC
```

20. Vektor, enthaltend eine Nukleinsäure, die für ein glykosyliertes Leptin nach einem der Ansprüche 13 bis 19 kodiert.

21. Vektor nach Anspruch 20, der ein Expressionsvektor ist.

22. Wirtszelle, enthaltend eine Nukleinsäure, die für ein glykosyliertes Leptin-Protein nach Anspruch 1 kodiert.

23. Wirtszelle nach Anspruch 22, ausgewählt aus prokaryontischen und eukaryontischen Zellen.

24. Prokaryontische Wirtszelle nach Anspruch 23, die eine bakterielle Zelle ist.

25. Eukaryontische Wirtszelle nach Anspruch 23, die ausgewählt ist aus Säugerzellen, Hefezellen und Insektenzellen.

26. Säugerwirtszelle nach Anspruch 25, ausgewählt aus menschlichen Zellen, Affenzellen, BHK Zellen und CHO Zellen.

27. Verfahren zur Herstellung eines Proteins nach Anspruch 1, umfassend:

(a) Kultivieren einer Zelle, die eine Nukleinsäure enthält, die für das glykosylierte Leptin-Protein kodiert, unter geeigneten Bedingungen für die Expression; und
(b) Erhalten des Proteins.

28. Pharmazeutische Zusammensetzung zur parenteralen Injektion, intravenösen Injektion, subkutanen Injektion, intratekalen Verabreichung, nasalen Verabreichung, pulmonaren Verabreichung und osmotische Pumpen-Verabreichung, umfassen ein glykosyliertes Leptin-Protein nach einem der Ansprüche 1 bis 12 in einem pharmazeutisch akzeptablen Träger.

29. Glykosyliertes menschliches Leptin nach Anspruch 1 zur Verwendung in einem Verfahren der Behandlung eines Menschen gegen einen Zustand ausgewählt aus Fettsucht, Diabetes und hohem Blutfettgehalt-Effekten; wobei das Verfahren Verabreichen einer effektiven Menge des Leptins umfaßt.

30. Leptin nach Anspruch 29, wobei die effektive Menge des glykosylierten menschlichen Leptins durch Gentherapie verabreicht wird.

31. Selektives Bindungsmolekül, das für ein glykosyliertes Leptin-Protein nach Anspruch 1 selektiv ist, wobei das Molekül ausgewählt ist aus einem polyklonalen Antikörper, einem monoklonalen Antikörper und einem rekombinanten Antikörper.

32. Selektives Bindungsmolekül nach Anspruch 31, wobei das Molekül ein monoklonaler Antikörper ist.

33. Verfahren zur Herstellung eines glykosylierten Leptin-Proteins nach Anspruch 27, umfassend:

(a) Kultivieren einer Wirtszelle unter geeigneten Bedingungen für die Expression, die eine DNA Sequenz enthält, die, in der 5' zu 3' Richtung, (i) ein Signalpeptid und (ii) eine DNA, die für ein glykosyliertes Leptin-Protein nach Anspruch 1 kodiert, umfaßt; und
(b) Erhalten des glykosylierten Leptin-Proteins.

34. Verfahren nach Anspruch 33, wobei das Signalpeptid ausgewählt ist aus:

(a) (SEQ ID NR. 3) (natives menschliches Leptin-Signalpeptid)

MHWGTLCGFLWLWPYLFYVQA

(b) (SEQ ID NR. 4) (modifiziertes menschliches Leptin-Signalpeptid)

MHWGTLCGFLWLWPYLFYVSPS

(c) (SEQ ID NR. 5) (modifiziertes menschliches Leptin-Signalpeptid)

MHWGTLCGFLWLWPYLFYVSP

(d) (SEQ ID NR. 6) (modifiziertes menschliches Leptin-Signalpeptid)

MHWGTLCGFLWLWPYLFYVSPA

(e) (SEQ ID NR. 7) (modifiziertes menschliches Leptin-Signalpeptid)

MHWGTLCGFLWLWPYLFYVSNS

(f) (SEQ ID NR. 8) (natives menschliches tPA Signalpeptid)

MDAMKRGLCCVLLLCGAVFVSPS

(g) (SEQ ID NR. 9) (natives menschliches tPA Signalpeptid)

MDAMKRGLCCVLLLCGAVFVSP

(h) (SEQ ID NR. 10) (modifiziertes tPA Signalpeptid)

MDAMKRGLCCVLLLCGAVFVSNS

(i) (SEQ ID NR. 11) (modifiziertes tPA Signalpeptid)

MDAMKRGLCCVLLLCGAVFVSPA

(j) (SEQ ID NR. 12) (Leptin/tPA Signalpeptid)

MHWGTLCCVLLLCGAVFVSPA

(k) (SEQ ID NR. 13) (Leptin/tPA Signalpeptid)

MHWGTLCCVLLLCGAVFVSP

**35.** Verfahren nach Anspruch 33, wobei das Signalpeptid ausgewählt ist aus dem Signalpeptid für:

Erythropoitin, Faktor VIII, beta-Interferon, Serumalbumin, Insulin, von Willebrand Faktor, CD11$\alpha$, IgG, Follistatin, intrinsischer Faktor, G-CSF, Zeruloplasmin und LAMP-1.

**Revendications**

1. Protéine leptine glycosylée comprenant la SEQ. ID N°1 ayant une ou plusieurs modification de séquence comme un site de glycosylation choisi parmi (où "T/S" désigne la thréonine ou la sérine) :

(a) 01V → N 02P → X (où X est un quelconque acide aminé excepté la proline) O3I → T/S
(b) 02P → N 03I 04Q → T/S
(c) 23D → N 24I 25S → T/S
(d) 47P → N 48I 49L → T/S
(e) 48I → N 49L 50T/S
(f) 69P → N 70S 71R → T/S
(g) 92F → N 93S 94K → T/S

(h) 101A → N 102S 103G → T/S
(i) 102S→N 103G 104L→T/S
(j) 103G→N 104L 105E→T/S.

2. Protéine leptine glycosylée comprenant les acides aminés 1-146 de la SEQ. ID N°1, ayant deux sites de glycosylation, lesdits deux sites choisis parmi (par rapport à la numérotation de la SEQ. ID N°1) :

47 + 69 ;
48 + 69 ;
69 + 101 ;
69 + 102 ;
69 + 103 ;
69 + 118 ; et,
100 + 102.

3. Protéine leptine glycosylée comprenant les acides aminés 1-146 de la SEQ. ID N°1, ayant trois sites de glycosylation, lesdits trois sites choisis parmi (par rapport à la numérotation de la SEQ. ID N°1) :

2 + 47 + 69
23 + 47 + 69 ;
47 + 69 + 100 ;
47 + 69 + 102 ;
48 + 69 + 118 ;
69 + 102 + 118 ; et,
69 + 103 + 118.

4. Protéine leptine glycosylée comprenant les acides aminés 1-146 de la SEQ. ID N°1, ayant quatre sites de glycosylation, lesdits quatre sites choisis parmi (par rapport à la numérotation de la SEQ. ID N°1) :

2 + 47 + 69 + 92 ;
2 + 47 + 69 + 102 ;
23 + 47 + 69 + 92 ;
23 + 47 + 69 + 102 ; et,
47 + 69 + 100 + 102.

5. Protéine leptine glycosylée comprenant les acides aminés 1-146 de la SEQ. ID N°1, ayant cinq sites de glycosylation, lesdits cinq sites choisis parmi (par rapport à la numérotation de la SEQ. ID N°1) :

2 + 23 + 47 + 69 + 92
2 + 47 + 69 + 92 + 102
23 + 47 + 69 + 92 + 102.

6. Leptine glycosylée 2,47,69, selon la revendication 3, comprenant la séquence d'acides aminés (SEQ. ID N°26) :

```
  1   VNITKVQDDT KTLIKTIVTR INDISHTQSV SSKQKVTGLD FIPGLHNITT

 51   LSKMDQTLAV YQQILTSMNS TNVIQISNDL ENLRDLLHVL AFSKSCHLPX

101   ASGLETLDSL GGVLEASGYS TEVVALSRLQ GSLQDMLWQL DLSPGC
```

7. Leptine glycosylée 2,47,69,92, selon la revendication 4, comprenant la séquence d'acides aminés (SEQ. ID N°28) :

```
  1  VNITKVQDDT KTLIKTIVTR INDISHTQSV SSKQKVTGLD FIPGLHNITT

 51  LSKMDQTLAV YQQILTSMNS TNVIQISNDL ENLRDLLHVL ANSTSCHLPX

101  ASGLETLDSL GGVLEASGYS TEVVALSRLQ GSLQDMLWQL DLSPGC
```

**8.** Leptine glycosylée 2,47,69,102, selon la revendication 4, comprenant la séquence d'acides aminés (SEQ. ID N°30) :

```
  1  VNITKVQDDT KTLIKTIVTR INDISHTQSV SSKQKVTGLD FIPGLHNITT

 51  LSKMDQTLAV YQQILTSMNS TNVIQISNDL ENLRDLLHVL AFSKSCHLPX

101  ANGTETLDSL GGVLEASGYS TEVVALSRLQ GSLQDMLWQL DLSPGC
```

**9.** Leptine glycosylée 47,69,102, selon la revendication 3, comprenant la séquence d'acides aminés (SEQ. ID N°32) :

```
  1  VPIQKVQDDT KTLIKTIVTR INDISHTQSV SSKQKVTGLD FIPGLHNITT

 51  LSKMDQTLAV YQQILTSMNS TNVIQISNDL ENLRDLLHVL AFSKSCHLPW

101  ANGTETLDSL GGVLEASGYS TEVVALSRLQ GSLQDMLWQL DLSPGC
```

**10.** Leptine glycosylée 2,47,69,92,102, selon la revendication 5, comprenant la séquence d'acides aminés (SEQ. ID N°34) :

```
  1  VNITKVQDDT KTLIKTIVTR INDISHTQSV SSKQKVTGLD FIPGLHNITT

 51  LSKMDQTLAV YQQILTSMNS TNVIQISNDL ENLRDLLHVL ANSTSCHLPW

101  ANGTETLDSL GGVLEASGYS TEVVALSRLQ GSLQDMLWQL DLSPGC
```

**11.** Leptine glycosylée 47,69,92,102, selon la revendication 4, comprenant la séquence d'acides aminés (SEQ. ID N°36) :

```
  1  VPIQKVQDDT KTLIKTIVTR INDISHTQSV SSKQKVTGLD FIPGLHNITT

 51  LSKMDQTLAV YQQILTSMNS TNVIQISNDL ENLRDLLHVL ANSTSCHLPW

101  ANGTETLDSL GGVLEASGYS TEVVALSRLQ GSLQDMLWQL DLSPGC
```

**12.** Protéine leptine glycosylée selon la revendication 1 ayant une séquence à résidu N-terminal choisie parmi:

un résidu de sérine, arginine, proline ou alanine à la position -1,
une sérine à la position -1 et une proline à la position -2,
une séquence sérine-proline-sérine aux positions -1, -2 et -3,
une sérine à la position -1 et une arginine à la position -2,
une sérine à la position -1 , une arginine à la position -2 et une sérine à la position -3,
une arginine à la position -1, et une sérine à la position -2 ; et,
une alanine à la position -1 et une proline aux positions -2.

**13.** Acide nucléique codant une protéine leptine glycosylée selon la revendication 1.

**14.** Acide nucléique codant une leptine glycosylée 2,47,69 comprenant la séquence d'acide nucléique (SEQ. ID N°25) :

```
  1   GTGAACATCA CAAAAGTCCA AGATGACACC AAAACCCTCA TCAAGACAAT
 51   TGTCACCAGG ATCAATGACA TTTCACACAC GCAGTCAGTC TCCTCCAAAC
101   AGAAAGTCAC CGGTTTGGAC TTCATTCCTG GGCTCCACAA CATCACGACC
151   TTATCCAAGA TGGACCAGAC ACTGGCAGTC TACCAACAGA TCCTCACCAG
201   TATGAATTCC ACAAACGTGA TCCAAATATC CAACGACCTG GAGAACCTCC
251   GGGATCTTCT TCACGTGCTG GCCTTCTCTA AGAGCTGCCA CTTGCCCTGG
301   GCCAGTGGCC TGGAGACCTT GGACAGCCTG GGGGGTGTCC TGGAAGCTTC
351   AGGCTACTCC ACAGAGGTGG TGGCCCTGAG CAGGCTGCAG GGGTCTCTGC
401   AGGACATGCT GTGGCAGCTG GACCTAAGCC CTGGGTGC
```

**15.** Acide nucléique codant une leptine glycosylée 2,47,69,92 comprenant la séquence d'acide nucléique (SEQ. ID N°27) :

```
  1   GTGAACATCA CAAAAGTCCA AGATGACACC AAAACCCTCA TCAAGACAAT
 51   TGTCACCAGG ATCAATGACA TTTCACACAC GCAGTCAGTC TCCTCCAAAC
101   AGAAAGTCAC CGGTTTGGAC TTCATTCCTG GGCTCCACAA CATCACGACC
151   TTATCCAAGA TGGACCAGAC ACTGGCAGTC TACCAACAGA TCCTCACCAG
201   TATGAATTCC ACAAACGTGA TCCAAATATC CAACGACCTG GAGAACCTCC
251   GGGATCTTCT TCACGTGCTG GCCAACTCTA CCAGCTGCCA CTTGCCCTGG
301   GCCAGTGGCC TGGAGACCTT GGACAGCCTG GGGGGTGTCC TGGAAGCTTC
351   AGGCTACTCC ACAGAGGTGG TGGCCCTGAG CAGGCTGCAG GGGTCTCTGC
401   AGGACATGCT GTGGCAGCTG GACCTCAGCC CTGGGTGC
```

**16.** Acide nucléique codant une leptine glycosylée 2,47,69,102 comprenant la séquence d'acide nucléique (SEQ. ID N°29) :

```
  1  GTGAACATCA CAAAAGTCCA AGATGACACC AAAACCCTCA TCAAGACAAT

 51  TGTCACCAGG ATCAATGACA TTTCACACAC GCAGTCAGTC TCCTCCAAAC

101  AGAAAGTCAC CGGTTTGGAC TTCATTCCTG GGCTCCACAA CATCACGACC

151  TTATCCAAGA TGGACCAGAC ACTGGCAGTC TACCAACAGA TCCTCACCAG

201  TATGAATTCC ACAAACGTGA TCCAAATATC CAACGACCTG GAGAACCTCC

251  GGGATCTTCT TCACGTGCTG GCCTTCTCTA AGAGCTGCCA CTTGCCCTGG

301  GCCAATGGCA CGGAGACCTT GGACAGCCTG GGGGGTGTCC TGGAAGCTTC

351  AGGCTACTCC ACAGAGGTGG TGGCCCTGAG CAGGCTGCAG GGGTCTCTGC

401  AGGACATGCT GTGGCAGCTG GACCTCAGCC CTGGGTGC
```

**17.** Acide nucléique codant une leptine glycosylée 47,69,102 comprenant la séquence d'acide nucléique (SEQ. ID N°31) :

```
  1  GTGCCCATCC AAAAAGTCCA AGATGACACC AAAACCCTCA TCAAGACAAT

 51  TGTCACCAGG ATCAATGACA TTTCACACAC GCAGTCAGTC TCCTCCAAAC

101  AGAAAGTCAC CGGTTTGGAC TTCATTCCTG GGCTCCACAA CATCACGACC

151  TTATCCAAGA TGGACCAGAC ACTGGCAGTC TACCAACAGA TCCTCACCAG

201  TATGAATTCC ACAAACGTGA TCCAAATATC CAACGACCTG GAGAACCTCC

251  GGGATCTTCT TCACGTGCTG GCCTTCTCTA AGAGCTGCCA CTTGCCCTGG

301  GCCAATGGCA CGGAGACCTT GGACAGCCTG GGCGGTGTCC TGGAAGCTTC

351  AGGCTACTCC ACAGAGGTGG TGGCCCTGAG CAGGCTGCAG GGGTCTCTGC

401  AGGACATGCT GTGGCAGCTG GACCTCAGCC CTGGGTGC
```

**18.** Acide nucléique codant une leptine glycosylée 2,47,69,92,102 comprenant la séquence d'acide nucléique (SEQ. ID N°33) :

```
  1   GTGAACATCA CAAAAGTCCA AGATGACACC AAAACCCTCA TCAAGACAAT
 51   TGTCACCAGG ATCAATGACA TTTCACACAC GCAGTCAGTC TCCTCCAAAC
101   AGAAAGTCAC CGGTTTGGAC TTCATTCCTG GGCTCCACAA CATCACGACC
151   TTATCCAAGA TGGACCAGAC ACTGGCAGTC TACCAACAGA TCCTCACCAG

201   TATGAATTCC ACAAACGTGA TCCAAATATC CAACGACCTG GAGAACCTCC
251   GGGATCTTCT TCACGTGCTG GCCAACTCTA CCAGCTGCCA CTTGCCCTGG
301   GCCAATGGCA CGGAGACCTT GGACAGCCTG GGGGGTGTCC TGGAAGCTTC
351   AGGCTACTCC ACAGAGGTGG TGGCCCTGAG CAGGCTGCAG GGGTCTCTGC
401   AGGACATGCT GTGGCAGCTG GACCTCAGCC CTGGGTGC
```

**19.** Acide nucléique codant une leptine glycosylée 47,69,92,102 comprenant la séquence d'acide nucléique (SEQ. ID N°35) :

```
  1   GTGCCCATCC AAAAAGTCCA AGATGACACC AAAACCCTCA TCAAGACAAT
 51   TGTCACCAGG ATCAATGACA TTTCACACAC GCAGTCAGTC TCCTCCAAAC
101   AGAAAGTCAC CGGTTTGGAC TTCATTCCTG GGCTCCACAA CATCACGACC
151   TTATCCAAGA TGGACCAGAC ACTGGCAGTC TACCAACAGA TCCTCACCAG
201   TATGAATTCC ACAAACGTGA TCCAAATATC CAACGACCTG GAGAACCTCC
251   GGGATCTTCT TCACGTGCTG GCCAACTCTA CCAGCTGCCA CTTGCCCTGG
301   GCCAATGGCA CGGAGACCTT GGACAGCCTG GGGGGTGTCC TGGAAGCTTC
351   AGGCTACTCC ACAGAGGTGG TGGCCCTGAG CAGGCTGCAG GGGTCTCTGC
401   AGGACATGCT GTGGCAGCTG GACCTCAGCC CTGGGTGC
```

**20.** Vecteur contenant un acide nucléique codant une protéine leptine glycosylée selon une quelconque des revendications 13-19.

**21.** Vecteur selon la revendication 20 qui est un vecteur d'expression.

**22.** Cellule hôte contenant un acide nucléique codant une protéine leptine glycosylée selon la revendication 1.

**23.** Cellule hôte de la revendication 22, choisie parmi des cellules procaryotes et eucaryotes.

**24.** Cellule hôte procaryote de la revendication 23 qui est une cellule bactérienne.

**25.** Cellule hôte eucaryote de la revendication 23, qui est choisie parmi des cellules de mammifère, de levure et d'insecte.

**26.** Cellule hôte de mammifère selon la revendication 25, choisie parmi des cellules humaines, des cellules de singe, des cellules BHK et des cellules CHO.

**27.** Procédé pour préparer une protéine selon la revendication 1, consistant à :

(a) mettre en culture une cellule contenant un acide nucléique codant pour ladite protéine leptine glycosylée dans des conditions d'expression appropriées ; et
(b) obtenir ladite protéine.

**28.** Composition pharmaceutique pour une injection parentérale, une injection intraveineuse, une injection sous-cutanée, une administration intrarachidienne, une administration nasale, une administration pulmonaire et une administration par pompe osmotique comprenant une protéine leptine glycosylée selon une quelconque des revendications 1-12 dans un support pharmaceutiquement acceptable.

**29.** Leptine humaine glycosylée selon la revendication 1 pour une utilisation dans un procédé de traitement d'un être humain pour un état choisi parmi l'obésité, le diabète et les effets de lipémie élevée ;
ledit procédé consistant à administrer une quantité efficace de ladite leptine.

**30.** Leptine selon la revendication 29 dans laquelle ladite quantité efficace de ladite leptine humaine glycosylée est administrée par thérapie génique.

**31.** Molécule de liaison sélective qui est sélective pour une protéine leptine glycosylée selon la revendication 1, laquelle molécule est choisie parmi un anticorps polyclonal, un anticorps monoclonal et un anticorps de recombinaison.

**32.** Molécule de liaison sélective selon la revendication 31, dans laquelle la molécule est un anticorps monoclonal.

**33.** Procédé de fabrication d'une protéine leptine glycosylée selon la revendication 27, consistant à :

a) mettre en culture, dans des conditions d'expression appropriées, une cellule hôte contenant une séquence d'ADN codant, dans la direction 5' à 3' (i) un peptide signal et (ii) un ADN codant une protéine leptine glycosylée selon la revendication 1 ; et
b) obtenir ladite protéine.

**34.** Procédé de la revendication 33, dans lequel ledit peptide signal est choisi parmi le peptide signal pour :

a) (SEQ. ID N°3) peptide signal de leptine humaine naturelle

MHWGTLCGFLWLWPYLFYVQA

b) (SEQ. ID N°4) peptide signal de leptine humaine modifiée

MHWGTLCGFLWLWPYLFYVSPS

c) (SEQ. ID N°5) peptide signal de leptine humaine modifiée

MHWGTLCGFLWLWPYLFYVSP

d) (SEQ. ID N°6) peptide signal de leptine humaine modifiée

MHWGTLCGFLWLWPYLFYVSPA

e) SEQ. ID N°7) peptide signal de leptine humaine modifiée

MHWGTLCGFLWLWPYLFYVSNS

f) (SEQ. ID N°8) peptide signal de leptine humaine naturelle

MDAMKRGLCCVLLLCGAVFVSPS

g) (SEQ. ID N°9) peptide signal de leptine humaine naturelle

MDAMKRGLCCVLLLCGAVFVSP

h) (SEQ. ID N°10) peptide signal de tPA modifié

MDAMKRGLCCVLLLCGAVFVSNS

i) (SEQ. ID N°11) peptide signal de tPA modifié

MDAMKRGLCCVLLLCGAVFVSPA

j) (SEQ. ID N°12) peptide signal leptine/tPA

MHWGTLCCVLLLCGAVFVSPS

k) SEQ. ID N°13) peptide signal leptine/tPA

MHWGTLCCVLLLCGAVFVSP.

**35.** Procédé de la revendication 33, dans lequel ledit peptide signal est choisi parmi le peptide signal pour :

l'érythropoïétine, le facteur VIII, le bêta-interféron, le sérum-albumine, l'insuline, le facteur de von Willebrand, CD11$\alpha$, IgG, la follistatine, le facteur intrinsèque, G-csf, la céruloplasmine et LAMP-1.

# FIG. 1

— — Leptin (0.2 mg/ml, 1 mg/kg, 100ul)
—■— Leptin (2 mg/ml, 10 mg/kg, 100ul)
—♦— Glycosylated CHO Leptin (0.2 mg/ml, 1 mg/kg, 100ul)
—⊠— Glycosylated CHO Leptin (2 mg/ml, 10 mg/kg, 100ul)
—●— A4S Control (400ul, day 0)
—⊞— Leptin (5 mg/ml, 100 mg/kg, 400ul, day 0)
—▲— Glycosylated CHO Leptin (5 mg/ml, 100 mg/kg, 400ul, day 0)

# FIG. 2

# FIG. 3

—△— Glycosylated Leptin
—○— r-metHuLeptin (from Study OBH.036)

# FIG. 4

—△— Glycosylated Leptin
—○— r-metHuLeptin (from Study OBH.036)

## FIG. 5

- ●— Vehicle
- ✕— hLeptin 0.5 mg/kg/d
- △— GE-Leptin 0.5 mg/kg/d

## FIG. 6

- ●— Vehicle
- ✕— hLeptin 0.5 mg/kg/d
- △— GE-Leptin 0.5 mg/kg/d

# FIG. 7

# FIG. 8

# FIG. 9

N-glycosylation Sites
N47+N69+N102

Signal Peptides

| Leptin | Tpa | Tpa/Leptin | | Leptin/Tpa | | Leptin (SP) | | Leptin (SPS) | |
|--------|-----|------------|---|------------|---|-------------|---|--------------|---|
| 1 | 1 | 1 | 2 | 1 | 2 | 1 | 2 | 1 | 2 |

Clone Number

Triple Glycosylated Leptin

Unglycosylated Leptin

EP 1 151 102 B1

# FIG. 10

# FIG. 11

| | Signal Peptide | Positions of N-glycosylation sites |
|---|---|---|
| Mock | | |
| W.T. | | |
| Leptin | N48 | |
| Leptin | N47+N69 | |
| Leptin | N47+N69+N102 | |
| Tpa (SNS) | N2+N47+N69+N92 | |
| Tpa | N2+N23+N47+N69+N92 | |

Mw
kDa

46

30

21.5

14.3